# EUROPEAN PATENT SPECIFICATION

(11) **EP 0 211 077 B1**
(45) Date of publication and mention of the grant of the patent: **05.05.1993**
(21) Application number: 86902087.5
(22) Date of filing: 05.02.1986
(51) Int. Cl.: C07K 15/00, C12N 15/62, C12N 15/70

(54) **METALLOPROTEINASE INHIBITOR SEQUENCE RECOMBINANT VECTOR SYSTEM FOR USING SAME AND RECOMBINANT-DNA METHOD FOR THE MANUFACTURE OF SAME**
REKOMBINANTES VEKTORSYSTEM FÜR METALLOPROTEINASE-INHIBITORSEQUENZ UND DESSEN VERWENDUNG UND HERSTELLUNG NACH DEM REKOMBINANT-DNS-VERFAHREN
VECTEURS RECOMBINANTS DE SEQUENCES D'INHIBITEURS DE METALLOPROTEINASE, SYSTEME D'UTILISATION ET PROCEDE DE PRODUCTION DE CES VECTEURS GRACE A L'ADN RECOMBINANT

(30) Priority: 05.02.1985 US 699181; 04.10.1985 US 784319
(43) Date of publication of application: 25.02.1987
(73) Proprietor: SYNERGEN BIOLOGICALS, INC., Boulder, CO 83031 (US)
(72) Inventor: CARMICHAEL, David, F., Louisville, CO 80027 (US); ANDERSON, David C., 1200 12th Avenue South Seattle,WA 98144 (US); STRICKLIN, George, P., Germantown, TN 38119 (US); WELGUS, Howard, G., St. Louis, MO 63011 (US)
(74) Representative: Grünecker, Kinkeldey, Stockmair & Schwanhäusser Anwaltssozietät
(86) International application number: PCT/US86/00302
(87) International publication number: WO 86/04608

(56) References cited:
- EP-A- 0 103 409
- EP-A- 0 114 506
- EP-A- 0 188 312
- EP-A- 0 189 784
- WO-A-84/03711
- WO-A-86/02100
- US-A- 4 276 284
- US-A- 4 438 032
- NATURE, vol. 302, 24th March 1983, pages 305-310, Macmillan Journals Ltd.; T. TANIGUCHI et al.: "Structure and expression of a cloned cDNA for human interleukin-2"
- AM. J. HUM. GENET, vol. 31, 1979, pages 531-538, American Society of Human Genetics; A.D. RIGGS et al.: "Synthetic DNA and medicine"
- NATURE, vol. 318, no. 6041, issued November, 1985, Docherty, et al, "Sequence of Human Tissue Inhibitor of Metalloproteinases and its Identity to Erythorid-Potentiating Activity", pp. 66-69
- J. BIOLOGICAL CHEMISTRY, vol. 258, No. 20, issued October, 1983, Stricklin, et al, "Human Skin Fibroblast Collagenase Inhibitor," pp. 12252-12258
- BIOCHIMICA ET BIOPHYSICA ACTA, issued April, 1985, Murphy et al, "Tissue inhibitor of metalloproteinase. Identification of Precursor forms synthesized by Human Fibroblasts in Culture, pp. 214-218
- J. BIOLOGICAL CHEMISTRY, VOL. 258, NO. 20 pp. 12259-12264
- NATURE, vol. 318, no. 6044, issued November 1985, Patarca et al, "A Major Retroviral Core Protein Related to EPA and TIMP", s. p. 390
- Chemical Abstracts, vol. 103, no. 11, issued 1985 (Columbus, Ohio, USA) Gasson, et al. "Molecular Characterization and Expression of the Gene Encoding Human Erythoried-Potentiating Activity", s. p. 152, Column 1, the abstract No. 82647y, NATURE (London) 1985, 315 (6022) 768-71 (Eng).

## Description

### BACKGROUND OF THE INVENTION

This is a continuation - in - part application of U.S. Patent Application Serial No. 784,319, filed October 4, 1985, which is a continuation - in - part application of U.S. Patent Application Serial No. 699,181, filed February 5, 1985. Endogenous proteolytic enzymes serve to degrade invading organisms, antigen- antibody complexes and certain tissue proteins which are no longer necessary or useful to the organism. In a normally functioning organism, proteolytic enzymes are produced in a limited quantity and are regulated in part through specific inhibitors.

Metalloproteinases are enzymes present in the body which are often involved in the degradation of connective tissue. While some connective tissue degradation is necessary for normal functioning of an organism, an excess of connective tissue degradation occurs in several disease states and is believed to be attributable, at least in part, to excess metalloproteinase. It is believed that metalloproteinases are at least implicated in periodontal disease, corneal and skin ulcers, rheumatoid arthritis and the spread of cancerous solid tumors.

These diseases generally occur in areas of the body which contain a high proportion of collagen, a particular form of connective tissue. An examination of patients with these diseases of connective tissue has revealed an excessive breakdown of the various components of connective tissues, including collagen proteoglycans and elastin. Therefore, it has been deduced that an excessive concentration of a particular metalloproteinase, for example collagenase, proteoglyconase, gelatinase, and certain elastases, may cause or exacerbate the connective tissue destruction associated with the aforementioned diseases.

In the normal state, the body possesses metalloproteinase inhibitors which bind to metalloproteinases to effectively prevent these enzymes from acting on their connective tissue substrates. Specifically, in a healthy organism, metalloproteinase inhibitors are present in concentrations sufficient to interact with metalloproteinases to an extent which allows sufficient quantities of metalloproteinase to remain active while binding the excess metalloproteinase so that the connective tissue damage seen in the various diseases does not occur.

It is postulated that one immediate cause of the connective tissue destruction present in the foregoing disease states is an imbalance in the relative metalloproteinase/metalloproteinase inhibitor concentrations. In these situations, either due to an excessive amount of active metalloproteinase or a deficiency in the amount of active metalloproteinase inhibitor, the excess metalloproteinase is believed to cause the connective tissue degradation responsible for causing or exacerbating the disease. It is postulated that, by treating persons with connective tissue diseases with metalloproteinase inhibitors, the degradative action of the excess metalloproteinase may be curtailed or halted. Therefore, particular metalloproteinase inhibitors of specific interest to the present inventors are collagenase inhibitors because it is believed that these inhibitors would be pharmaceutically useful in the treatment or prevention of connective tissue diseases.

The existence of metalloproteinase and metalloproteinase inhibitors has been discussed in the scientific literature. For example, Sellers et al., Biochemical And Biophysical Research Communications 87:581 -587 (1979), discusses isolation of rabbit bone collagenase inhibitor. Collagenase inhibitor isolated from human skin fibroblasts is discussed in Stricklin and Welgus, J.B.C. 258:12252 - 12258 (1983) and Welgus and Stricklin, J.B.C. 258:12259 - 12264 (1983). The presence of collagenase inhibitors in naturally - occurring body fluids is further discussed in Murphy et al., Biochem. J. 195:167 - 170 (1981) and Cawston et al., Arthritis and Rheumatism, 27:285 (1984). In addition, metalloproteinase inhibitors are discussed by Reyn - olds et al. in Cellular Interactions, Dingle and Gordon, eds., (1981). Although these articles characterize particular, isolated metalloproteinase inhibitors and discuss, to some extent, the role or potential role of metalloproteinases in connective tissue disease treatment and speculate on the ability of metalloproteinase inhibitors to counteract this destruction, none of these researchers had previously been able to isolate a portable DNA sequence capable of directing intracellular production of metalloproteinase inhibitors or to create a recombinant - DNA method for the production of these inhibitors.

Surprisingly, the present inventors have discovered a portable DNA sequence capable of directing the recombinant - DNA synthesis of metalloproteinase inhibitors. These metalloproteinase inhibitors are biologically equivalent to those isolated from human skin fibroblast cultures. The metalloproteinase inhibitors of the present invention, prepared by the recombinant-DNA methods set forth herein, will enable increased research into prevention and treatment of metalloproteinase - induced connective tissue diseases. In addition, the metalloproteinase inhibitors of the present invention are useful in neutralizing metalloproteinases, including the excess metalloproteinase associated with disease states. Therefore, it is believed that a cure for these diseases will be developed which will embody, as an active ingredient, the metalloproteinase inhibitors of the present invention. Furthermore, the metalloproteinase inhibitors of the present invention are capable of interacting with their metalloproteinase targets in a manner which allows the development of diagnostic tests for degradative connective tissue diseases using the newly discovered inhibitors.

The recombinant metalloproteinase inhibitors discussed herein interact stoichiometrically (i.e., in a 1:1 ratio) with their metalloproteinase targets. In addition, these metalloproteinase inhibitors are heat resistant, acid stable, glycosylated, and exhibit a high isoelectric point.

### SUMMARY OF THE INVENTION

The present invention relates to metalloproteinase inhibitors and a recombinant-DNA method of producing the same and to portable DNA sequences capable of directing intracellular production of the metalloproteinase inhibitors. Particularly, the present invention relates to a collagenase inhibitor, a recombinant-DNA method for producing the same and to portable DNA sequences for use in the recombinant method. The present invention also relates to a series of vectors containing these portable DNA sequences.

One object of the present invention is to provide a metalloproteinase inhibitor, which can be produced in sufficient quantities and purities to provide economical pharmaceutical compositions which possess metalloproteinase inhibitor activity.

An additional object of the present invention is to provide a recombinant - DNA method for the production of these metalloproteinase inhibitors. The recombinant metalloproteinase inhibitors produced by this method are biologically equivalent to the metalloproteinase inhibitor isolable from human skin fibroblast cultures.

To facilitate the recombinant-DNA synthesis of these metalloproteinase inhibitors, it is a further object of the present invention to provide portable DNA sequences capable of directing intracellular production of metalloproteinase inhibitors. It is also an object of the present invention to provide cloning vectors containing these portable sequences. These vectors are capable of being used in recombinant systems to produce pharmaceutically useful quantities of metalloproteinase inhibitors.

Additional objects and advantages of the invention will be set forth in part in the description which follows, and in part will be obvious from the description or may be learned from practice of the invention. The objects and advantages may be realized and attained by means of the instrumentalities and combinations particularly pointed out in the appended claims.

To achieve the objects and in accordance with the purposes of the present invention, metalloproteinase inhibitors are set forth, which are capable of stoichiometric reaction with metalloproteinases. These metalloproteinase inhibitors are remarkably heat resistant, acid stable, glycosylated, and exhibit a high isoelectric point. Furthermore, these metalloproteinase inhibitors are biologically equivalent to those inhibi - tors isolated from human skin fibroblast cultures.

To further achieve the objects and in accordance with the purposes of the present invention, as embodied and broadly described herein, portable DNA sequences coding for metalloproteinase inhibitors are provided. These sequences comprise nucleotide sequences capable of directing intracellular production of metalloproteinase inhibitors. The portable sequences may be either synthetic sequences or restriction fragments ("natural" DNA sequences). In a preferred embodiment, a portable DNA sequence is isolated from a human fibroblast cDNA library and is capable of directing intracellular production of a collagenase inhibitor which is biologically equivalent to that inhibitor which is isolable from a human skin fibroblast culture.

The coding strand of a first preferred DNA sequence which has been discovered has the following nucleotide sequence:

10 20 30 40 50 60
GTTGTTGCTG TGGCTGATAG CCCCAGCAGG GCCTGCACCT GTGTCCCACC CCACCCACAG

70 80 90 100 110 120
ACGGCCTTCT GCAATTCCGA CCTCGTCATC AGGGCCAAGT TCGTGGGGAC ACCAGAAGTC

130 140 150 160 170 180
AACCAGACCA CCTTATACCA GCGTTATGAG ATCAAGATGA CCAAGATGTA TAAAGGGTTC

190 200 210 220 230 240
CAAGCCTTAG GGGATGCCGC TGACATCCGG TTCGTCTACA CCCCCGCCAT GGAGAGTGTC

250 260 270 280 290 300
TGCGGATACT TCCACAGGTC CCACAACCGC AGCGAGGAGT TTCTCATTGC TGGAAAACTG

310 320 330 340 350 360
CAGGATGGAC TCTTGCACAT CACTACCTGC AGTTTCGTGG CTCCCTGGAA CAGCCTGAGC

370 380 390 400 410 420
TTAGCTCAGC GCCGGGGCTT CACCAAGACC TACACTGTTG GCTGTGAGGA ATGCACAGTG

430 440 450 460 470 480
TTTCCCTGTT TATCCATCCC CTGCAAACTG CAGAGTGGCA CTCATTGCTT GTGGACGGAC

490 500 510 520 530 540
CAGCTCCTCC AAGGCTCTGA AAAGGGCTTC CAGTCCCGTC ACCTTGCCTG CCTGCCTCGG

550 560 570 580 590 600
GAGCCAGGGC TGTGCACCTG GCAGTCCCTG CGGTCCCAGA TAGCCTGAAT CCTGCCCGGA

610 620 630 640 650 660
GTGGAAGCTG AAGCCTGCAC AGTGTCCACC CTGTTCCCAC TCCCATCTTT CTTCCGGACA

670 680 690 700
ATGAAATAAA GAGTTACCAC CCAGCAAAAA AAAAAAGGAA TTC

The nucleotides represented by the foregoing abbreviations are set forth in the Detailed Description of the Preferred Embodiments.

A second preferrred DNA sequence has been discovered which has an additional nucleotide sequence 5' to the initiator sequence. This sequence, which contains as the eighty - second through four - hundred - thirty-second nucleotides nucleotoides 1 through 351 of the first preferred sequence set forth above, has the following nucleotide sequence:

10 20 30 40 50 60
GGCCATCGCC GCAGATCCAG CGCCCAGAGA GACACCAGAG AACCCACCAT GGCCCCCTTT

70 80 90 100 110 120
GACCCCTGGC TTCTGCATCC TGTTGTTGCT GTGGCTGATA GCCCCAGCAG GGCCTGCACC

130 140 150 160 170 180
TGTGTCCCAC CCCACCCACA GACGGCCTTC TGCAATTCCG ACCTCGTCAT CAGGGCCAAG

190 200 210 220 230 240
TTCGTGGGGA CACCAGAAGT CAACCAGACC ACCTTATACC AGCGTTATGA GATCAAGATG

250 260 270 280 290 300
ACCAAGATGT ATAAAGGGTT CCAAGCCTTA GGGGATGCCG CTGACATCCG GTTCGTCTAC

310 320 330 340 350 360
ACCCCCGCCA TGGAGAGTGT CTGCGGATAC TTCCACAGGT CCCACAACCG CAGCGAGGAG

370 380 390 400 410 420
TTTCTCATTG CTGGAAAACT GCAGGATGGA CTCTTGCACA TCACTACCTG CAGTTTCGTG

430
GCTCCCTGGA AC

A third preferred DNA sequence which incorporates the 5' region of the second preferred sequence and the 3' sequence of the first preferred sequence, has the following nucleotide sequence:

10 20 30 40 50 60
GGCCATCGCC GCAGATCCAG CGCCCAGAGA GACACCAGAG AACCCACCAT GGCCCCCTTT

70 80 90 100 110 120
GACCCCTGGC TTCTGCATCC TGTTGTTGCT GTGGCTGATA GCCCCAGCAG GGCCTGCACC

130 140 150 160 170 180
TGTGTCCCAC CCCACCCACA GACGGCCTTC TGCAATTCCG ACCTCGTCAT CAGGGCCAAG

190 200 210 220 230 240
TTCGTGGGGA CACCAGAAGT CAACCAGACC ACCTTATACC AGCGTTATGA GATCAAGATG

250 260 270 280 290 300
ACCAAGATGT ATAAAGGGTT CCAAGCCTTA GGGGATGCCG CTGACATCCG GTTCGTCTAC

310 320 330 340 350 360
ACCCCCGCCA TGGAGAGTGT CTGCGGATAC TTCCACAGGT CCCACAACCG CAGCGAGGAG

370 380 390 400 410 420
TTTCTCATTG CTGGAAAACT GCAGGATGGA CTCTTGCACA TCACTACCTG CAGTTTCGTG

430 440 450 460 470 480
GCTCCCTGGA ACAGCCTGAG CTTAGCTCAG CGCCGGGGCT TCACCAAGAC CTACACTGTT

490 500 510 520 530 540
GGCTGTGAGG AATGCACAGT GTTTCCCTGT TTATCCATCC CCTGCAAACT GCAGAGTGGC

550 560 570 580 590 600
ACTCATTGCT TGTGGACGGA CCAGCTCCTC CAAGGCTCTG AAAAGGGCTT CCAGTCCCGT

610 620 630 640 650 660
CACCTTGCCT GCCTGCCTCG GGAGCCAGGG CTGTGCACCT GGCAGTCCCT GCGGTCCCAG

670 680 690 700 710 720
ATAGCCTGAA TCCTGCCCGG AGTGGAAGCT GAAGCCTGCA CAGTGTCCAC CCTGTTCCCA

730 740 750 760 770 780
CTCCCATCTT TCTTCCGGAC AATGAAATAA AGAGTTACCA CCCAGCAAAA AAAAAAAGGA

Currently, for expression of the instant metalloproteinase inhibitors in animal cells, the inventors most prefer a method which utilizes a fourth preferred DNA sequence. The coding strand of this sequence reads as follows:

10 20 30 40 50 60
GGCCATCGCC GCAGATCCAG CGCCCAGAGA GACACCAGAG AACCCACCAT GGCCCCCTTT

70 80 90 100 110 120
GAGCCCCTGG CTTCTGGCAT CCTGTTGTTG CTGTGGCTGA TAGCCCCCAG CAGGGCCTGC

130 140 150 160 170 180
ACCTGTGTCC CACCCCACCC ACAGACGGCC TTCTGCAATT CCGACCTCGT CATCAGGGCC

190 200 210 220 230 240
AAGTTCGTGG GGACACCAGA AGTCAACCAG ACCACCTTAT ACCAGCGTTA TGAGATCAAG

250 260 270 280 290 300
ATGACCAAGA TGTATAAAGG GTTCCAAGCC TTAGGGGATG CCGCTGACAT CCGGTTCGTC

310 320 330 340 350 360
TACACCCCCG CCATGGAGAG TGTCTGCGGA TACTTCCACA GGTCCCACAA CCGCAGCGAG

370 380 390 400 410 420
GAGTTTCTCA TTGCTGGAAA ACTGCAGGAT GGACTCTTGC ACATCACTAC CTGCAGTTTC

430 440 450 460 470 480
GTGGCTCCCT GGAACAGCCT GAGCTTAGCT CAGCGCCGGG GCTTCACCAA GACCTACACT

490 500 510 520 530 540
GTTGGCTGTG AGGAATGCAC AGTGTTTCCC TGTTTATCCA TCCCCTGCAA ACTGCAGAGT

550 560 570 580 590 600
GGCACTCATT GCTTGTGGAC GGACCAGCTC CTCCAAGGCT CTGAAAAGGG CTTCCAGTCC

610 620 630 640 650 660
CGTCACCTTG CCTGCCTGCC TCGGGAGCCA GGGCTGTGCA CCTGGCAGTC CCTGCGGTCC

670 680 690 700 710 720
CAGATAGCCT GAATCCTGCC CGGAGTGGAA GCTGAAGCCT GCACAGTGTC CACCCTGTTC

730 740 750 760 770 780
CCACTCCCAT CTTTCTTCCG GACAATGAAA TAAAGAGTTA CCACCCAGCA AAAAAAAAAA

GGAATTC

To facilitate identification and isolation of natural DNA sequences for use in the present invention, the inventors have developed a human skin fibroblast cDNA library. This library contains the genetic information capable of directing a cell to synthesize the metalloproteinase inhibitors of the present invention. Other natural DNA sequences which may be used in the recombinant DNA methods set forth herein may be isolated from human genomic libraries.

Additionally, portable DNA sequences useful in the processes of the present invention may be synthetically created. These synthetic DNA sequences may be prepared by polynucleotide synthesis and sequencing techniques known to those of ordinary skill in the art.

Additionally, to achieve the objects and in accordance with the purposes of the present invention, a recombinant-DNA method is disclosed which results in microbial manufacture of the instant metal - loproteinase inhibitors using the portable DNA sequences referred to above. This recombinant DNA method comprises:
(a) preparation of a portable DNA sequence capable of directing a host microorganism to produce a protein having metalloproteinase inhibitor activity, preferably collagenase inhibitor activity;
(b) cloning the portable DNA sequence into a vector capable of being transferred into and replicating in a host microorganism, such vector containing operational elements for the portable DNA sequence;
(c) transferring the vector containing the portable DNA sequence and operational elements into a host microorganism capable of expressing the metalloproteinase inhibitor protein;
(d) culturing the host microorganism under conditions appropriate for amplification of the vector and expression of the inhibitor; and
(e) in either order:
   (i) harvesting the inhibitor; and
   (ii) causing the inhibitor to assume an active, tertiary structure whereby it possesses metalloproteinase inhibitor activity.

To further accomplish the objects and in further accord with the purposes of the present invention, a series of cloning vectors are provided comprising at least one of the portable DNA sequences discussed above. In particular, plasmid pUC9 - F5/237P1 0 is disclosed.

It is understood that both the foregoing general description and the following detailed description are exemplary and explanatory only and are not restrictive of the invention, as claimed.

The accompanying drawing, which is incorporated in and constitutes a part of this specification, illustrates one embodiment of the invention and, together with the description, serves to explain the principles of the invention.

### BRIEF DESCRIPTION OF THE DRAWINGS

Figure 1 is a partial restriction map of the plasmid pUC9 - F5/237P10.

### DETAILED DESCRIPTION OF THE PREFERRED EMBODIMENTS

Reference will now be made in detail to the presently preferred embodiments of the invention, which, together with the drawing and the following examples, serve to explain the principles of the invention.

As noted above, the present invention relates in part to portable DNA sequences capable of directing intracellular production of metalloproteinase inhibitors in a variety of host microorganisms. "Portable DNA sequence" in this context is intended to refer either to a synthetically - produced nucleotide sequence or to a restriction fragment of a naturally occuring DNA sequence. For purposes of this specification, "metalloproteinase inhibitor" is intended to mean the primary structure of the protein as defined by the codons present in the deoxyribonucleic acid sequence which directs intracellular production of the amino acid sequence, and which may or may not include post - translational modifications. It is contemplated that such post - translational modifications include, for example, glycosylation. It is further intended that the term "metalloproteinase inhibitor" refers to either the form of the protein as would be excreted from a microorganism or the methionyl - metalloproteinase inhibitor as it may be present in microorganisms from which it was not excreted.

In a preferred embodiment, the portable DNA sequences are capable of directing intracellular produc - tion of collagenase inhibitors. In a particularly preferred embodiment, the portable DNA sequences are capable of directing intracellular production of a collagenase inhibitor biologically equivalent to that previously isolated from human skin fibroblast cultures. By "biologically equivalent," as used herein in the specification and claims, it is meant that an inhibitor, produced using a portable DNA sequence of the present invention, is capable of preventing collagenase - induced tissue damage of the same type, but not necessarily to the same degree, as a native human collagenase inhibitor, specifically that native human collagenase inhibitor isolable from human skin fibroblast cell cultures.

A first preferred portable DNA sequence of the present invention has a nucleotide sequence as follows: 
10 20 30 40 50 60
GTTGTTGCTG TGGCTGATAG CCCCAGCAGG GCCTGCACCT GTGTCCCACC CCACCCACAG

70 80 90 100 110 120
ACGGCCTTCT GCAATTCCGA CCTCGTCATC AGGGCCAAGT TCGTGGGGAC ACCAGAAGTC

130 140 150 160 170 180
AACCAGACCA CCTTATACCA GCGTTATGAG ATCAAGATGA CCAAGATGTA TAAAGGGTTC

190 200 210 220 230 240
CAAGCCTTAG GGGATGCCGC TGACATCCGG TTCGTCTACA CCCCCGCCAT GGAGAGTGTC

250 260 270 280 290 300
TGCGGATACT TCCACAGGTC CCACAACCGC AGCGAGGAGT TTCTCATTGC TGGAAAACTG

310 320 330 340 350 360
CAGGATGGAC TCTTGCACAT CACTACCTGC AGTTTCGTGG CTCCCTGGAA CAGCCTGAGC

370 380 390 400 410 420
TTAGCTCAGC GCCGGGGCTT CACCAAGACC TACACTGTTG GCTGTGAGGA ATGCACAGTG

430 440 450 460 470 480
TTTCCCTGTT TATCCATCCC CTGCAAACTG CAGAGTGGCA CTCATTGCTT GTGGACGGAC

490 500 510 520 530 540
CAGCTCCTCC AAGGCTCTGA AAAGGGCTTC CAGTCCCGTC ACCTTGCCTG CCTGCCTCGG

550 560 570 580 590 600
GAGCCAGGGC TGTGCACCTG GCAGTCCCTG CGGTCCCAGA TAGCCTGAAT CCTGCCCGGA

610 620 630 640 650 660
GTGGAAGCTG AAGCCTGCAC AGTGTCCACC CTGTTCCCAC TCCCATCTTT CTTCCGGACA

670 680 690 700
ATGAAATAAA GAGTTACCAC CCAGCAAAAA AAAAAAGGAA TTC
 wherein the following nucleotides are represented by the abbreviations indicated below.

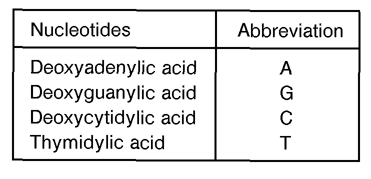

A second preferred portable DNA sequence of the present invention has the following nucleotide sequence:

10 20 30 40 50 60
GGCCATCGCC GCAGATCCAG CGCCCAGAGA GACACCAGAG AACCCACCAT GGCCCCCTTT

70 80 90 100 110 120
GACCCCTGGC TTCTGCATCC TGTTGTTGCT GTGGCTGATA GCCCCAGCAG GGCCTGCACC

130 140 150 160 170 180
TGTGTCCCAC CCCACCCACA GACGGCCTTC TGCAATTCCG ACCTCGTCAT CAGGGCCAAG

190 200 210 220 230 240
TTCGTGGGGA CACCAGAAGT CAACCAGACC ACCTTATACC AGCGTTATGA GATCAAGATG

250 260 270 280 290 300
ACCAAGATGT ATAAAGGGTT CCAAGCCTTA GGGGATGCCG CTGACATCCG GTTCGTCTAC

310 320 330 340 350 360
ACCCCCGCCA TGGAGAGTGT CTGCGGATAC TTCCACAGGT CCCACAACCG CAGCGAGGAG

370 380 390 400 410 420
TTTCTCATTG CTGGAAAACT GCAGGATGGA CTCTTGCACA TCACTACCTG CAGTTTCGTG

430
GCTCCCTGGA AC

In this second preferred sequence, an open reading frame exists from nucleotides 1 through 432. The first methionine of this reading frame is encoded by nucleotides by 49 through 51 and is the site of translation initiation. It should be noted that the amino acid sequence prescribed by nucleotides 49 through 114 is not found in the mature metalloproteinase. It is believed that this sequence is the leader peptide of the human protein.

A third preferred portable DNA sequence has the nucleotide sequence:

10 20 30 40 50 60
GGCCATCGCC GCAGATCCAG CGCCCAGAGA GACACCAGAG AACCCACCAT GGCCCCCTTT

70 80 90 100 110 120
GACCCCTGGC TTCTGCATCC TGTTGTTGCT GTGGCTGATA GCCCCAGCAG GGCCTGCACC

130 140 150 160 170 180
TGTGTCCCAC CCCACCCACA GACGGCCTTC TGCAATTCCG ACCTCGTCAT CAGGGCCAAG

190 200 210 220 230 240
TTCGTGGGGA CACCAGAAGT CAACCAGACC ACCTTATACC AGCGTTATGA GATCAAGATG

250 260 270 280 290 300
ACCAAGATGT ATAAAGGGTT CCAAGCCTTA GGGGATGCCG CTGACATCCG GTTCGTCTAC

310 320 330 340 350 360
ACCCCCGCCA TGGAGAGTGT CTGCGGATAC TTCCACAGGT CCCACAACCG CAGCGAGGAG

370 380 390 400 410 420
TTTCTCATTG CTGGAAAACT GCAGGATGGA CTCTTGCACA TCACTACCTG CAGTTTCGTG

430 440 450 460 470 480
GCTCCCTGGA ACAGCCTGAG CTTAGCTCAG CGCCGGGGCT TCACCAAGAC CTACACTGTT

490 500 510 520 530 540
GGCTGTGAGG AATGCACAGT GTTTCCCTGT TTATCCATCC CCTGCAAACT GCAGAGTGGC

550 560 570 580 590 600
ACTCATTGCT TGTGGACGGA CCAGCTCCTC CAAGGCTCTG AAAAGGGCTT CCAGTCCCGT

610 620 630 640 650 660
CACCTTGCCT GCCTGCCTCG GGAGCCAGGG CTGTGCACCT GGCAGTCCCT GCGGTCCCAG

670 680 690 700 710 720
ATAGCCTGAA TCCTGCCCGG AGTGGAAGCT GAAGCCTGCA CAGTGTCCAC CCTGTTCCCA

730 740 750 760 770 780
CTCCCATCTT TCTTCCGGAC AATGAAATAA AGAGTTACCA CCCAGCAAAA AAAAAAAGGA

This third sequence contains the 5' nontranslated region of the second preferred sequence and the 3' region of the first preferred sequence. It is envisioned that this third preferred sequence is capable of directing intracellular production of a metalloproteinase analogous to a mature human collagenase inhibitor in a microbial or mammalian expression system.

Currently, for expression of the instant metalloproteinase inhibitors in animal cells, the inventors most prefer a method which utilizes a fourth preferred DNA sequence. The coding strand of this sequence reads as follows:

10 20 30 40 50 60
GGCCATCGCC GCAGATCCAG CGCCCAGAGA GACACCAGAG AACCCACCAT GGCCCCCTTT

70 80 90 100 110 120
GAGCCCCTGG CTTCTGGCAT CCTGTTGTTG CTGTGGCTGA TAGCCCCCAG CAGGGCCTGC

130 140 150 160 170 180
ACCTGTGTCC CACCCCACCC ACAGACGGCC TTCTGCAATT CCGACCTCGT CATCAGGGCC

190 200 210 220 230 240
AAGTTCGTGG GGACACCAGA AGTCAACCAG ACCACCTTAT ACCAGCGTTA TGAGATCAAG

250 260 270 280 290 300
ATGACCAAGA TGTATAAAGG GTTCCAAGCC TTAGGGGATG CCGCTGACAT CCGGTTCGTC

310 320 330 340 350 360
TACACCCCCG CCATGGAGAG TGTCTGCGGA TACTTCCACA GGTCCCACAA CCGCAGCGAG

370 380 390 400 410 420
GAGTTTCTCA TTGCTGGAAA ACTGCAGGAT GGACTCTTGC ACATCACTAC CTGCAGTTTC

430 440 450 460 470 480
GTGGCTCCCT GGAACAGCCT GAGCTTAGCT CAGCGCCGGG GCTTCACCAA GACCTACACT

490 500 510 520 530 540
GTTGGCTGTG AGGAATGCAC AGTGTTTCCC TGTTTATCCA TCCCCTGCAA ACTGCAGAGT

550 560 570 580 590 600
GGCACTCATT GCTTGTGGAC GGACCAGCTC CTCCAAGGCT CTGAAAAGGG CTTCCAGTCC

610 620 630 640 650 660
CGTCACCTTG CCTGCCTGCC TCGGGAGCCA GGGCTGTGCA CCTGGCAGTC CCTGCGGTCC

670 680 690 700 710 720
CAGATAGCCT GAATCCTGCC CGGAGTGGAA GCTGAAGCCT GCACAGTGTC CACCCTGTTC

730 740 750 760 770 780
CCACTCCCAT CTTTCTTCCG GACAATGAAA TAAAGAGTTA CCACCCAGCA AAAAAAAAAA

It must be borne in mind in the practice of the present invention that the alteration of some amino acids in a protein sequence may not affect the fundamental properties of the protein. Therefore, it is also contemplated that other portable DNA sequences, both those capable of directing intracellular production of identical amino acid sequences and those capable of directing intracellular production of analogous amino acid sequences which also possess metalloproteinase inhibitor activity, are included within the ambit of the present invention.

It is contemplated that some of these analogous amino acid sequences will be substantially homologous to native human metalloproteinase inhibitors while other amino acid sequences, capable of functioning as metalloproteinase inhibitors, will not exhibit substantial homology to native inhibitors. By "substantial homology," as used herein, is meant a degree of homology to a native metalloproteinase inhibitor in excess of 50%, preferably in excess of 60%, preferably in excess of 80%. The percentage homology as discussed herein is calculated as the percentage of amino acid residues found in the smaller of the two sequences that align with identical amino acid residues in the sequence being compared when four gaps in a length of 100 amino acids may be introduced to assist in that alignment as set forth by Dayhoff, M.O. in Atlas of Protein Sequence and Structure Vol. 5, p. 124 (1972), National Biochemical Research Foundation, Wash - ington, D.C., specifically incorporated herein by reference.

As noted above, the portable DNA sequences of the present invention may be synthetically created. It is believed that the means for synthetic creation of these polynucleotide sequences are generally known to one of ordinary skill in the art, particularly in light of the teachings contained herein. As an example of the current state of the art relating to polynucleotide synthesis, one is directed to Matteucci, M.D. and Caruthers, M.H., in J. Am. Chem. Soc. 103: 3185 (1981) and Beaucage, S.L. and Caruthers, M.H. in Tetrahedron Lett. 22: 1859 (1981), specifically incorporated herein by reference.

Additionally, the portable DNA sequence may be a fragment of a natural sequence, i.e., a fragment of a polynucleotide which occurred in nature and which has been isolated and purified for the first time by the present inventors. In one embodiment, the portable DNA sequence is a restriction fragment isolated from a cDNA library. In this preferred embodiment, the cDNA library is created from human skin fibroblasts.

In an alternative embodiment, the portable DNA sequence is isolated from a human genomic library. An example of such a library useful in this embodiment is set forth in Lawn et al. Cell 15: 1157-1174 (1978), specifically incorporated herein by reference.

As also noted above, the present invention relates to a series of vectors, each containing at least one of the portable DNA sequences described herein. It is contemplated that additional copies of the portable DNA sequence may be included in a single vector to increase a host microorganism's ability to produce large quantities of the desired metalloproteinase inhibitor.

In addition, the cloning vectors within the scope of the present invention may contain supplemental nucleotide sequences preceding or subsequent to the portable DNA sequence. These supplemental sequences are those that will not interfere with transcription of the portable DNA sequence and will, in some instances as set forth more fully hereinbelow, enhance transcription, translation, or the ability of the primary amino acid structure of the resultant metalloproteinase inhibitor to assume an active, tertiary form.

A preferred vector of the present invention is set forth in Figure 1. This vector, pUC9-F5/237P10, contains the preferred nucleotide sequence set forth above. Vector pUC9 - F5/237P10 is present in the C600/pUC9 - F5/237P10 cells on deposit in the American Type Culture Collection in Rockville, Maryland under Accession No. 53003.

A preferred nucleotide sequence encoding the metalloproteinase inhibitor is identified in Figure 1 as region A. Plasmid pUC9-F5/237P10 also contains supplemental nucleotide sequences preceding and subsequent to the preferred portable DNA sequence in region A. These supplemental sequences are identified as regions B and C, respectively.

In alternate preferred embodiments, either one or both of the preceding or subsequent supplemental sequences may be removed from the vector of Fig. 1 by treatment of the vector with restriction endonucleases appropriate for removal of the supplemental sequences. The supplemental sequence subsequent to the portable DNA sequence, identified in Fig. 1 as region C, may be removed by treatment of the vector with a suitable restriction endonuclease, preferably HgiAl followed by reconstruction of the 3' end of region A using synthetic oligonucleotides and ligation of the vector with T - 4 DNA ligase. Deletion of the supplemental sequence preceding the portable DNA sequence, identified as region B in Fig. 1, would be specifically accomplished by the method set forth in Example 2.

In preferred embodiments, cloning vectors containing and capable of expressing the portable DNA sequence of the present invention contain various operational elements. These "operational elements," as discussed herein, include at least one promoter, at least one Shine - Dalgarno sequence, at least one terminator codon. Preferably, these "operational elements" also include at least one operator, at least one leader sequence, and for proteins to be exported from intracellular space, at least one regulator and any other DNA sequences necessary or preferred for appropriate transcription and subsequent translation of the vector DNA.

Additional embodiments of the present invention are envisioned as employing other known or currently undiscovered vectors which would contain one or more of the portable DNA sequences described herein. In particular, it is preferred that these vectors have some or all of the following characteristics: (1) possess a minimal number of host-organism sequences; (2) be stable in the desired host; (3) be capable of being present in a high copy number in the desired host; (4) possess a regulatable promoter; (5) have at least one DNA sequence coding for a selectable trait present on a portion of the plasmid separate from that where the portable DNA sequence will be inserted; and (6) be integrated into the vector.

The following, noninclusive, list of cloning vectors is believed to set forth vectors which can easily be altered to meet the above-criteria and are therefore preferred for use in the present invention. Such alterations are easily performed by those of ordinary skill in the art in light of the available literature and the teachings herein.

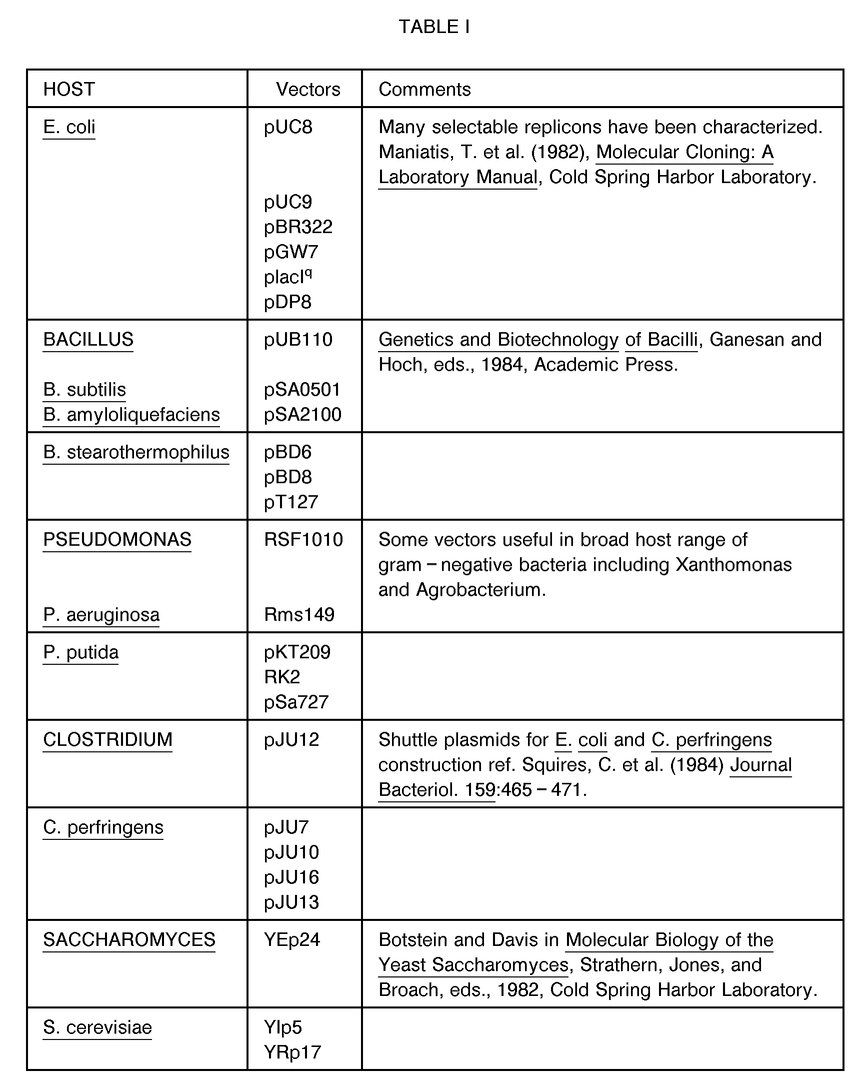

It is to be understood that additional cloning vectors may now exist or will be discovered which have the above-identified properties and are therefore suitable for use in the present invention. These vectors are also contemplated as being within the scope of the disclosed series of cloning vectors into which the portable DNA sequences may be introduced, along with any necessary operational elements, and which altered vector is then included within the scope of the present invention and would be capable of being used in the recombinant - DNA method set forth more fully below.

In addition to the above list, an E. coli vector system, as set forth in Example 2, is preferred in one embodiment as a cloning vector. Moreover, several vector plasmids which autonomously replicate in a broad range of Gram Negative bacteria are preferred for use as cloning vehicles in hosts of the genera Pseudomonas. These are described by Tait, R.C., Close, T.J., Lundquist, R.C., Hagiya, M., Rodriguez, R.L., and Kado, C.I. in Biotechnology, May, 1983, pp. 269-275; Panopoulos, N.J. in Genetic Engineering in the Plant Sciences, Praeger Publishers, New York, New York, pp. 163 - 185, (1981); and Sakaguchi, K. in Current Topic in Microbiology and Immunology 96:31 -45, (1982), each of which is specifically incorporated herein by reference.

One particularly preferred construction employs the plasmid RSF1010 and derivatives thereof as described by Bagdasarian, M., Bagdasarian, M.M., Coleman, S., and Timmis, K.N. in Plasmids of Medical Environmental and Commercial Importance, Timmis, K.N. and Puhler, A. eds., Elsevier/North Holland Biomedical Press, (1979), specifically incorporated herein by reference. The advantages of RSF1010 are that it is relatively small, high copy number plasmid which is readily transformed into and stably maintained in both E. coli and Pseudomonas species. In this system, it is preferred to use the Tac expression system as described for Escherichia, since it appears that the E. coli trp promoter is readily recognized by Pseudomonas RNA polymerase as set forth by Sakaguchi, K. in Current Topics in Microbiology and Immunology 96:31 -45 (1982) and Gray, G.L., McKeown, K.A., Jones, A.J.S., Seeburg, P.H., and Heyneker, H.L. in Biotechnology Feb. 1984, pp. 161 - 165, both of which are specifically incorporated herein by reference. Transcriptional activity may be further maximized by requiring the exchange of the promoter with, e.g., an E. coli or P. aeruginosa trp promoter.

In a preferred embodiment, P. aeruginosa is transformed with vectors directing the synthesis of the metalloproteinase inhibitor as either an intracellular product or as a product coupled to leader sequences that will effect its processing and export from the cell. In this embodiment, these leader sequences are preferably selected from the group consisting of betalactamase, OmpA protein, the naturally occurring human signal peptide, and that of carboxypeptidase G2 from Pseudomonas. Translation may be coupled to translation initiation for any of the E. coli proteins as described in Example 2, as well as to initiation sites for any of the highly expressed proteins of the host to cause intracellular expression of the metalloproteinase inhibitor.

In those cases where restriction minus strains of a host Pseudomonas species are not available, transformation efficiency with plasmid constructs isolated from E. coli are poor. Therefore, passage of the Pseudomonas cloning vector through an r - m + strain of another species prior to transformation of the desired host, as set forth in Bagdasarian, M., et al., Plasmids of Medical, Environmental and Commercial Importance, pp. 411 -422, Timmis and Puhler eds., Elsevier/North Holland Biomedical Press (1979), specifically incorporated herein by reference, is desired.

Furthermore, a preferred expression system in hosts of the genera Bacillus involves using plasmid pUB110 as the cloning vehicle. As in other host vector systems, it is possible in Bacillus to express the metalloproteinase inhibitors of the present invention as either an intracellular or a secreted protein. The present embodiments include both systems. Shuttle vectors that replicate in both Bacillus and E. coli are available for constructing and testing various genes as described by Dubnau, D., Gryczan, T., Contente, S., and Shivakumar, A.G. in Genetic Engineering, Vol. 2, Setlow and Hollander eds., Plenum Press, New York, New York, pp. 115-131, (1980), specifically incorporated herein by reference. For the expression and secretion of metalloproteinase inhibitors from B. subtilis, the signal sequence of alpha-amylase is preferably coupled to the coding region for the metalloproteinase inhibitor. For synthesis of intracellular metalloproteinase inhibitor, the portable DNA sequence will be translationally coupled to the ribosome binding site of the alpha - amylase leader sequence.

Transcription of either of these constructs is preferably directed by the alpha-amylase promoter or a derivative thereof. This derivative contains the RNA polymerase recognition sequence of the native alpha-amylase promoter but incorporates the lac operator region as well. Similar hybrid promoters constructed from the penicillinase gene promoter and the lac operator have been shown to function in Bacillus hosts in a regulatable fashion as set forth by Yansura, D.G. and Henner in Genetics and Biotechnology of Bacilli, Ganesan, A.T. and Hoch, J.A., eds., Academic Press, pp. 249-263, (1984), specifically incorporated by reference. The lacl gene of lacl^{q} would also be included to effect regulation.

One preferred construction for expression in Clostridium is in plasmid pJU12 described by Squires, C. H. et al in J. Bacteriol. 159:465 - 471 (1984), specifically incorporated herein by reference, transformed into C. perfringens by the method of Heefner, D. L. et al. as described in J. Bacteriol. 159:460-464 (1984), specifically incorporated herein by reference. Transcription is directed by the promoter of the tetracycline resistance gene. Translation is coupled to the Shine - Dalgarno sequences of this same tet' gene in a manner strictly analogous to the procedures outlined above for vectors suitable for use in other hosts.

Maintenance of foreign DNA introduced into yeast can be effected in several ways (Botstein, D., and Davis, R. W., in The Molecular Biology of the Yeast Saccharomyces, Cold Spring Harbor Laboratory, Strathern, Jones and Broach, eds., pp. 607 - 636 (1982). One preferred expression system for use with host organisms of the genus Saccharomyces harbors the anticollagenase gene on the 2 micron plasmid. The advantages of the 2 micron circle include relatively high copy number and stability when introduced into cir strains. These vectors preferably incorporate the replication origin and at least one antibiotic resistance marker from pBR322 to allow replication and selection in E. coli. In addition, the plasmid will preferably have 2 micron sequences and the yeast LEU2 gene to serve the same purposes in LEU2 mutants of yeast.

The regulatable promoter from the yeast GAL1 gene will preferably be adapted to direct transcription of the portable DNA sequence gene. Translation of the portable DNA sequence in yeast will be coupled to the leader sequence that directs the secretion of yeast alpha-factor. This will cause formation of a fusion protein which will be processed in yeast and result in secretion of a metalloproteinase inhibitor. Alternatively, a methionylmetalloproteinase inhibitor will be translated for inclusion within the cell.

It is anticipated that translation of mRNA coding for the metalloproteinase inhibitor in yeast will be more efficient with the preferred codon usage of yeast than with the sequence present in pUC8 - Fic, as identified in Example 2, which has been tailored to the prokaryotic bias. For this reason, the portion of the 5' end of the portable DNA sequence beginning at the Tth1111 site is preferably resynthesized. The new sequence favors the codons most frequently used in yeast. This new sequence preferably has the following nucleotide sequence:

HgiAI
5'   GAT CCG TGC ACT TGT GTT CCA CCA CAC
        GC ACG TGA ACA CAA GGT GGT GTG

     CCA CAA ACT GCT TTC TGT AAC TCT GAC C
     GGT GTT TGA CGA AAG ACA TTG AGA CTG GA   3'

As will be seen from an examination of the individual cloning vectors and systems contained on the above list and description, various operational elements may be present in each of the preferred vectors of the present invention. It is contemplated any additional operational elements which may be required may be added to these vectors using methods known to those of ordinary skill in the art, particularly in light of the teachings herein.

In practice, it is possible to construct each of these vectors in a way that allows them to be easily isolated, assembled, and interchanged. This facilitates assembly of numerous functional genes from combinations of these elements and the coding region of the metalloproteinase inhibitor. Further, many of these elements will be applicable in more than one host.

At least one origin of replication recognized by the contemplated host microorganism, along with at least one selectable marker and at least one promoter sequence capable of initiating transcription of the portable DNA sequence are contemplated as being included in these vectors. It is additionally contemplated that the vectors, in certain preferred embodiments, will contain DNA sequences capable of functioning as regulators ("operators"), and other DNA sequences capable of coding for regulator proteins. In preferred vectors of this series, the vectors additionally contain ribosome binding sites, transcription terminators and leader sequences.

These regulators, in one embodiment, will serve to prevent expression of the portable DNA sequence in the presence of certain environmental conditions and, in the presence of other environmental conditions, allow transcription and subsequent expression of the protein coded for by the portable DNA sequence. In particular, it is preferred that regulatory segments be inserted into the vector such that expression of the portable DNA sequence will not occur in the absence of, for example, isopropylthio - {3 d - galactoside. In this situation, the transformed microorganisms containing the portable DNA may be grown to a desired density prior to initiation of the expression of the metalloproteinase inhibitor. In this embodiment, expression of the desired protease inhibitor is induced by addition of a substance to the microbial environment capable of causing expression of the DNA sequence after the desired density has been achieved.

Additionally, it is preferred that an appropriate secretory leader sequence be present, either in the vector or at the 5' end of the portable DNA sequence, the leader sequence being in a position which allows the leader sequence to be immediately adjacent to the initial portion of the nucleotide sequence capable of directing expression of the protease inhibitor without any intervening transcription or translation termination signals. The presence of the leader sequence is desired in part for one or more of the following reasons: 1) the presence of the leader sequence may facilitate host processing of the initial product to the mature recombinant metalloproteinase inhibitor; 2) the presence of the leader sequence may facilitate purification of the recombinant metalloproteinase inhibitors, through directing the metalloproteinase inhibitor out of the cell cytoplasm; 3) the presence of the leader sequence may affect the ability of the recombinant metal - loproteinase inhibitor to fold to its active structure through directing the metalloproteinase inhibitor out of the cell cytoplasm.

In particular, the leader sequence may direct cleavage of the initial translation product by a leader peptidase to remove the leader sequence and leave a polypeptide with the amino acid sequence which has the potential of metalloproteinase inhibitory activity. In some species of host microorganisms, the presence of the appropriate leader sequence will allow transport of the completed protein into the periplasmic space, as in the case of E. coli. In the case of certain yeasts and strains of Bacillus and Pseudomonas, the appropriate leader sequence will allow transport of the protein through the cell membrane and into the extracellular medium. In this situation, the protein may be purified from extracellular protein.

Thirdly, in the case of some of the metalloproteinase inhibitors prepared by the present invention, the presence of the leader sequence may be necessary to locate the completed protein in an environment where it may fold to assume its active structure, which structure possesses the appropriate metal - loproteinase activity.

Additional operational elements include, but are not limited to, ribosome - binding sites and other DNA sequences necessary for microbial expression of foreign proteins. The operational elements as discussed herein can be routinely selected by those of ordinary skill in the art in light of prior literature and the teachings contained herein. General examples of these operational elements are set forth in B. Lewin, Genes, Wiley & Sons, New York (1983), which is specifically incorporated herein by reference. Various examples of suitable operational elements may be found on the vectors discussed above and may be elucidated through review of the publications discussing the basic characteristics of the aforementioned vectors.

In one preferred embodiment of the present invention, an additional DNA sequence is located immediately preceding the portable DNA sequence which codes for the metalloproteinase inhibitor. The additional DNA sequence is capable of functioning as a translational coupler, i.e., it is a DNA sequence that encodes an RNA which serves to position ribosomes immediately adjacent to the ribosome binding site of the metalloproteinase inhibitor RNA with which it is contiguous.

Upon synthesis and/or isolation of all necessary and desired component parts of the above - discussed cloning vectors, the vectors are assembled by methods generally known to those of ordinary skill in the art. Assembly of such vectors is believed to be within the duties and tasks performed by those with ordinary skill in the art and, as such, is capable of being performed without undue experimentation. For example, similar DNA sequences have been ligated into appropriate cloning vectors, as set forth in Schoner et al., Proceedings of the National Academy of Sciences U.S.A., 81 :5403 - 5407 (1984), which is specifically incorporated herein by reference.

In construction of the cloning vectors of the present invention, it should additionally be noted that multiple copies of the portable DNA sequence and its attendant operational elements may be inserted into each vector. In such an embodiment, the host organism would produce greater amounts per vector of the desired metalloproteinase inhibitor. The number of multiple copies of the DNA sequence which may be inserted into the vector is limited only by the ability of the resultant vector, due to its size, to be transferred into and replicated and transcribed in an appropriate host microorganism.

Additionally, it is preferred that the cloning vector contain a selectable marker, such as a drug resistance marker or other marker which causes expression of a selectable trait by the host microorganism. In a particularly preferred embodiment of the present invention, the gene for ampicillin resistance is included in vector pUC9-F5/237P10.

Such a drug resistance or other selectable marker is intended in part to facilitate in the selection of transformants. Additionally, the presence of such a selectable marker on the cloning vector may be of use in keeping contaminating microorganisms from multiplying in the culture medium. In this embodiment, such a pure culture of the transformed host microorganisms would be obtained by culturing the microorganisms under conditions which require the induced phenotype for survival.

It is noted that, in preferred embodiment, it is also desirable to reconstruct the 3' end of the coding region to allow assembly with 3' non-translated sequences. Included among these non-translated sequences are those which stabilize the mRNA or enhance its transcription and those that provide strong transcriptional termination signals which may stabilize the vector as they are identified by Gentz, R., Langner, A., Chang, A.C.Y., Cohen, S.H., and Bujard, H. in Proc. Natl. Acad. Sci. USA 78:4936-4940 (1981), specifically incorporated herein by reference.

This invention also relates to a recombinant-DNA method for the production of metallproteinase inhibitors. Generally, this method includes:
(a) preparation of a portable DNA sequence capable of directing a host microorganism to produce a protein having metalloproteinase inhibitor activity;
(b) cloning the portable DNA sequence into a vector capable of being transferred into and replicating in a host microorganism, such vector containing operational elements for the portable DNA sequence;
(c) transferring the vector containing the portable DNA sequence and operational elements into a host microorganism capable of expressing the metalloproteinase inhibitor protein;
(d) culturing the host microorganism under conditions appropriate for amplification of the vector and expression of the inhibitor; and
(e) in either order:
   (i) harvesting the inhibitor; and
   (ii) causing the inhibitor to assume an active, tertiary structure whereby it possesses metalloproteinase inhibitor activity.

In this method, the portable DNA sequences are those synthetic or naturally - occurring polynucleotides described above. In a preferred embodiment of the present method, the portable DNA sequence has the nucleotide sequence as follows:

10 20 30 40 50 60
GTTGTTGCTG TGGCTGATAG CCCCAGCAGG GCCTGCACCT GTGTCCCACC CCACCCACAG

70 80 90 100 110 120
ACGGCCTTCT GCAATTCCGA CCTCGTCATC AGGGCCAAGT TCGTGGGGAC ACCAGAAGTC

130 140 150 160 170 180
AACCAGACCA CCTTATACCA GCGTTATGAG ATCAAGATGA CCAAGATGTA TAAAGGGTTC

190 200 210 220 230 240
CAAGCCTTAG GGGATGCCGC TGACATCCGG TTCGTCTACA CCCCCGCCAT GGAGAGTGTC

250 260 270 280 290 300
TGCGGATACT TCCACAGGTC CCACAACCGC AGCGAGGAGT TTCTCATTGC TGGAAAACTG

310 320 330 340 350 360
CAGGATGGAC TCTTGCACAT CACTACCTGC AGTTTCGTGG CTCCCTGGAA CAGCCTGAGC

370 380 390 400 410 420
TTAGCTCAGC GCCGGGGCTT CACCAAGACC TACACTGTTG GCTGTGAGGA ATGCACAGTG

430 440 450 460 470 480
TTTCCCTGTT TATCCATCCC CTGC[?]AACTG CAGAGTGGCA CTCATTGCTT GTGGACGGAC

490 500 510 520 530 540
CAGCTCCTCC AAGGCTCTGA AAAGGGCTTC CAGTCCCGTC ACCTTGCCTG CCTGCCTCGG

550 560 570 580 590 600
GAGCCAGGGC TGTGCACCTG GCAGTCCCTG CGGTCCCAGA TAGCCTGAAT CCTGCCCGGA

610 620 630 640 650 660
GTGGAAGCTG AAGCCTGCAC AGTGTCCACC CTGTTCCCAC TCCCATCTTT CTTCCGGACA

670 680 690 700
ATGAAATAAA GAGTTACCAC CCAGCAAAAA AAAAAAGGAA TTC

The vectors contemplated as being useful in the present method are those described above. In a preferred embodiment, the cloning vector pUC9 - F5/237P1 0 is used in the disclosed method.

The vector thus obtained is then transferred into the appropriate host microorganism. It is believed that any microorganism having the ability to take up exogenous DNA and express those genes and attendant operational elements may be chosen. It is preferred that the host microorganism be an anaerobe, facultative anaerobe or aerobe. Particular hosts which may be preferable for use in this method include yeasts and bacteria. Specific yeasts include those of the genus Saccharomyces, and especially Saccharomyces cerevisiae.

Specific bacteria include those of the genera Bacillus and Escherichia and Pseudomonas. Various other preferred hosts are set forth in Table I, supra. In other, alternatively preferred embodiments of the present invention, Bacillus subtilis, Escherichia coli or Pseudomonas aeruginosa are selected as the host microorganisms.

After a host organism has been chosen, the vector is transferred into the host organism using methods generally known by those of ordinary skill in the art. Examples of such methods may be found in Advanced Bacterial Genetics by R. W. Davis et al., Cold Spring Harbor Press, Cold Spring Harbor, New York, (1980), which is specifically incorporated herein by reference. It is preferred, in one embodiment, that the transformation occur at low temperatures, as temperature regulation is contemplated as a means of regulating gene expression through the use of operational elements as set forth above. In another embodiment, if osmolar regulators have been inserted into the vector, regulation of the salt concentrations during the transformation would be required to insure appropriate control of the synthetic genes.

If it is contemplated that the recombinant metalloproteinase inhibitors will ultimately be expressed in yeast, it is preferred that the cloning vector first be transferred into Escherichia coli, where the vector would be allowed to replicate and from which the vector would be obtained and purified after amplification. The vector would then be transferred into the yeast for ultimate expression of the metalloproteinase inhibitor.

The host microorganisms are cultured under conditions appropriate for the expression of the metal - loproteinase inhibitor. These conditions are generally specific for the host organism, and are readily determined by one of ordinary skill in the art, in light of the published literature regarding the growth conditions for such organisms, for example Bergey's Manual of Determinative Bacteriology, 8th Ed., Williams & Wilkins Company, Baltimore, Maryland, which is specifically incorporated herein by reference.

Any conditions necessary for the regulation of the expression of the DNA sequence, dependent upon any operational elements inserted into or present in the vector, would be in effect at the transformation and culturing stages. In one embodiment, the cells are grown to a high density in the presence of appropriate regulatory conditions which inhibit the expression of the DNA sequence. When optimal cell density is approached, the environmental conditions are altered to those appropriate for expression of the portable DNA sequence. It is thus contemplated that the production of the metalloproteinase inhibitor will occur in a time span subsequent to the growth of the host cells to near optimal density, and that the resultant metalloproteinase inhibitor will be harvested at some time after the regulatory conditions necessary for its expression were induced.

In a preferred embodiment of the present invention, the recombinant metalloproteinase inhibitor is purified subsequent to harvesting and prior to assumption of its active structure. This embodiment is preferred as the inventors believe that recovery of a high yield of re-folded protein is facilitated if the protein is first purified. However, in one preferred, alternate embodiment, the metalloproteinase inhibitor may be allowed re-fold to assume its active structure prior to purification. In yet another preferred, alternate embodiment, the metalloproteinase inhibitor is caused to assume its re-folded, active state upon recovery from the culturing medium.

In certain circumstances, the metalloproteinase inhibitor will assume its proper, active structure upon expression in the host microorganism and transport of the protein through the cell wall or membrane or into the periplasmic space. This will generally occur if DNA coding for an appropriate leader sequence has been linked to the DNA coding for the recombinant protein. The preferred metalloprotienase inhibitors of the present invention will assume their mature, active form upon translocation out of the inner cell membrane. The structures of numerous signal peptides have been published, for example by Marion E.E. Watson in Nuc. Acid Res. 12:515 - 5164, 1984, specifically incorporated herein by reference. It is intended that these leader sequences, together with portable DNA, will direct intracellular production of a fusion protein which will be transported through the cell membrane and will have the leader sequence portion cleaved upon release from the cell.

In a preferred embodiment, the signal peptide of the E.coli OmpA protein is used as a leader sequence and is located in a position contiguous with the portable DNA sequence coding for the metalloproteinese inhibitor structure.

Additionally preferred leader sequences include those of beta-lactamase, carboxypeptidase G2 and the human signal protein. These and other leader sequences are described.

If the metalloproteinase inhibitor does not assume its proper, active structure, any disulfide bonds which have formed and/or any noncovalent interactions which have occurred will first be disrupted by denaturing and reducing agents, for example, guanidinium chloride and (3 mercaptoethanol, before the metal - loproteinase inhibitor is allowed to assume its active structure following dilution and oxidation of these agents under controlled conditions.

The transcription terminators contemplated herein serve to stabilize the vector. In particular, those sequences as described by Gentz et al., in Proc. Natl. Acad. Sci. USA 78: 4936 - 4940 (1981), specifically incorporated herein by reference, are contemplated for use in the present invention.

It is to be understood that application of the teachings of the present invention to a specific problem or environment will be within the capabilities of one having ordinary skill in the art in light of the teachings contained herein. Examples of the products of the present invention and representative processes for their isolation and manufacture appear in the following examples.

### EXAMPLES

### EXAMPLE 1

### Preparation of Poly(A⁺) RNA from HEF - SA Fibroblasts

HEF-SA cells were grown to near confluence in 75 cm² T-flasks. Cells were washed twice in Dulbecco's phosphate buffered saline solution and harvested by the addition of 2 ml of 10 mM Tris, pH 7.5 containing 1% w/v SDS (obtained from BDH chemicals, Ltd., Poole, England), 5 mM EDTA and 20 ug/ml protease K (obtained from Boehringer Mannheim Biochemicals, Indianapolis, Indiana). Each flask was subsequently washed with an additional milliliter of this same solution.

The pooled aliquots from the cell harvest were made to 70 ug/ml in protease K and incubated at 40 ° C for 45 minutes. The proteolyzed solution was brought to a NaCl concentration of 150 mM by the addition of 5 M stock and subsequently extracted with an equal volume of phenol:chloroform 1:1. The aqueous phase was reextracted with an equal volume of chloroform. Two volumes of ethanol were added to the aqueous phase and incubated overnight at - 20 ° C. The precipitated nucleic acids were recovered by centrifugation at 17,500 xg for 10 minutes in a Beckman J2-21 centrifuge, Beckman Instruments, Palo Alto, California, and were redissolved in 25 ml of 0.1% w/v SDS. This solution was again extracted with an equal volume of chloroform. The aqueous phase was added to two volumes of cold ethanol and kept at - -20°C for 2 hours. The precipitate was collected by centrifugation at 10,000 xg for 15 minutes and redissolved in 10 ml of 1 mM Tris, 0.5 mM EDTA, 0.1 % SDS, pH 7.5. RNA was precipitated from this solution by the addition of 10 ml of 4 M LiCI, 20 mM NaoAc, pH 5.0 and incubated at -20°C for 18 hours. The precipitate was again recovered by centrifugation and washed twice with 2 M LiCl before redissolving in 1 mM Tris, 0.5 mM EDTA, 0.1 % SDS, pH 7.5. This solution was stored at -70°C.

### Chromatography on Oligo dT Cellulose

Total cellular RNA prepared as above was ethanol precipitated and redissolved in 0.5 M NaCI. Five ml of RNA at 0.45 mg/ml were applied to a 1 ml column of washed type VII oligo dT cellulose (obtained from PL Biochemicals, Milwaukee, Wisconsin). The column was then washed with 10 ml of 0.5 M NaCl and eluted with 2.0 ml of sterile H₂0. The eluted poly(A⁺) fraction of RNA was ethanol precipitated and dissolved to give a 1 mg/ml solution in 1 mM Tris, 0.1 mM EDTA, pH 8.0. This was stored at -70°C.

### cDNA Synthesis

Poly(A⁺) RNA was primed with oligo dT (obtained from PL Biochemicals, Milwaukee, Wisconsin) to serve as a template for cDNA synthesis by AMV reverse transcriptase (obtained from Life Sciences, Inc., St. Petersburg, Florida). Following the synthesis reaction, the RNA was hydrolyzed by the addition of 0.1 volume of 3 N NaOH and incubated at 67°C for 10 minutes. The solution was then neutralized and the cDNA purified by gel filtration chromatography on biogel A 1.5 (obtained from BioRad Laboratories, Richmond, California) in a 0.7x25 cm column in a 10 mM Tris, 5 mM EDTA, and 1% SDS, pH 7.5 solution. Fractions containing cDNA were pooled and concentrated by ethanol precipitation. The cDNA was dG tailed and purified by gel filtration using the procedure set forth above. Second strand synthesis was primed with oligo dC and polymerized in an initial reaction with the large (Klenow) fragment of DNA polymerase (obtained from Boehringer Mannheim). Following second strand synthesis, E. coli DNA polymerase I (obtained from Boehringer Mannheim) was added and incubation continued to form blunt ends. The double stranded cDNA was again purified by chromatography. EcoRl restriction sites within the cDNAs were modified by the action of EcoRl methylase, obtained from New England Biolabs, Beverly, Massachusetts. The cDNA was again purified and ligated to synthetic EcoRl linkers. Finally, the ends were then trimmed with the endonuclease and the cDNA purified by gel filtration. This DNA was ligated into a unique EcoRl site in lambda gt10 DNA packaged in vitro and used to infect E. coli strain hflA according to the method set forth by Huynh, T.V., Young, R.A., and Davis, R.W., in DNA Cloning Techniques, A Practical Approach (ed. Glover, D.M.) (IRL Press Oxford), in press, specifically incorporated herein by reference. Approximately 25,000 recombinants were amplified in this manner.

### Screening

Recombinant-phage-containing sequences of interest were selected by their preferential hybridization to synthetic oligonucleotides encoding portions of the primary structure of the desired metalloproteinase inhibitor, hereinafter referred to as FIBAC. These portions of the protein sequence correspond in part to those set forth in the published literature by Stricklin, G.P. and Welgus, H.G., J. Biol. Chem. 258: 12252-12258 (1983), specifically incorporated herein by reference. Recombinant phage were used to infect E. coli strain hflA and plated at a density of approximately 2x10³ pfu/150 mm petri dish. Phage were blotted onto nitrocellulose filters (BA85, Schleicher & Schuell Inc., Keene, New Hampshire), and DNA was denatured and fixed essentially as described by Benton and Davis in Science 196:180-182 (1979) specifically incorporated herein by reference.

Using that procedure, the filters were treated sequentially for 10- 15 minutes each in 0.5 M NaCI, then 1.0 M Tris, 1.5 M NaCl pH 8.0, and finally submerged in 2x SSPE. (2x SSPE is 0.36 M NaCI, 20 mM NaH₂P0₄, 2 mM EDTA pH 7.4). Filters were blotted dry and baked 75° -80° for 3-4 hours. Duplicate filters were made of each plate. Filters were prehybridized for 1 -3 hours at 37° in 5x SSPE containing 0.1x SET, 0.15% NaPPi, and 1x Denhardts solutions. Filters were then hybridized for 72 hours at 37° in this same solution containing 5x10⁵ cpm/ml of 5' end - labeled 51 - mer oligonucleotide specific activity approximately 10⁶ cpm/pmole). Following hybridization, filters were washed six times in 5x SSPE containing 0.1x SET and 0.05% sodium pyrophosphate at 37°, then three times in 2x SSPE at 21 ° . These were then blotted dry and autoradiographed on Kodak XAR-5 5 film at 70 with a Kodak lightening - plus intensifying screen. Signals clearly visible from duplicate filters were used to pick phage for plaque purification. Filter preparations and hybridization procedures for plaque purification steps were the same as above. The washing procedure was simplified to 6 changes of 2x SSPE at 37°. Six isolates purified by repetitive plating were then arranged on a single lawn of E. coli strain C600 for testing with subsequent probes.

Preferential hybridization of the 17-mer to each of the isolates (as opposed to control plaques) was observed under a condition identical to that used for plaque purification. Probe C was used in a similar test, except that the SSPE concentration during hybridization was reduced to 4x. Again, each of the isolates demonstrated stronger hybridization to the probe than did control plaques.

### Phage Purification and cDNA Characterization

Quantities of each of the six isolated phage were made by the plate stock technique and purified by serial CsCl block gradient centrifugation. DNA was extracted from these by dialysis against 50% formamide as described by Davis, R.W., Botstein, D., Roth, J.R., in A Manual for Genetic Engineering: Advanced Bacterial Genetics, 1980, Cold Spring Harbor Laboratory, specifically incorporated herein by reference. DNA from each of the isolates was digested with EcoRI and the products were analyzed by agarose gel electrophoresis. The insert from one of the larger clones, lambda FIBAC 5, was found to lack internal sites for Sall, Hindlll, BamHl, and EcoRl. The cDNA insert was released from lambda FIBAC 5 DNA and the lambda arms digested by co - digesting with these four enzymes. The fragments were then ethanol - precipitated and ligated into the EcoRI site of plasmid pUC9 without further purification. These plasmids were then used to transform E. coli strain JM83. Transformants were selected on ampicillin containing plates. Plasmids from several transformants were purified and characterized on the basis of the EcoRI digestion products. One was selected which had an insert co - migrating with the insert from lambda FIBAC 5 on agarose gel electrophoresis. This plasmid has been named pUC9 - F5/237P10.

### Mapping and Subcloning

The insert in pUC9 - F5/237P10 was mapped with respect to internal Pstl sites. Double digests with EcoRl and Pst demonstrated three internal Pstl recognition sites. The entire insert and the component pieces were subcloned into M13 bacteriophage mp19 and mp18, respectively. Sequencing of the pieces was performed by the dideoxynucleotide method described by Sanger et al. in Sanger, F., Nicklen, S., and Coulson, A.R., Proc. Natl. Acad. Sci. USA 74:5463-5467 (1977), specifically incorporated herein by reference.

The sequence of the DNA insert from pUC9 - F5/237P1 showed an open reading frame which encodes the primary structure of a mature fibroblast collagenase inhibitor biologically equivalent to that isolable from human skin fibroblasts. The salient features of the sequence are:
(1) The insert is flanked by EcoRl restriction sites and by G/C and A/T homopolymeric tracts consistent with the cloning methodology;
(2) The coding strand is presented in the 5' to 3' convention with poly C at the 5' end and poly A at the 3' end, again consistent with the techniques employed;
(3) If the first G in the sequence GTTGTTG immediately adjacent to the 3' end of the poly C tract is considered as nucleotide 1, then an open reading frame is presented which encodes the primary structure of the mature human fibroblast collagenase inhibitor beginning at nucleotide 34 and continuing through nucleotide 585;
(4) The termination codon TGA at nucleotides 586 through 588 defines the carboxy terminus of the translation product which is the same as that of the mature protein;
(5) Nucleotides 1 through 33 define an amino acid sequence which is not found in the primary structure of the processed protein, but which is probably a portion of a leader peptide characteristic of secreted proteins;
(6) The three internal Pstl sites have as their first base nucleotides 298, 327, and 448;
(7) There is a single recognition sequence for the restriction enzyme Tth1111 beginning at nucleotide 78; and
(8) There is a single recognition sequence for the restriction endonuclease Ncol beginning at nucleotide 227.

The sequence of nucleotides 1 through 703 and restriction site analysis are shown.

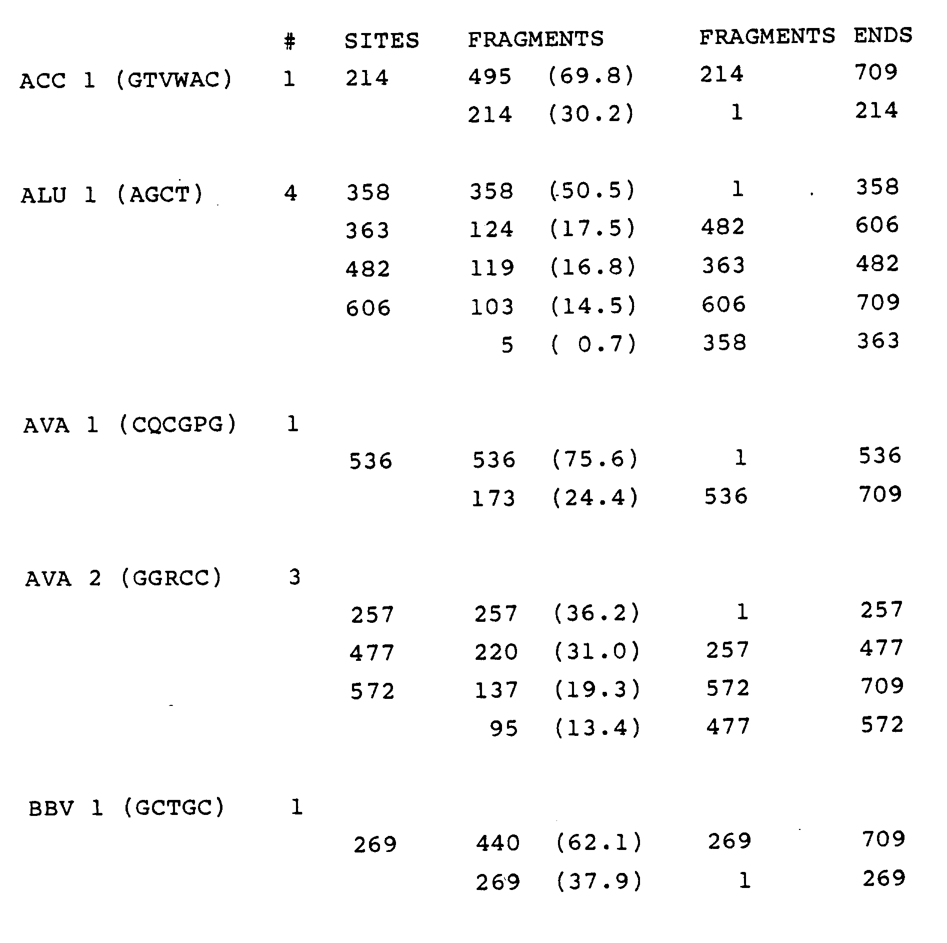

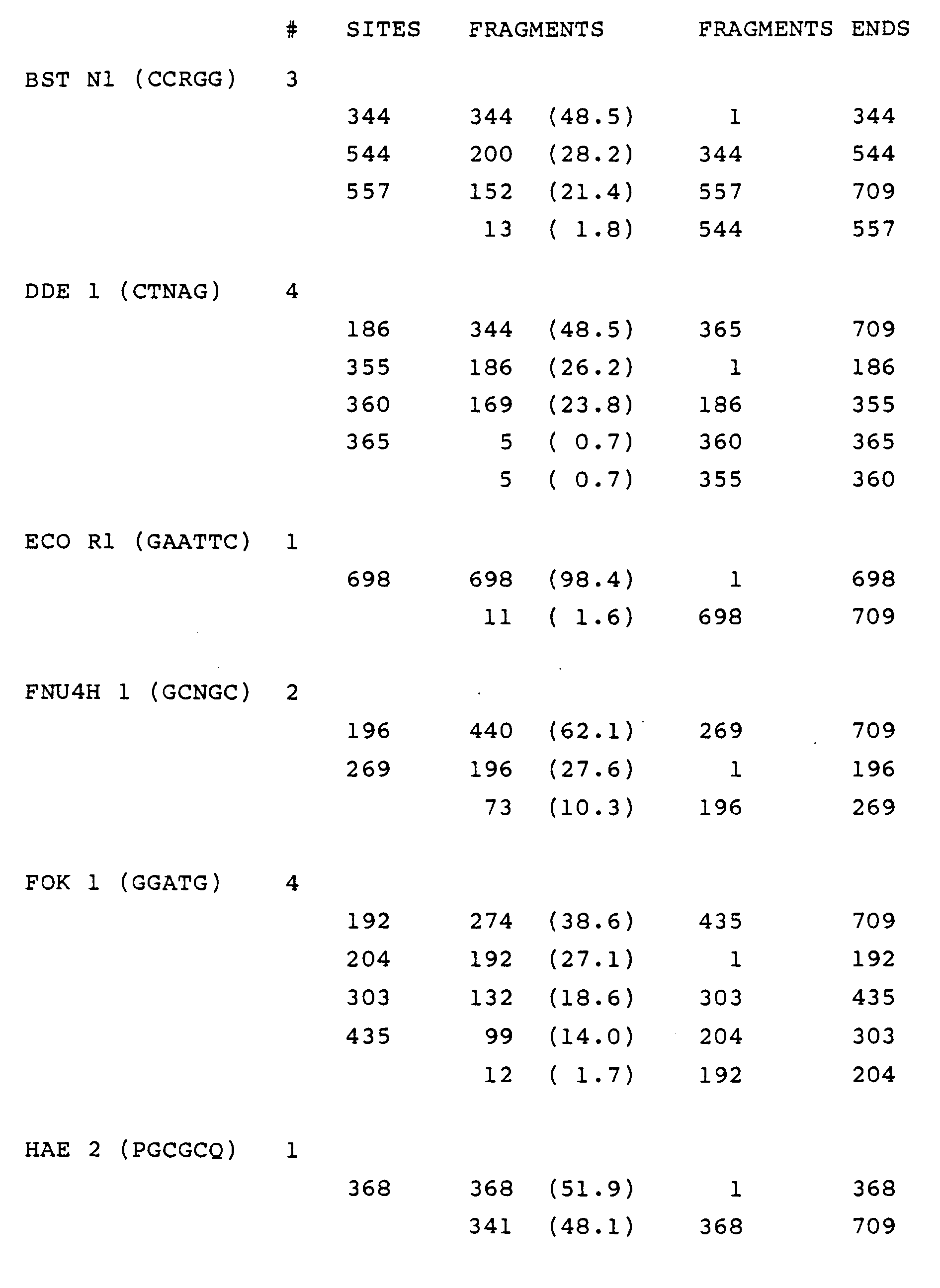

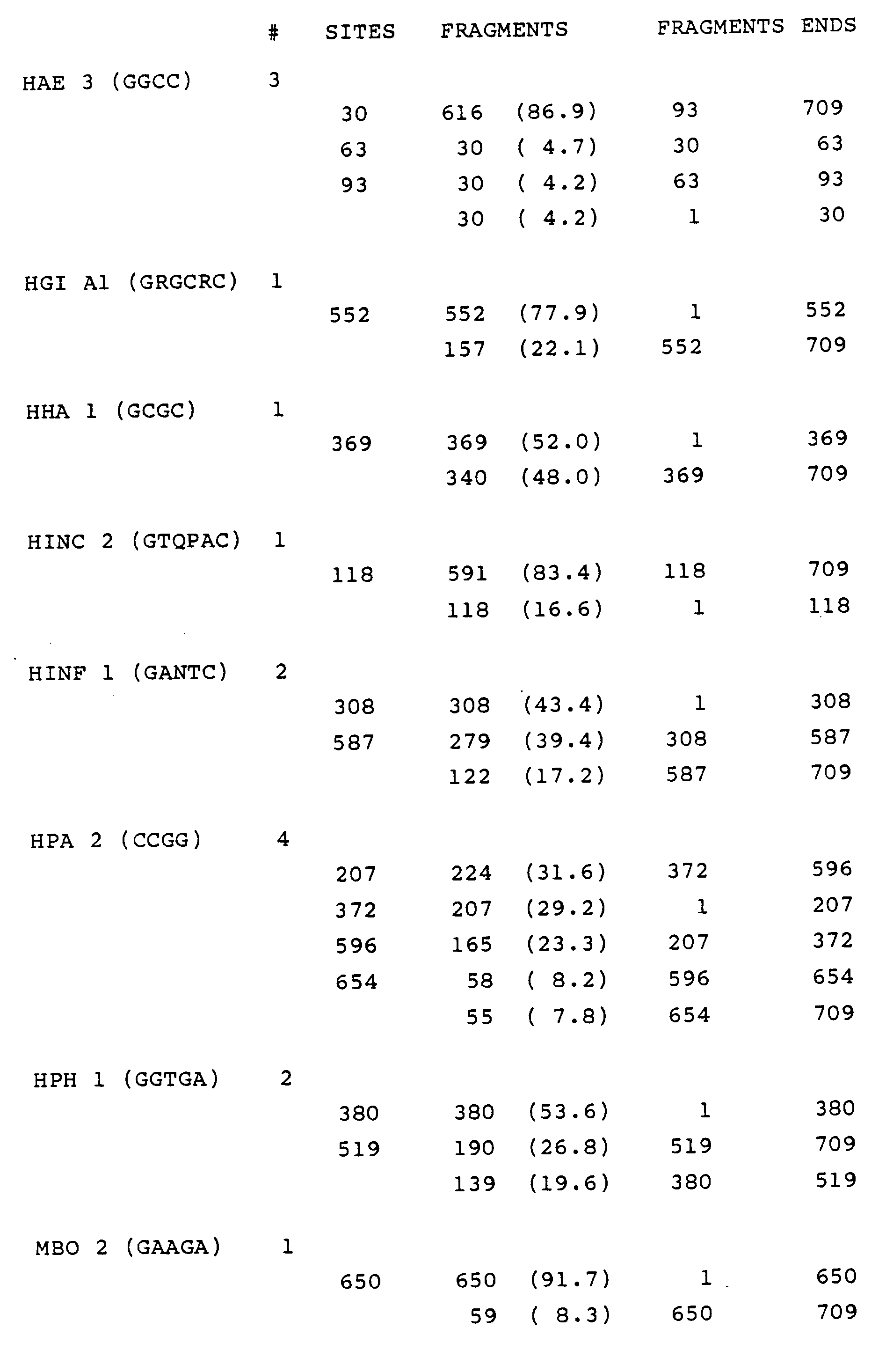

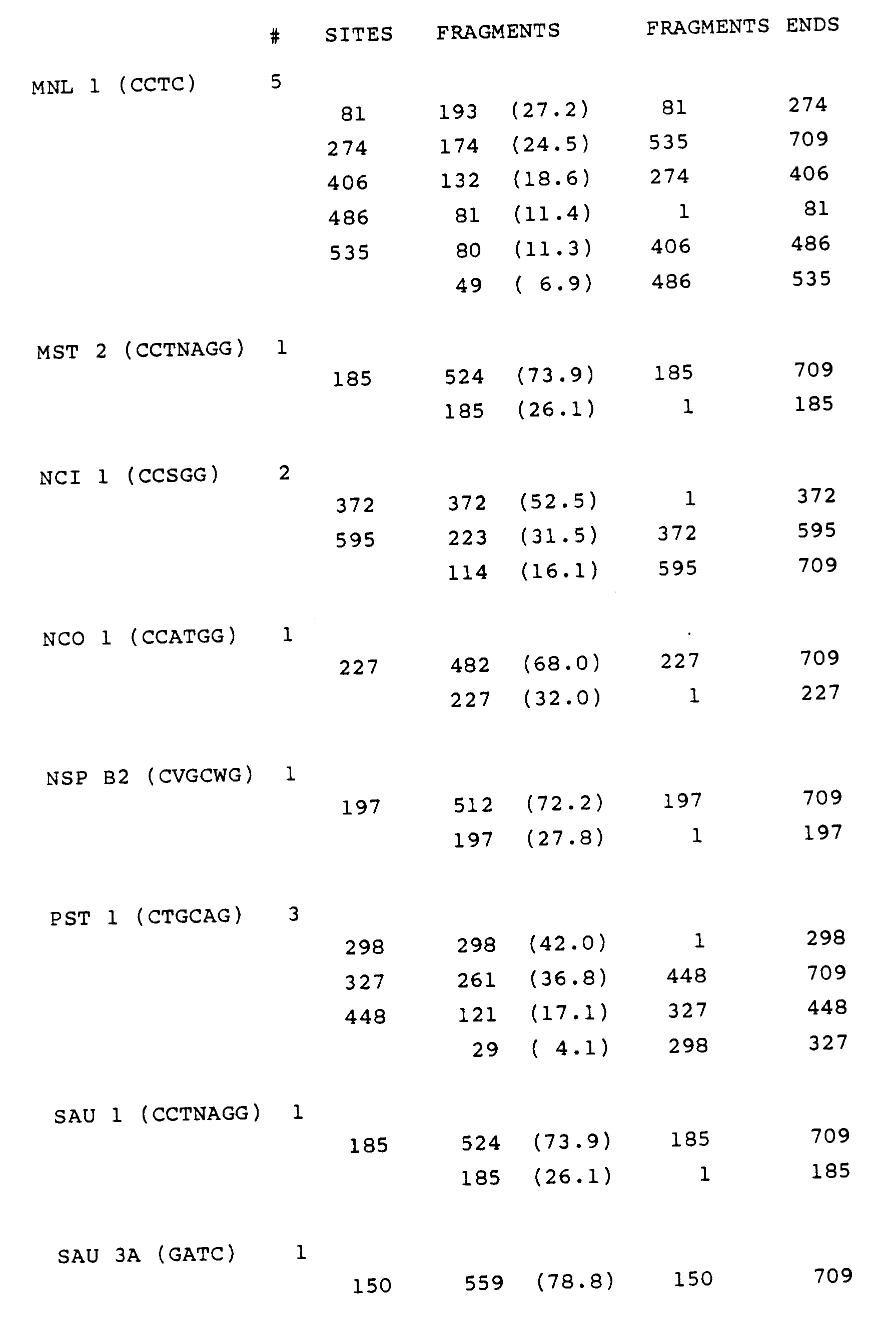

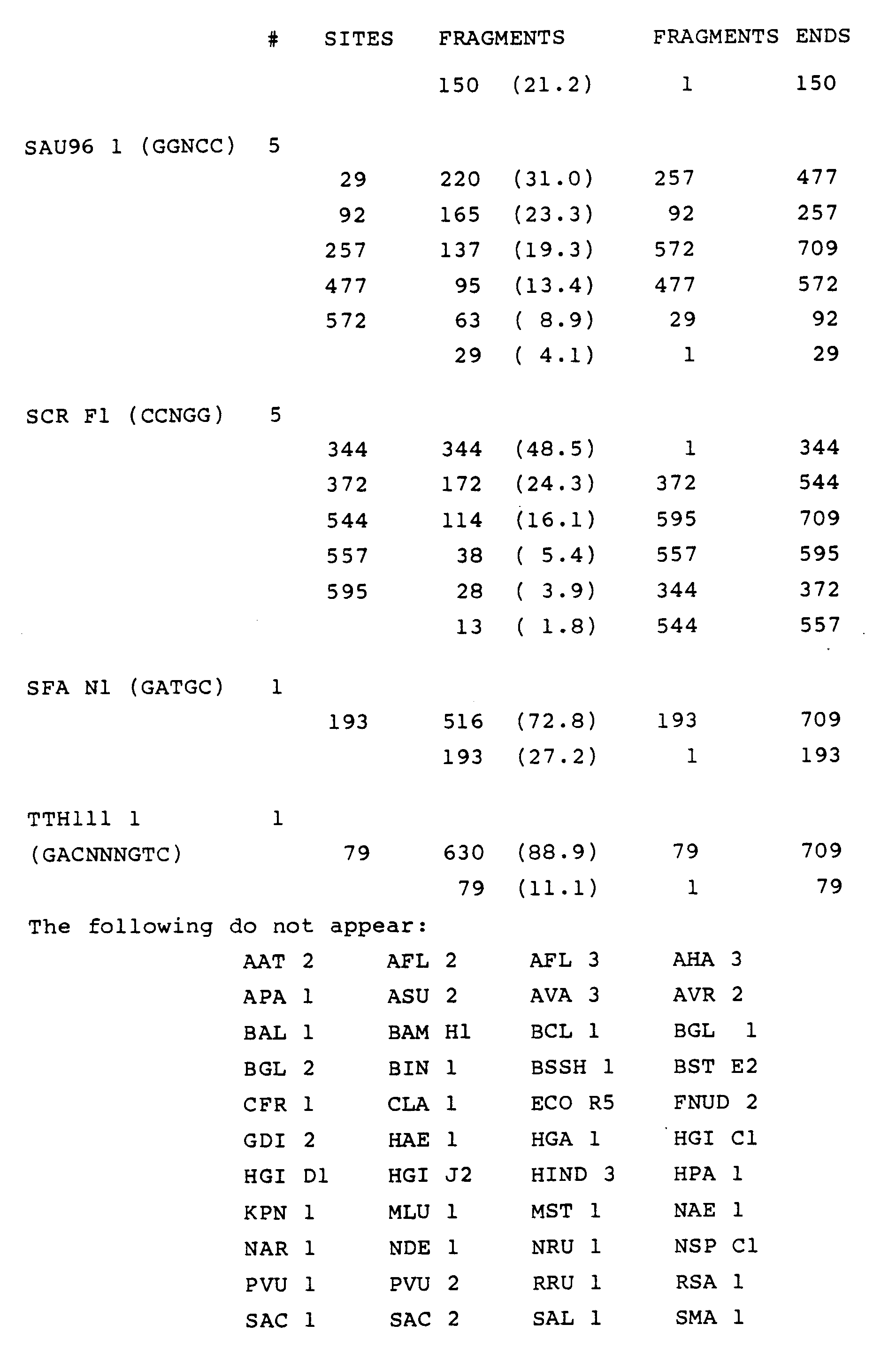

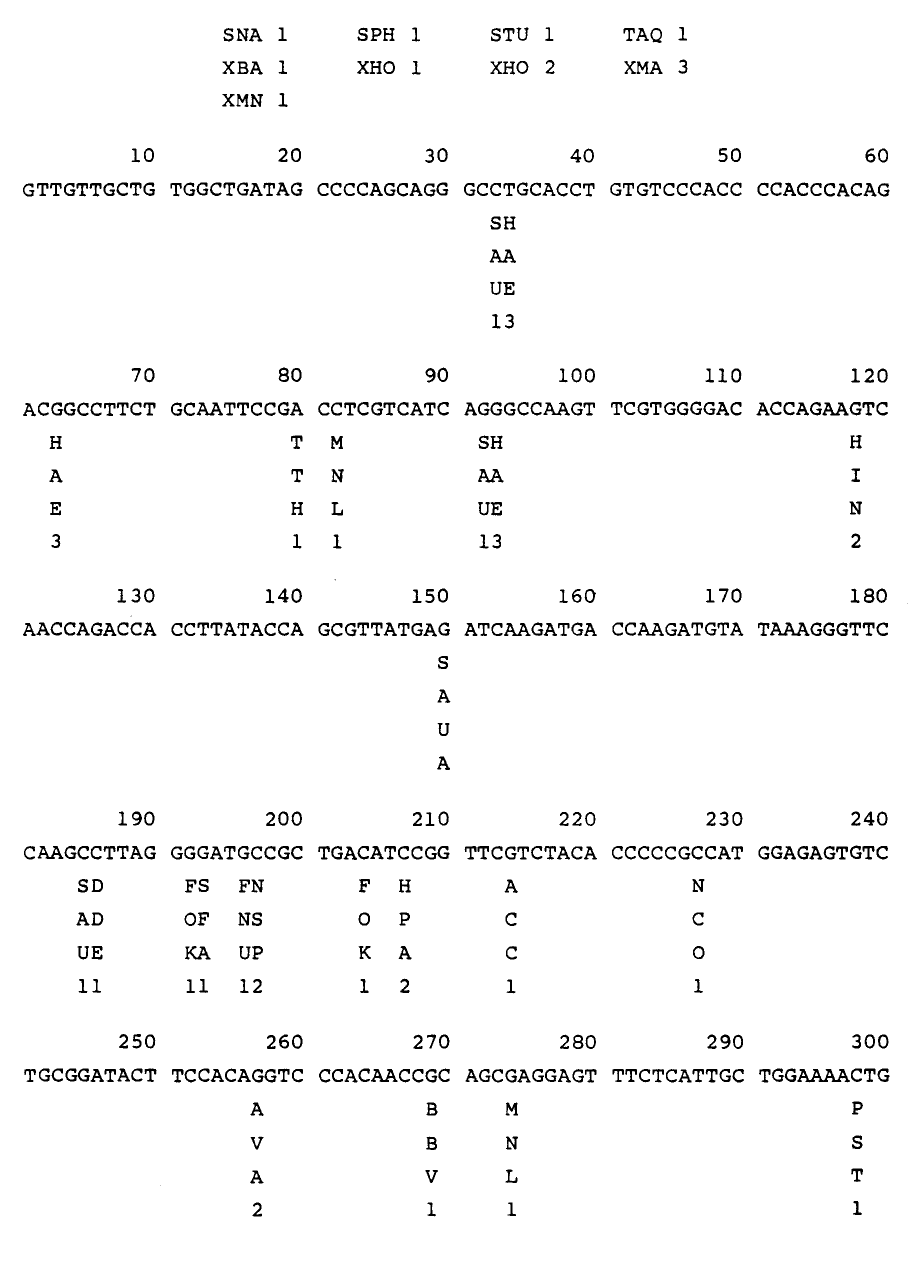

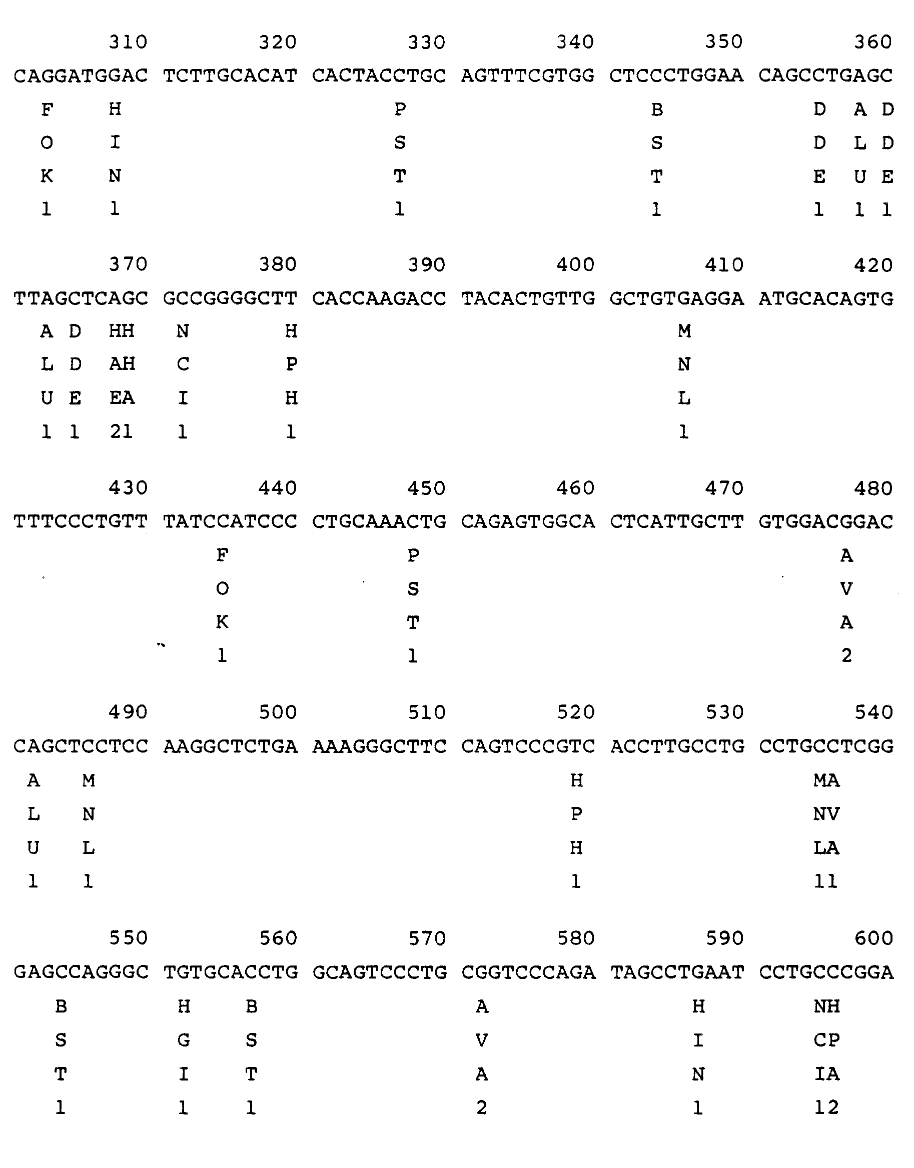

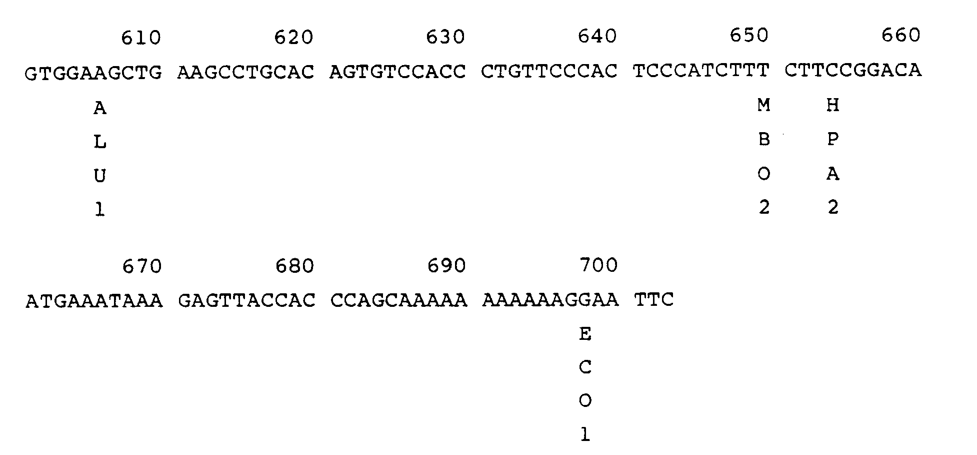

### EXAMPLE 2-EXPRESSION OF COLLAGENESE INHIBITOR IN E. COLI

In this Example, a preferred method of coupling a preferred portable DNA sequence to the 5' end of the cloned cDNA is set forth. This involves making a nucleolytic cleavage at a specified point within the coding sequence and reconstructing the desired portion of the coding sequence by means of synthetic oligonucleotides in a manner that allows its excision and recombination (i.e., by incorporating useful restriction sites).

Trimming the 5' end of the coding region will be accomplished by synthesizing both strands of the DNA extending from the Tth1111 site in the 5' direction and ending in a BamHl overhang. This synthetic oligonucleotide, referred to as FIBAC A, has the following features:
(1) Codon selection has been biased toward those most frequently found in the genes of highly expressed bacterial proteins;
(2) A methionine codon from which to initiate translation has been provided immediately upstream from the cysteine which begins the coding region of human processed FIBAC;
(3) The spacing of the BamHl site to the methionine codon is such that when cloned into pUC8, the coding region of FIBAC will be in-frame with the 5' end of the beta - galactosidase gene;
(4) An in-frame stop codon and Shine Dalgarno sequence are also presented. Translation of this frame for the amino terminal portion of the beta - galactosidase is terminated at the TAA codon, and translation of FIBAC should be initiated at the following ATG;
5) Codons have been selected to create a HgiAl site beginning with the G in the FIBAC initiation codon; and
(6) There is a Pvul site separated by one base from the 3' end of the BamHl sequence. The structure of FIBAC A is

GA TCC GCG ATC GGA GTG TAA GAA ATG TGC ACT
      G CGC TAG CCT CAC ATT CTT TAC ACG TGA

TGC GTT CCG CCG CAT CCG CAG ACT GCT TTC
ACG CAA GGC GGC GTA GGC GTC TGA CGA AAG

TGC AAC TCT GAC C
ACG TTG AGA CTG GA

FIBAC A is synthesized using the ABI DNA synthesizer (Foster City, California) as a series of four component oligonucleotides.

Component oligonucleotide FA1 is:
GATCC GCGAT CGGAG TGTAA GAAAT GTGCA CTTGC

Component oligonucleotide FA2 is:
GGAACG CAAGT GCACA TTTCT TACAC TCCGA TCGCG

Component oligonucleotide FA3 is:
GTTC CGCCG CATCC GCAGA CTGCT TTCTG CAACT CTGAC C

Component oligonucleotide FA4 is:
AGGTC AGAGT TGCAG AAAGC AGTCT GCGGA TGCGG C

The remainder of the coding portion of the FIBAC gene is isolated as the 3' Tth1111 to EcoRI fragment generated by a double digest of pUC9 - F5/237P1 with these enzymes.

A synthetic linker is made to couple the 3' end of the Tth111I to EcoRI fragment to a Sail site. These oligonucleotides will be designed to recreate the Sail site and destroy the EcoRl site. The linker is comprised of the oligonucleotides linker A1 and linker A2.
Linker A1 is: AATTGGCAG
Linker A2 is: TCGACTGCC

These oligonucleotides and oligonucleotides FA1 - FA4 are kinased separately and annealed in equal molar ratios with the Tth111I to EcoRI 3' end of the cDNA and BamHI/Sall cut mp19RF DNA. The ligated DNA is used to transfect JM105. Plaques are picked by their color in the presence of IPTG and X-gal and by hybridization to oligonucleotide FA2. Several positive plaques are to be sequenced. Those containing the designed sequence are subcloned into BamHI/Sall digested pUC8. Translation of the FIBAC gene in this construct is coupled to translation initiated for beta - galactosidase. This expression vector is referred to as pUC8 - Fic.

Coupling translation of FIBAC to translation initiated for other highly expressed proteins is similarly arranged. For example, a portion of the OmpA gene which contains the Shine - Dalgarno and initiator methionine sequences has been synthesized. This sequence encodes the entire signal peptide of OmpA protein and had convenient restriction sites, including those for EcoRl, EcoRV, Pvul, and Stul. The sequence of the sense strand is:

        10         20         30         40         50         60
GAATTCGATA TCTCGTTGGA GATATTCATG ACGTATTTTG GATGATAACG AGGCGCAAAA
E   T E                                   F          M   H
C   A C                                   O          N   H
O   Q O                                   K          L   A
1   1 5                                   1          1   1

        70        80        90       100       110
AATGAAAAAG ACAGCTATCG CGATCGCAGT GGCACTGGCT GGTTTCGCTA CCGTA
            A   NF  PS
            L   RN  VA
            U   UU  UU
            1   12  1A

  120       130
GCGCA GGCCTCTGGT AAAAGCTT
H   S H M         HA
H   T A N         IL
A   U E L         NU
1   1 3 1         31

This sequence is hereinafter referred to as OmpA leader. Coupling the translation of FIBAC to OmpA is accomplished by cutting the pUC8-Fic with Pvul and Sail and isolating the coding region. This, together with the EcoRl to Pvul fragment isolated from OmpA leader, will be cloned into EcoRI/Sall- cut pUC8. As in the prior example, transcription is driven by the lac promoter and regulated by the lac I gene product at the lac operator. This FIBAC expression vector is referred to as pUC8-F/OmpAic.

To effect the translocation of FIBAC across the inner cell membrane, an appropriate leader sequence is added to the amino terminus of FIBAC. The protein thus produced will be translocated and processed to yield the mature form.

To effect such a translocation, a FIBAC gene encoding the signal peptide of the E. coli OmpA protein continuous with the structural region of FIBAC is created. This particular FIBAC gene necessitates having in frame stop codons at the 5' end of the FIBAC coding region changed. To accomplish this, the portion of the 5' coding region from pUC8-Fic that extends from the HgiAl site to the Ncol site is isolated. Upstream sequences are resynthesized as a linker having cohesive ends from BamHl and HgiAl and containing an internal Stul site. This is synthesized as two oligonucleotides, linker B1 and linker B2.
Linker B1 is: GATCCCAGGCCTGCA
Linker B2 is: GGCCTGG

Linkers B1 and B2 are kinased separately and annealed in equal molar ratios with the HgiAl to Ncol fragment described above and BamHI/Ncol cut pUC8-Fic. The resulting construct has the coding sequence of FIBAC in frame with the translation of the amino terminus of beta - galactosidase. Translation of this sequence forms a fusion protein with FIBAC. This plasmid is referred to as pUC8 - Ff.

Attaching the OmpA leader sequences to the coding region of FIBAC is accomplished by ligating EcoRI/Stul cut pUC8 - Ff with an excess of the purified EcoRl to Stul fragment of OmpA leader. Following transformation, plasmids from several colonies will be characterized by hybridization. Those that have incorporated the OmpA leader fragment are characterized further to verify the structure. This plasmid, pUC8 - F OmpA1, will direct the synthesis of a fusion protein beginning in the signal peptide of the E. coli OmpA protein and ending in human FIBAC. The signals present in the OmpA portion of the protein effect the protein's export from the cytoplasm and appropriate cleavage from the primary structure of FIBAC.

If the efficiency of expression were to be compromised by the sequence of the leader peptide or its combination with FIBAC either at the protein or at the nucleic acid level, the gene could be altered to encode any of several known E. coli leader sequences.

Transcription of all of the genes discussed is effected by the lac promoter. As in the case of initiation sites for translation, the promoter and operator region of the gene may be interchanged. FIBAC may also be expressed from vectors incorporating the lambda P_{L} promoter and operator (O_{L}), and the hybrid promoter operator, Tac as described in Amann, E., Brosius, J., and Ptashne, M. Gene 25:167-178 (1983), specifically incorporated herein by reference. Excision of those portions of the gene including ribosome binding site structural region and 3' non-translated sequences and insertion in alternate vectors containing the P_{L} or Tac promoter makes use of the unique restriction sites that flank these structures in pUC8-F/OmpAic and pUC8-F/OmpA1. Insertion of the EcoRl to Sall fragment from either into similarly digested plasmid pDP8 effects transcription of these genes directed by the lambda P_{L} promoter. Transcriptional regulation would be temperature sensitive by merit of the cl857 mutation harbored on this same plasmid.

Putting similar gene fragments into the transcription unit of the Tac promoter will be accomplished by first isolating the EcoRV to Sail fragment. This, together with the synthetic Tac promoter sequence which is flanked by BamHl and Pvull sites and which contains the lac operator will be inserted into the BamHl to Sail sites of pBR322 or preferably derivatives. The derivatives in this case refer to constructs containing either the lacl gene or the I^{q} gene.

Expression of FIBAC in host microorganisms other than Escherichia is considered. Yeast and bacteria of the genera Bacillus, Pseudomonas, and Clostridium may each offer particular advantages. The processes outlined above could easily be adapted to others.

In general, expression vectors for any microorganism will embody features analogous to those which we have incorporated in the above mentioned vectors of E. coli. In some cases, it will be possible to simply move the specific gene constructs discussed above directly into a vector compatible with the new host. In others, it may be necessary or desirable to alter certain operational or structural elements of the gene.

### EXAMPLE 3

The human collagenase inhibitor may be readily purified after expression in a variety of microbes. In each case, the spectrum of contaminant proteins will differ. Thus, appropriate purification steps will be selected from a variety of steps already known to give a good separation of the human collagenase inhibitor from other proteins and from other procedures which are likely to work.

If the inhibitor is not secreted from the microbes, it may form inclusion bodies inside the recombinant microbes. These bodies are separated from other proteins by differential centrifugation after disruption of the cells with a French Press. The insoluble inclusion bodies are solubilized in 6 M guanidine hydrochloride or 8 M urea, and the inhibitor protein is more completely solubilized by reaction of its cysteines with sodium sulfite. At any time subsequent to this step, the cysteines are converted back to their reduced form with dithiothreitol. Once the inhibitor protein is solubilized from inclusion bodies, immunoaffinity chromatography using antibodies raised against the unfolded inhibitor are used for purification before refolding.

The inhibitor can be refolded according to the protocol mentioned in Example 6, infra. After refolding of the inhibitor, or if the inhibitor is secreted from the microbes, purification from other proteins is accom - plished by a variety of methods. Initial steps include ultrafiltration through a 50 K dalton cutoff membrane or ammonium sulfate fractionation. Other useful methods include, but are not limited to, ion-exchange chromatography, gel filtration, heparin-sepharose chromatography, reversed-phase chromatography, or zinc-chelate chromatography. All of these steps have been successfully used in purification protocols. Additional high resolution steps include hydrophobic interaction chromatography or immunoaffinity chromatography. After purification, the metalloproteinase inhibitor is preferably at least 90 - 95% pure.

### EXAMPLE 4

### Purification of Human Collagenase Inhibitor from Human Amniotic Fluid

Human amniotic fluid obtained from discarded amniocentesis samples was pooled and 6 liters were subjected to ultrafiltration through a 100 kD MW cutoff filter, obtained from Millipore Corporation, in a Millipore Pellicon Cassette System. The eluate was concentrated through a 10 kD cutoff filter, obtained from Millipore Corporation, then through an Amicon PM-10 membrane. Aliquots (10 ml) of concentrated amniotic fluid were eluted through a 2.5 x 100 cm column of Ultrogel AcA54, obtained from LKB Corporation, which was equilibrated with pH 7.6, 0.05 M hepes, 1 M sodium chloride, 0.01 M calcium chloride, and 0.02% sodium aside (all chemicals were obtained from Sigma Chemical Company). Fractions containing the inhibitor were collected and pooled, dialyzed against pH 7.5, 0.025 M Hepes buffer containing 0.01 M calcium chloride and 0.02% sodium azide, and loaded onto a 1.5 x 28 cm heparin - sepharose CL-6B (obtained from Pharmacia, Inc.) column equilibrated with the same buffer. This column was rinsed with 1 liter of the above buffer and eluted with a linear gradient of 0-0.3 M sodium chloride. The fractions from the largest peak of inhibitor activity, eluting at about 0.1 -0.15 M sodium chloride, were pooled, concentrated to 1 ml, and loaded onto a Synchropak rp-8 reverse phase HPLC column equilibrated with 0.05% trifluoroacetic acid (Aldrich Chemical Company). The column was eluted with a linear gradient of 0-40% acetonitrile (J. T. Baker Chemical Company) at 1/2% per minute. All fractions were immediately dried in a Savant speed-vac concentrator to remove acetonitrile, and redissolved in pH 7.5, 0.1 M Hepes before assay. The inhibitor eluted between 32-38% acetonitrile. Fractions containing the inhibitor were pooled, and 100 ul aliquots were eluted over a Bio - rad biosil - TSK 250 HPLC gel filtration column. The pooled peaks of inhibitor activity contained 0.1 mg of inhibitor, which was over 95% pure as judged by SDS - polyacrylamide gel electrophoresis.

### EXAMPLE 5

### Purification of Human Fibroblast Collagenase Inhibitor from Human Embryonic Skin Fibroblast Serum - Free Medium

Human embryonic skin fibroblasts were grown in serum-free tissue culture medium. Ten liters of this medium were collected, dialyzed against pH 7.5, 0.02 M hepes buffer containing 0.02% sodium azide and 0.01 M calcium chloride, and applied to a 2.8 x 48 cm column of heparin-sepharose CL-6B (Pharmacia, Inc.) equilibrated with the same buffer. The column was rinsed with 2 liters of this buffer and was then eluted with linear gradient of 0-0.3 M sodium chloride contained in this buffer. The fractions obtained were tested for the presence of inhibitor by their ability to inhibit human fibroblast collagenase. The fractions corresponding to the peak of activity were those obtained near 0.15 M sodium chloride. These fractions were concentrated to about 5 ml by ultrafiltration through an Amicon YM10 filter and the concentrate was applied in four separate runs to a 250 x 4.1 mm Synchropak rp-8 reverse phase HPLC column, equilibrated with 1 % trifluoroacetic acid. The column was eluted with a 0 - 60% linear gradient of acetonitrile in 0.1% trifluoroacetic acid. The gradient was run at 1/2% acetontrile per minute. The inhibitor eluted in two sharp peaks between 26-29% acetonitrile. All fractions were immediately dried in a Savant speed-vac concentrator, redissolved in pH 7.5, 0.1 M Hepes, and assayed. At least 1.2 mg of collagenase inhibitor was recovered, which was 90 - 95% pure. This material gives a single band when run on a 17.5% reducing SDS gel. After carboxymethylation of the cysteines and elution through the same RP - 8 column under identical conditions, the inhibitor is suitably homogenous for protein sequencing.

### EXAMPLE 6

It is contemplated that the human collagenase inhibitor can be readily refolded into its native structure from its denatured state after expression of its gene in a microbe and separation of the collagenase inhibitor from most of the other proteins produced by the microbe. By analogy to the conditions necessary for the refolding of other disulfide-containing proteins as set forth by Freedman, R. B. and Hillson, D. A., in "Formation of Disulfide Bonds," In: The Enzymology of Post - Translational Modification of Proteins, Vol. 1, R. B. Freedman and H. C. Hawkins, eds., pp. 158-207 (1980), specifically incorporated herein by reference, refolding of the human collagenase inhibitor should occur in solutions with a pH of 8.0 or greater. At this pH, the cysteines of the protein are partially ionized, and this condition is necessary for the attainment of native disulfide bond pairings. The inhibitor concentration should be relatively low, less than 0.1 mg/ml, to minimize the formation of intermolecular disulfide - linked aggregates which will interfere with the refolding process.

Since the stability of the refolded (native) disulfide bonded structure relative to the unfolded (reduced) structure depends on both the solution oxidation - reduction potential and the concentrations of other redox - active molecules, it is contemplated that the redox potential should be buffered with a redox buffer giving a potential equivalent to a reduced: oxidized glutathione ratio of 10. The preferred concentration range of reduced glutathione would be 0.1 -1.0 mM. At higher concentrations, mixed disulfides will form with protein, reducing the yield of the refolded (native) structure. The relative stabilities of the unfolded protein and the native structure, and thus the rate and yield of refolding, will also depend on other solution variables, such as the pH, temperature, type of hydrogen - ion buffer, ionic strength, and the presence or absence of particular anions or cations as discussed in Privalov, P. L., "Stability of Proteins, Small Globular Proteins," in Advances in Protein Chemistry, Vol. 33, pp. 167-236, (1979), specifically incorporated herein by reference. These conditions vary for every protein and can be determined experimentally. It is contemplated that addition of any molecule that strongly prefers to bind the native (as opposed to the unfolded) structure, and which can be readily separated afterwards from the native (refolded) protein, will increase not only the yield but the rate of re-folding. These molecules include monoclonal antibodies raised against the native structure, and other proteins which tightly bind the native collagenase inhibitor, such as the mammalian enzymes collagenase or gelatinase.

### Example 7

The second preferred sequence as set forth herein, i.e.,

10 20 30 40 50 60
GGCCATCGCC GCAGATCCAG CGCCCAGAGA GACACCAGAG AACCCACCAT GGCCCCCTTT
H F XB HH N S
A N HI AH C A
E U ON EA O U
3 1 21 21 1 1

70 80 90 100 110 120
GACCCCTGGC TTCTGCATCC TGTTGTTGCT GTGGCTGATA GCCCCAGCAG GGCCTGCACC
B SF S H
S FO A A
T AK U E
1 11 1 3

130 140 150 160 170 180
TGTGTCCCAC CCCACCCACA GACGGCCTTC TGCAATTCCG ACCTCGTCAT CAGGGCCAAG
H T M SH
A T N AA
E H L UE
3 1 1 13

190 200 210 220 230 240
TTCGTGGGGA CACCAGAAGT CAACCAGACC ACCTTATACC AGCGTTATGA GATCAAGATG
H S
I A
N U
2 A

250 260 270 280 290 300
ACCAAGATGT ATAAAGGGTT CCAAGCCTTA GGGGATGCCG CTGACATCCG GTTCGTCTAC
SD FS FN F H A
AD OF NS O P C
UE KA UP K A C
11 11 12 1 2 1

310 320 330 340 350 360
ACCCCCGCCA TGGAGAGTGT CTGCGGATAC TTCCACAGGT CCCACAACCG CAGCGAGGAG
N A B M
C V B N
O A V L
1 2 1 1

370 380 390 400 410 420
TTTCTCATTG CTGGAAAACT GCAGGATGGA CTCTTGCACA TCACTACCTG CAGTTTCGTG
P F H P
S O I S
T K N T
1 1 1 1

430
GCTCCCTGGA AC
B
S
T
1
has the following restriction sites:
# | SITES | FRAGMENTS | | FRAGMENTS | ENDS

ACC 1 (GTVWAC)
1
| 295 | 295 | (68.3) | 1 | 295
| | 137 | (31.7) | 295 | 432

AVA 2 (GGRCC)
1
| 338 | 338 | (78.2) | 1 | 338
| | 94 | (21.8) | 338 | 432

BBV 1 (GCTGC)
1
| 350 | 350 | (81.0) | 1 | 350
| | 82 | (19.0) | 350 | 432

BIN 1 (GGATC)
1
| 14 | 418 | (96.8) | 14 | 432
| | 14 | ( 3.2) | 1 | 14

BST N1 (CCRGG)
2
| 65 | 360 | (83.3) | 65 | 425
| 425 | 65 | (15.0) | 1 | 65
| | 7 | ( 1.6) | 425 | 432

DDE 1 (CTNAG)
1

# | SITES | FRAGMENTS | | FRAGMENTS | ENDS
| 267 | 267 | (61.8) | 1 | 267
| | 165 | (38.2) | 267 | 432

FNU4H 1 (GCNGC)
3
| 8 | 269 | (62.3) | 8 | 277
| 277 | 82 | (19.0) | 350 | 432
| 350 | 73 | (16.9) | 277 | 350
| | 8 | ( 1.9) | 1 | 8

FOK 1 (GGATG)
4
| 76 | 197 | (45.6) | 76 | 273
| 273 | 99 | (22.9) | 285 | 384
| 285 | 76 | (17.6) | 1 | 76
| 384 | 48 | (11.1) | 384 | 432
| | 12 | ( 2.8) | 273 | 285

HAE 2 (PGCGCQ)
1
| 19 | 413 | (95.6) | 19 | 432
| | 19 | ( 4.4) | 1 | 19

HAE 3 (GGCC)
5
| 1 | 258 | (59.7) | 174 | 432
| 51 | 60 | (13.9) | 51 | 111
| 111 | 50 | (11.6) | 1 | 51
| 144 | 33 | ( 7.6) | 111 | 144
| 174 | 30 | ( 6.9) | 144 | 174
| | 1 | ( 0.2) | 1 | 1

HHA 1 (GCGC)
1
| 20 | 412 | (95.4) | 20 | 432
| | 20 | ( 4.6) | 1 | 20

# | SITES | FRAGMENTS | FRAGMENTS ENDS
HINC 2 (GTQPAC)
1
199 | 233 (53.9) | 199 | 432
| 199 (46.1) | 1 | 199
HINF 1 (GANTC)
1
389 | 389 (90.0) | 1 | 389
| 43 (10.0) | 389 | 432
HPA 2 (CCGG)
1
288 | 288 (66.7) | 1 | 288
| 144 (33.3) | 288 | 432
MNL 1 (CCTC)
2
162 | 193 (44.7) | 162 | 355
355 | 162 (37.5) | 1 | 162
| 77 (17.8) | 355 | 432
MST 2 (CCTNAGG)
1
266 | 266 (61.6) | 1 | 266
| 166 (38.4) | 266 | 432
NCO 1 (CCATGG)
2
47 | 261 (60.4) | 47 | 308
308 | 124 (28.7) | 308 | 432
| 47 (10.9) | 1 | 47
NSP B2 (CVGCWG)
1
278 | 278 (64.4) | 1 | 278
| 154 (35.6) | 278 | 432

# | SITES | FRAGMENTS | FRAGMENTS ENDS
PST 1 (CTGCAG)
2
379 | 379 (87.7) | 1 | 379
408 | 29 ( 6.7) | 379 | 408
| 24 ( 5.6) | 408 | 432

SAU 1 (CCTNAGG)
1
266 | 266 (61.6) | 1 | 266
| 166 (38.4) | 266 | 432

SAU 3A (GATC)
2
14 | 217 (50.2) | 14 | 231
231 | 201 (46.5) | 231 | 432
| 14 ( 3.2) | 1 | 14

SAU96 1 (GGNCC)
4
51 | 165 (38.2) | 173 | 338
110 | 94 (21.8) | 338 | 432
173 | 63 (14.6) | 110 | 173
338 | 59 (13.7) | 51 | 110
| 51 (11.8) | 1 | 51

SCR F1 (CCNGG)
2
65 | 360 (83.3) | 65 | 425
425 | 65 (15.0) | 1 | 65
| 7 ( 1.6) | 425 | 432

SFA N1 (GATGC)
2
75 | 199 (46.1) | 75 | 274
274 | 158 (36.6) | 274 | 432
| 75 (17.4) | 1 | 75

# SITES FRAGMENTS FRAGMENTS ENDS

STY 1 (CCRRGG)
2
47 | 261 | (60.4) | 47 | 308
308 | 124 | (28.7) | 308 | 432
 | 47 | (10.9) | 1 | 47

TTH111 1 (GACNNNGTC)
1
160 | 272 | (63.0) | 160 | 432
 | 160 | (37.0) | 1 | 160

XHO 2 (PGATCQ)
1
13 | 419 | (97.0) | 13 | 432
 | 13 | ( 3.0) | 1 | 13
The following do not appear:
AAT 2 | AFL 2 | AFL 3 | AHA 2
AHA 3 | ALU 1 | APA 1 | ASU 2
AVA 1 | AVA 3 | AVR 2 | BAL 1
BAM H1 | BAN 1 | BAN 2 | BCL 1
BGL 1 | BGL 2 | BSM 1 | BSP 1286
nSSH 1 | BST E2 | CFR 1 | CLA 1
ECO R1 | ECO R5 | FNUD 2 | GDI 2
HAE 1 | HGA 1 | HGI A1 | HGI C1
HGI D1 | HGI J2 | HIND 3 | HPA 1
HPH 1 | KPN 1 | MBO 2 | MLU 1
MST 1 | NAE 1 | NAR 1 | NCI 1
NDE 1 | NHE 1 | NOT 1 | NRU 1
NSP C1 | PVU 1 | PVU 2 | RRU 1
RSA 1 | SAC 1 | SAC 2 | SAL 1
SCA 1 | SMA 1 | SNA 1 | SNA B1
SPE 1 | SPH 1 | SSP 1 | STU 1
TAQ 1 | XBA 1 | XHO 1 | XMA 3
XMN 1

The salient features of this cDNA are:
1. The coding strand is presented in the 5' to 3' convention with the polyC tract at the 5' end.
2. If the first G in the sequence GGC CAT CGC CGC is considered as nucleotide 1, then an open reading frame exists from nucleotide 1 through nucleotide 432, which is the 3' end of this partial cDNA.
3. The first methionine in this reading frame is encoded by nucleotides 49 through 51 and represents the initiation site of translation.
4. The amino acid sequence prescribed by nucleotides 49 through 114 is not found in the primary structure of the mature protein, but it is the sequence of the leader peptide of human protein.
5. The sequence of nucleotides 82 through 432 is identical to the sequence of nucleotides numbered 1 through 351 in the insert from the first preferred sequence of Example 1.
6. The amino acid sequence of the mature protein displays two consensus sequences for sugar attachment. These sequences, - -N-Q-T- prescribed by nucleotides 202 through 210 and -N-R-S - prescribed by nucleotides 346 through 354, are amino acid residues 30 through 32 and 78 through 80, respectively, in the mature protein. Both sites are glycosylated in the human inhibitor protein.

### EXAMPLE 8

A series of expression vectors have been constructed which direct the transcription and translation of the FIBAC gene in E. coli.

### A. Expression Vector pFib51

The first of these constructions is a derivative of plasmid pUC8 which contains the coding region of the human fibroblast collagenase inhibitor ("FIBAC") gene arranged in such a way as to allow its transcription to be directed and regulated by the lac promoter and operator. This construct, pFib51, was made with minor modification of the procedure outlined in Example Two for the assembly of expression vector pUC8 - Fic.

Trimming of the 5' end of the coding region was effected by isolating that piece of DNA extending from the Haelll site at nucleotide 93 through the 3' EcoRI site beginning at nucleotide 698. Reconstruction of the 5' end was accomplished as described except that oligonucleotides FA3 and FA4 were each lengthened by 12 bases to extend the reconstruction to the Haelll recognition site. Hence, the structure of FIBAC A' was created:

GA TCC GCG ATC GGA GTG TAA GAA
G CGC TAG CCT CAC ATT CTT

ATG TGC ACT TGC GTT CCG CCG CAT
TAC ACG TGA ACG CAA GGC GGC GTA

CCG CAG ACT GCT TTC TGC AAC TCT
GGC GTC TGA CGA AAG ACG TTG AGA

GAC CTG GTG ATC AGG G
CTG GAC CAC TAG TCC C

The salient features of FIBAC A' have remained as described in Example 2.

A synthetic linker was made to couple the 3' end of the Haelll to EcoRI fragment to a Sail site. These oligonucleotides were designed to recreate the Sall site and to destroy their EcoRI site. In addition, the linkers were modified from the original description to include an internal Kpnl site. The new linker is comprised of the oligonucleotides "modified - linker A1 " and "modified - linker A2."
Modified - linker A1 is: AATTGGTACCAG
Modified - linker A2 is: TCGACTGGTACC

Ligation into M13 mp19, cloning, and selection were essentially as described in previous Examples. The coding region of FIBAC was removed from a clone with the designed sequence by digestion with BamHI and Hindlll and subcloned into these restriction sites in pUC8. The resulting plasmid is pFib51. In this plasmid, the transcription of the FIBAC gene is directed by the lac promoter. Translation of methionyl FIBAC is coupled to translation initiated for beta - galactosidase.

### B. Expression Vector pFib55

Creation of the HgiAl restriction site at the 5' end of the mature FIBAC coding sequence would allow the entire FIBAC coding sequence to be portable via a HgiAl/Sall, Kpnl, or Hindlll double digestion in plasmid pFib51, except for another HgiAl site at position 552 within the coding sequence. This restriction site was removed using in vitro oligonucleotide-directed, site-specific mutagenesis essentially as described by Zoller and Smith in Methods in Enzymology 100:468, specifically incorporated herein by reference.

Single-stranded DNA isolated from a derivative of bacteriophage mp18 containing the met-FIBAC gene translationally coupled to lacZ as described above was annealed to the synthetic oligonucleotide GGGCTTTGCACCTGGCAG. This oligonucleotide anneals to the FIBAC coding region across the HigiAl site with a single mismatch. The resultant DNA was then incubated with the Klenow fragment of E. coli DNA polymerase, T4 DNA ligase and a mixture of all four deoxy - nucleotidetriphosphates.

The covalently-closed, double - stranded phage DNA thus obtained was used to transfect E. coli strain JM107. Plaques were assayed for the presence of the mutant sequence by their hybridization to the mutagenic oligonucleotide shown above at 58°C in 6x SSC.

The selected clone had the leu codon CTT replacing CTG at amino acid position 173. The coding region from this clone was excised as a BamHl to Hindlll fragment and ligated into a similarly - digested pUC8. The resulting plasmid, pFib55, has all of the features of pFib51 as well as a more easily mobilized FIBAC coding region.

### C. Expression Vector pFib56

Alternate methods of translational coupling to beta - galactosidase or any E. coli protein can be similarly constructed. For one embodiment, a clone has been designed which is similar from a regulation of expression point of view to plasmid pFib51 (i.e., FIBAC translationally coupled to lacZ in pUC8), but which uses an alternate translational coupler. To create pFib56, the following pieces of DNA were synthesized:

EcoRI end HgiAI end
5' A A T T C C A A G G A G A A A T A A A T G T G C A 3'
HgiAI end EcoRI end
5' C A T T T A T T T C T C C T T G G 3'

This double-stranded EcoRl/HgiAl fragment was then combined with the HgiAl/HindIII FIBAC coding sequence and plasmid pUC8 that had been digested with EcoRl and Hindlll and ligated. The resulting plasmid has been called pFib56. When this plasmid is transformed into E. coli strain JM107, the strain, JM107/pFib56, can be induced to express even more methionyl FIBAC than JM107/pFib51 or JM107/pFib55. From this, it has been concluded that the translational coupler in pFib56 is more efficient than that in pFib51.

### D. Expression Vectors pFib10 and pFib11

To direct the expressed FIBAC to the periplasmic space of E. coli, a leader peptide is added to the amino terminus of FIBAC. The leader peptide will effect the transport of the fusion protein out of the inner cell membrane. Cellular processing removes the signal peptide and yields the mature form of FIBAC.

Two signal sequences have been separately fused to FIBAC for this purpose. They are the leader peptides of E. coli ompA and phoS gene products. Both ompA_{L} - FIBAC and phoS_{L}-FIBAC fusion proteins contain signals to direct them to be located in the periplasm of E. coli and to allow proteolytic processing of the fusion to ompA or phoS leader fragments and native FIBAC.

Plasmid pFib10 is a derivative of pUC8 into which the coding sequence for the ompA_{L} - FIBAC fusion protein has been inserted. In addition, some 5' nontranslated sequences from the ompA gene are included. Transcription of this plasmid is directed by the lac promoter/operator of pUC8. Translation is initiated at the methionine codon beginning the ompA leader sequence and uses the Shine - Dalgarno sequence found in the ompA gene.

The plasmid was constructed by ligating the FIBAC coding sequence contained on a HgiAl/HindIII fragment of pFib55 together with synthetic oligonucleotides encoding the ompA leader peptide and with EcoRI/HindIII- digested pUC8. The coding strand of the synthesized oligonucleotide is:

ompA_L
EcoRI end
5' A A T T C G A T A T C T C G T T G G A G A T A T T C A T G A C
G T A T T T T G G A T G A T A A C G A G G C G C A A A A A A T
PvuI
G A A A A A G A C A G C T A T C G C G A T C G C A G T G G C A
C T G G C T G G T T T C G C T A C C G T A G C G C A G G C C T
G C A 3'
HgiAI end

The synthesis was accomplished as four oligonucleotides, two for each of the strands. Together, the double - stranded DNA features:
(a) An EcoRI cohesive end at the 5' end and a HgiAl cohesive end at the 3' end of the coding strands;
(b) An open reading frame encoding the leader peptide of the ompA gene product which is in frame with the FIBAC gene when ligated at the HgiAl site;
(c) Non-coding sequences 5' to the translated portion which contain the ribosome binding site normally found in the ompA gene; and
(d) An internal, unique Pvul site.

Plasmid pFib11 is a derivative of plasmid pKK223-3 and is identical to pFib10 except that its pUC8 portion has been replaced by the 4550 bp EcoRI/HindIII fragment of plasmid pKK223-3. In this construct, transcription is directed and regulated by the hybrid tac promoter/operator. Additionally, this plasmid contains a transcriptional terminator which may stabilize the plasmid in a high expression system.

### E. Expression Vector pFib13

In plasmid pFib13, the 5' non-coding region of the ompA_{L} sequence has been eliminated and the ompA_{L} - FIBAC fusion gene is translationally coupled directly to the N-terminal portion of the lacZ gene as it appears in plasmid pUC8. This has been accomplished by ligating the EcoRI/Pvul (3200 bp) fragment of plasmid pFib10 to a synthetic oligonucleotide shown here.

EcoRI end
5' A A T T C C A A G G A G A A A T A A A T G A A A A A G A C A G C
T A T C G C G A T 3'
PvuI end

PvuI end
5' C G C G A T A G C T G T C T T T T T C A T T T A T T T C T C C T
T G G 3'
EcoRI end

### F. Expression Vector pFib31

The phoS gene of E. coli codes for the phosphate binding protein and has been described and sequenced by Surin, B.D. et al., in J. Bacteriol. 157:772-778 (1984), specifically incorporated herein by reference. This protein is periplasmic, with a 25-amino-acid leader sequence that directs it to that compartment. The leader sequence is proteolytically removed during the translation process to leave only mature phoS protein in the periplasm. We have synthesized the phoS leader sequence as two double - stranded DNA fragments with EcoRl and HgiAI ends as shown:

EcoRI/ClaI

EcoRI end
5' A A T T C A T G A A A G T T A T G C G T A C C A C C G T C G C
A A C T G T T G T C G C C G C G A C C T T A T 3'
ClaI end

ClaI end
5' C G A T A A G G T C G C G G C G A C A A C A G T T G C G A C G
G T G G T A C G C A T A A C T T T C A T G 3'
EcoRI end

ClaI/HgiAI

ClaI end HgiAI end
5' C G A T G A G T G C T T T C T C T G T G T T T G C G T G C A 3'

HgiAI end ClaAI end
5' C G C A A A C A C A G A G A A A G C A C T C A T 3'

These fragments were ligated together at a Clal site internal to the phoS leader. These fragments were simultaneously combined with the HgiAl/Hindlll FIBAC coding fragment described above and plasmid pKK223 - that had been digested with EcoRl and Hindlll. The resulting plasmid has been called pFib31.

### EXAMPLE 9

### EXPRESSION OF THE FIBAC GENE IN E. COLI

Three methods have been employed to qualitatively determine the amount and form of FIBAC produced by E. coli cells harboring the plasmids described above. They are:
(1) specific reaction of FIBAC antibody to E. coli proteins produced following induction of the FIBAC gene resolved by polyacrylamide - SDS - electrophoresis and subsequently bound to nitrocellulose paper (western blotting);
(2) labeling of E. coli proteins with ³⁵ S - cysteine, ³⁵S - methionine, or ³⁵SO²⁻₄; following induction of the FIBAC gene; and
(3) inspection of polyacrylamide SDS gels containing E. coli proteins and FIBAC without antibody coupling or radioactive - labeling.

These methods have allowed not only comparison of the amounts of FIBAC produced by each strain, but also purification of the FIBAC following expression without the need for functional metalloproteinase inhibition. All of the plasmids discussed in Example 8 have been expressed in the background of E. coli strain JM107. The expression of FIBAC in E. coli has so far been greater in those systems designed to transport the protein outside of the inner cell membrane. This is possibly due to degradation of the expressed protein in the cytoplasm.

Processing ompa_{L}-FIBAC fusion protein to yield the mature form of FIBAC has been found to be partially dependent on the phase of growth of the cells. Cells induced with IPTG in early log phase of growth accumulate a mixture of processed and unprocessed FIBAC, while cell cultures induced in late log phase accumulate only processed FIBAC. Strains expressing the phoS_{L}-FIBAC fusion appear to process the protein completely independent of growth phase.

All of the expression vectors described above in Example 8 have ampicillin resistance as the selectable marker. For the purposes of production, it might be preferable to have a tetracycline resistance marker. One plasmid generally useful as an expression vector has been constructed with a tetracycline resistance marker. This plasmid is a derivative of pKK223 - 3 in which the truncated tetracycline resistance gene has been replaced with fully functional tet^{r} gene adapted from pBR322.

### EXAMPLE 10

### PURIFICATION OF FIBAC EXPRESSED IN E. COLI

The recombinant human colleagenase inhibitor (FIBAC) has been purified from E. coli strain JM107 transformed with plasmid pFib11. In this strain, JM107/pFib11, FIBAC can be made to accumulate as an insoluble aggregate. In this example, the conditions for growth of the cells, induction of FIBAC gene expression, cell harvesting, and purification of FIBAC from the insoluble fraction of a total cell lysate are described. The same protocol may also be used to substantially enrich Fibac from the soluble fraction of total cell lysate.

### A. Insoluble Fraction

Luria broth containing ampicillin at a concentration of 100 ug/ml was innoculated with an overnight culture of JM107/pFib11 to an initial OD₆oo of 0.15-0.20. The shake flask cell culture was allowed to grow at 37°C to an OD₆oo of 1.5, at which time the culture medium was supplemented with IPTG to a final concentration of 0.5 mM. Incubation at 37°C was then continued for 2.5 to 3 hours. The cell culture was rapidly cooled to 4 °C in an ice bath and the cells harvested by centrifugation. A one - liter culture grown as described above yielded on the average 3 gr of cells (wet weight). The cells were washed once in cold lysis buffer (50 mM MES, pH 6.0, 4 mM EDTA) and then resuspended in the lysis buffer to a final concentration of 0.26 g cells (wet weight)/ml. The cell suspension was frozen at 70°C until further processing.

Total cell lysate was prepared by passing the cell suspension two times through a French pressure cell (SLM - Aminco model #FA-079 fitted with piston #FA-073 and operated at 20,000 psti, SLM Instruments, Inc., Urbana, Illinois). The resultant cell lysate was incubated with 10 ug of DNase I/gr of cells (wet weight) for 2 - hours on ice. The cell lysate was then divided into small aliquots and stored frozen at -70°C until further processing.

A cell lysate supernatant and cell lysate pellet fraction were obtained by centrifugation of a 5 ml portion of cell lysate for 30 minutes at 4 °C in an Eppendorf micro centrifuge. The resultant pellet was washed twice with 3 ml of 50 mM Tris - HCI, pH 8.0, 4 mM EDTA, 50 mM DTT. The supernatants from these washes were pooled and saved for further analysis. The washed pellet was then solubilized by resuspension in 3 ml of 50 mM MES, pH 6.0, 4 mM EDTA, 50 mM DTT, 10 M urea, and incubated at room temperature for 15 minutes. Should protein carbamylation occur due to urea solubilization the side reaction could be quenched by the addition of a one hundred fold excess of a suitable nucleophile over total protein amino groups. The resultant solution was clarified by centrifugation for 15 minutes at 4 °C in an Eppendorf micro centrifuge. The supernatant contained essentially all of the protein from the solubilization procedure and was saved for further analysis. The remaining small pellet consisted mostly of cell wall debris and few proteins (none of which were FIBAC). This was discarded.

The identification of FIBAC in the various fractions prepared as described above was accomplished by SDS-PAGE and probing of western blots with anti-FIBAC antibodies. SDS-gel analysis of total cell lysate protein obtained from IPTG-induced JM107/pFib11 shows the presence of a protein band of approximately 20,000 dalton apparent molecular mass that is absent in the gel pattern from a cell lysate of non - included JM107/pFib11. The 20,000 Da protein and a faster migrating band, presumably a degradation product of FIBAC, react with anti - FIBAC antibodies in the western blot analysis. The IPTG induction - dependent presence of this protein, the molecular weight, and the reactivity with anti - FIBAC antibodies suggest that the protein represents the expressed recombinant FIBAC. Analysis of the cell lysate supernatant fraction obtained from IPTG-induced JM107/pFib11 and the cell lysate pellet wash revealed little FIBAC. The bulk of the FIBAC was found, however, in the urea-DTT solubilized cell lysate pellet fraction. This was interpreted to mean that, upon IPTG induction, the FIBAC accumulates an insoluble fraction and can be isolated in a substantially purified form from the washed cell lysate pellet.

The urea - DTT solubilized cell lysate pellet was used as the starting material for the CM - chromatog - raphy. A 1.2 ml aliquot of solubilized cell lysate pellet (16 mg protein) was diluted to 25 ml with cold CM - buffer (50 mM MES, pH 6.0, 6 M urea, 14 mM 2-ME). The sample was then applied to a carboxymethyl cellulose column (25x130 mm) previously equilibrated with CM - buffer at 4°C. After sample application, the column was washed with CM - buffer until the A₂₈₀ returned to baseline. Adsorbed protein was eluted with a linear sodium chloride gradient (0 - 200 mM) in CM - buffer. Total gradient volume was 400 ml, flow rate was 26 ml/hr, and 5 ml fractions were collected. The "flow-through" fraction (CM-FT) and the peak fractions 58-61 were pooled and analyzed by SDS-PAGE. The electrophoretic and immunological analysis of these fractions revealed that the recombinant FIBAC, including some degraded FIBAC, eluted at approximately 120 mM NaCl without any other detectable proteins. Under the chromatographic conditions employed, most non - FIBAC proteins did not adsorb to the CM - column and were found in the "flow - through" fraction.

The amount of CM - purified FIBAC obtained in this procedure was estimated to represent 1.3% of the total cell protein. Bradford protein assays were used for quantitation throughout the isolation and purification procedure.

Two ml (100 ug) of purified FIBAC in 50 mM MES, 6 M urea, 14 mM 2-mercaptoethanol were concentrated to 200 ul by Centricon centrifugation and adjusted to pH 8.5 by addition of 2 M Tris - HCI, pH 8.5 to a final Tris concentration of 0.5 M. The cysteine residues of FIBAC were then carboxymethylated using ³H-iodoacetic acid. The alkylation reaction mixture was desalted by reverse phase HPLC. The modified FIBAC eluted at 30% acetonitrile and was collected for further analysis. An aliquot of the modified FIBAC isolated from the HPLC was subjected to SDS - PAGE analysis. Comparison of the CM - purified FIBAC that was used for the carboxymethylation (starting material) and the FIBAC after alkylation and HPLC desalting showed that the modified FIBAC migrated slightly slower in the SDS gel than the non - modified FIBAC. This is not an unusual observation, particularly in view of the substantial number of modified cysteines present in FIBAC.

The carboxymethylated FIBAC was then applied to an Applied Biosystems (model 470A) gas-phase protein sequencer (Foster City, CA) for automatic Edman degredation, and the amino acid sequence for the first 24 residues was identified. The sequencing data for the first 6 cycles of the Edman degradation are shown in the table below. The data clearly establish that the N-terminal amino acid sequence of the purified FIBAC (C-T-V-P-P...) is identical to that previously determined for native FIBAC. It is therefore concluded that pFib11 properly processes the recombinant FIBAC by cleaving the ompA-FIBAC fusion protein at its ala - cys junction to produce the mature form of the FIBAC protein.

### N - TERMINAL AMINO ACID SEQUENCE ANALYSIS OF PURIFIED RECOMBINANT FIBAC

### (cysteine residues were labeled with ³H - iodoacetic acid prior to sequencing of the protein)

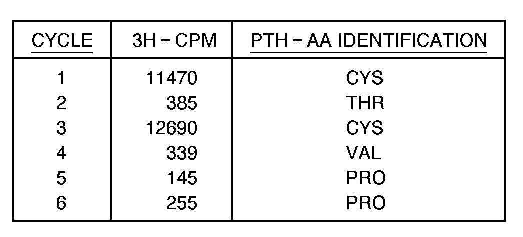

### B. Soluble Fraction

Because of the additional contaminants in the starting material, the procedure discussed herein does not initially result in a homogeneous preparation of FIBAC. However, the procedure does provide sufficient purification to allow refolding of FIBAC to its native conformation. Subsequent purification steps may then be used to complete the isolation procedure.

In this example, the cell growth, induction, harvesting, and preparation of a total cell lysate were as in the previous example. In order to demonstrate the ability of the present procedure to purify FIBAC from any fraction of the cell lysate, the original centrifugal fractionation of the homogenate was omitted. Instead, the total cell lysate was made to 10 M urea, 4 mM EDTA, 50 mM DTT, 50 mM MES, pH 6.0 and incubated at 22 ° C for 15 minutes. The solution was then centrifuged for 15 minutes in an Eppendorf microcentrifuge at 4°C. The pellet was discarded and the supernatant diluted with CM-buffer (50 mM MES, pH 6.0, 6 M urea, 14 mM 2-mercaptoethanol) and chromatographically fractionated on carboxymethyl cellulose as in the previous example. FIBAC eluted at approximately 120 mM salt along with some degraded FIBAC and several immunologically non - related contaminants. Estimation of the purity of FIBAC by SDS - PAGE analysis showed it to be greater than 50 percent of the total protein in this fraction. At this level of purity, it is possible to refold the FIBAC to its native conformation using the refolding procedure below. The refolded FIBAC is fully functional as a metalloprotease inhibitor and may be further purified by anion exchange chromatography.

Anion exchange chromatography was effected on a column 10x100 mm of Whatman DE-52 (Whatman Inc., Clifton, New Jersey) equilibrated in 600 mM urea, 50 mM Tris, pH 9.6. The solution containing the impure, refolded FIBAC was titrated to a pH of 9.6 by drop-wise addition of 5 N NaOH and applied to the DEAE cellulose. Analysis of the flow - through fraction demonstrated that the FIBAC was not retained. The immunologically non - related contaminants bound to the matrix and were thereby removed from the solution. The flow - through fractions can be concentrated on a CM-cellulose column as shown below.

The flow - through fraction from the anion exchange column was titrated to pH 7.5 by the addition of 5 N HCI. This solution was applied to a CM-cellulose column (25x130 mm) previously equilibrated with 600 mM urea and 50 mM Tris, pH 7.5. The column was washed with this same buffer until no protein could be spectrally observed to elute. The FIBAC was then eluted with the above buffer made to 250 mM in NaCL. The protein peak was pooled and dialyzed to equilibrium against 50 mM Tris, pH 7.5.

Electrophoretic, immunological, and functional assays of the resulting FIBAC demonstrate an active, refolded collagenase inhibitor of greater than 90% purity. The only detectable contaminant is a FIBAC degradation product as in the purification from the cell lysate pellet. Because of the identical amino terminal sequence of this contaminant and its apparent molecular mass, it has been concluded that a proteolytic clip has been made close to the carboxy terminus. This material can be removed in further purification steps (e.g., higher resolution ion exchange chromatography or affinity chromatography of the refolded FIBAC).

### EXAMPLE 11

### CONSTRUCTION OF A YEAST FIBAC EXPRESSION CLONE

Another organism in which gene expression and protein export vectors have been constructed is the yeast Saccharomyces cerevisiae. The yeast alpha-factor is a mating hormone which is produced intracellularly and exported to the growth medium. A single peptide sequence directs this transport. The FIBAC coding sequence has been cloned into a yeast expression vector to create a fusion of the yeast alpha-factor leader sequence to FIBAC. A construct was first made as a derivative of pGS385. Plasmid pGS385 has the following features:
(a) It contains portions of the yeast alpha-factor gene including the promoter, leader peptide, polyadenylation signal, and transcriptional termination signals;
(b) The portion of the alpha-factor gene between the two most distant Hindlll sites has been deleted, creating a unique Hindlll site;
(c) It contains a unique Sail site 3' to the Hindlll site; and
(d) It has the pBR322 origin of replication and ampicillin resistance gene to allow replication and selection in E. coli.

The plasmid pGS385 was digested with Hindlll and Sall. The Hindlll site defines the carboxy terminus of the alpha-factor leader sequence. The synthetic octanucleotide 5'-AGCTTGCA-3' was used to bridge the alpha-factor C-terminus to the N-terminus of FIBAC. The alpha-factor transcription-translation sequences drive gene expression in this vector. The entire alpha-factor-FIBAC fusion was then removed by digestion with EcoRl and inserted into plasmid YiP5, a derivative of plasmid pBR322, contains the yeast ura3 gene. This plasmid is suitable for use as an expression vector following digestion at a unique Stul site in ura3 and transformation into S. cerevisiae to direct integration of the entire plasmid into the chromosomal ura3 locus. Such integrants of an alpha - FIBAC fusion derivative of YiP5 have been obtained and here produced and secreted immunoreactive material as determined by colony screening techniques.

### EXAMPLE 12

### EXPRESSION OF RECOMBINANT FIBAC IN ANIMAL CELLS

Two expression systems for the production of FIBAC in animal cells are proposed. The first incorporates the SV40 late promoter to direct transcription in COS -1 cells. This expression system is primarily useful for studying the expression, protein synthesis, post-translational modification, and transport of FIBAC in COS - 1 cells. Although the system is rapid and convenient, it is limited to the COS - monkey cell line. The SV40 expression plasmid will be a derivative of pJC119, the construction of which is described in detail by Sprague, J., Condra, J.H., Arnheiter, H. and Lazzarini, R.A., in J. Virol. 45:773-781 (1983), specifically incorporated herein by reference. The complete FIBAC coding region, including the naturally -occurring signal sequence, has been assembled from the partial cDNA clones. The Ncol site coinciding with the initiator methionine for the leader peptide will be linked via a short synthetic oligonucleotide to the unique Xhol site in pJC119. The entire FIBAC coding region and 3' nontranslated sequences are inserted at this site where transcription is directed by the SV40 late promoter. The plasmid would thus contain SV40 origin of replication, the pBR322 origin of replication and the ampicillin resistance selectable marker.

The second system would be preferred for production of FIBAC in animal cells because it will result in the stable and continuous expression of FIBAC from a human cell line. This vector will be a derivative of pBPV52-1, described by Florkiewicz, R.Z., Smith, A., Bergmann, J.E., and Rose, J.K. in Cell Biol. 97:1381-1388 (1983), specifically incorporated herein by reference. This plasmid features the bovine papilloma virus origin of replication, pBR322 origin of replication, beta-lactamase gene, and the 69% transforming fragment of BPV DNA. The SV40 origin of replication and the SV40 early promoter will be cloned into this plasmid. Sequences from pBR322 that interfere with the replication of the vector in human cells will be excluded. As with the previous plasmid, the entire coding portion of the FIBAC cDNA will be inserted so as to direct its transcription by the SV40 early promoter.

It is expected that purification of FIBAC from the medium following expression and secretion from these cells will be possible essentially as described previously in Examples 4 and 5.

### EXAMPLE 13

### REFOLDING FIBAC

Two assays have been used to monitor the refolding of FIBAC. Both assays measure the appearance of the functional capacity of FIBAC as its native structure. The first assay is an inhibition assay which measures the inhibitory effect of the sample on the ability of human fibroblast collagenase to degrade¹⁴C-labeled collagen. The second assay is a modified ELISA which measures the binding of the refolded FIBAC to human collagenase.

The collagenase binding ELISA is the primary assay by which FIBAC activity was detected. Here, collagenase is coated overnight at 4°C in 96-well Immulon II plates (1.0 ug/ml in 50 mM Tris, pH 8.2, 5 mM CaC1₂; 100 ul per well). After the wells are blocked for 45 minutes with 150 ul/well of 3% BSA in washing buffer (50 mM Tris, pH 7.5, 5 mM CaCI₂, 0.02% Tween-20), varying dilutions of FIBAC standards or unknown samples diluted in blocking buffer are pipetted into the wells (100 ul/well). Following a 45 - minute incubation period (37°C), the wells are washed three times with washing buffer. Affinity-purified rabbit and anti-FIBAC is added to the wells (diluted 1/100, 100 ul/well) and incubated at 37° C for 45 minutes. The wells are again washed and alkaline phosphatase - conjugated goat anti-rabbit IgG (Sigma, diluted 1/1000 in washing buffer) is then added to the wells (100 ul/well). Following a one-hour incubation period at 37 °C, the wells are washed a last time and alkaline phosphatase substrate (Sigma #104- 105, 1 mg/ml in 10% diethanolamine, 100 mM MgC1₂, pH 9.8) is added to the wells. Color development is monitored at 495 nm using a Titertek Multiskan MG ELISA reader (Flow Laboratories). Native FIBAC serves as a standard curve against which unknown samples may be quantitated.

In the collagenase inhibition assay, ¹⁴C-labeled guinea pig skin collagen pellets (25 ul/pellet = 2100 cpm) is digested with 50 microliters of trypsin-activated collagenase (approximately 75 ug/ml in 50 mM Tris, pH 7.5, 10 mM caCl₂), which releases ¹⁴C into solution. After incubating the pellets for 1 -3 hours (depending on the rate of digestion), the reaction is stopped by adding 100 ul of Tris buffer and centrifuging for 10 minutes at 10,000 rpm. The supernatant is then pipetted into scintillation vials containing 3 mls of scintillation fluid and counted. Preincubation of the collagenase with 50 ul of varying dilutions of standard or purified FIBAC prior to adding the solution to the collagen pellet should inhibit digestions of the collagen and the subsequent release of ¹⁴C into solution. The quantitation of inhibitory activity of an unknown sample depends on the amount of active collagenase used in the assay. From this, the activity of the unknown sample may be calculated by assuming a 1:1 molar ratio between inhibitor and enzyme.

Using these assays, the efficiency of the refolding process with respect to protein concentration, oxidized gluthathione concentration, pH, and temperature has been examined. While all combinations of these parameters have not been exhaustively examined, a procedure has been developed which allows efficient renaturation.

The refolding of FIBAC depends greatly on the concentration of FIBAC at which refolding is performed. Under oxidizing conditions, dilute solutions prevent the formation of interchain disulfides, which eventually lead to the precipitation of aggregates.

Purified recombinant FIBAC can be refolded and remains soluble with 100% recovery of protein by following the protocol described below:
(1) Dilute, purified FIBAC (less than 300 ug/ml) in 6 M urea, 50 MES, pH 6.0, 14 mM 2 - mercap - toethanol, is incubated in the presence of 70 mM oxidized glutathione.
(2) After a 10 - minute incubation period at room temperature, the sample is diluted ten - fold with 50 mM Tris, pH 9.0.
(3) The sample is then incubated overnight at 4 C.

SDS - PAGE analysis of this reactivated material indicates the presence of both intact and degraded FIBAC. The degraded FIBAC is carried through from the purification procedure and does not appear to degrate further during the reactivation procedure.

This solubilized FIBAC has been demonstrated to have both collagenase binding activity (ELISA) and collagenase inhibitory activity. The amount of activity relative to the amount of protein appears to be greater than 90% as determined by the collagenase inhibition assay. This number was derived from calculations based on the estimated amount of collagenase in the assay. Although this is an estimate, it is not believed it to be off by more than 50%. Binding activity as measured against the FIBAC standard has enabled us to monitor the relative reactivation of different samples.

The refolding process has also been shown to work on 50% pure FIBAC preparations. The nature and amount of contaminating protein that can be tolerated is still uncertain, however, some active FIBAC has been detected in total cell lysates without purification demonstrating that at least some yields are obtainable at less than 5% purity.

It will be apparent to those skilled in the art that various modifications and variations can be made in the processes and products of the present invention. Thus, it is intended that the present invention cover the modifications and variations of this invention provided they come within the scope of the appended claims and their equivalence.

## Claims (Claims for the following Contracting State(s) : BE, CH, DE, DK, ES, FR, GB, GR, IE, IT, LI, LU, MC, NL, PT, SE)

1. A portable DNA sequence capable of directing intracellular production of metalloproteinase inhibitors comprising the sequence:

10 20 30 40 50 60
GTTGTTGCTG TGGCTGATAG CCCCAGCAGG GCCTGCACCT GTGTCCCACC CCACCCACAG

70 80 90 100 110 120
ACGGCCTTCT GCAATTCCGA CCTCGTCATC AGGGCCAAGT TCGTGGGGAC ACCAGAAGTC

130 140 150 160 170 180
AACCAGACCA CCTTATACCA GCGTTATGAG ATCAAGATGA CCAAGATGTA TAAAGGGTTC

190 200 210 220 230 240
CAAGCCTTAG GGGATGCCGC TGACATCCGG TTCGTCTACA CCCCCGCCAT GGAGAGTGTC

250 260 270 280 290 300
TGCGGATACT TCCACAGGTC CCACAACCGC AGCGAGGAGT TTCTCATTGC TGGAAAACTG

310 320 330 340 350 360
CAGGATGGAC TCTTGCACAT CACTACCTGC AGTTTCGTGG CTCCCTGGAA CAGCCTGAGC

370 380 390 400 410 420
TTAGCTCAGC GCCGGGGCTT CACCAAGACC TACACTGTTG GCTGTGAGGA ATGCACAGTG

430 440 450 460 470 480
TTTCCCTGTT TATCCATCCC CTGCAAACTG CAGAGTGGCA CTCATTGCTT GTGGACGGAC

490 500 510 520 530 540
CAGCTCCTCC AAGGCTCTGA AAAGGGCTTC CAGTCCCGTC ACCTTGCCTG CCTGCCTCGG

550 560 570 580 590 600
GAGCCAGGGC TGTGCACCTG GCAGTCCCTG CGGTCCCAGA TAGCCTGAAT CCTGCCCGGA
610 620 630 640 650 660
GTGGAAGCTG AAGCCTGCAC AGTGTCCACC CTGTTCCCAC TCCCATCTTT CTTCCGGACA

670 680 690 700
ATGAAATAAA GAGTTACCAC CCAGCAAAAA AAAAAAGGAA TTC

2. A recombinant-DNA cloning vector capable of expression in a bacterial system comprising a nucleotide sequence capable of directing intracellular production of metalloproteinase inhibitors having a cysteine residue at the N-terminal, wherein said vector comprises a nucleotide sequence containing at least a portion of the following nucleotides coding for an active metalloproteinase inhibitor:

10 20 30 40 50 60
GTTGTTGCTG TGGCTGATAG CCCCAGCAGG GCCTGCACCT GTGTCCCACC CCACCCACAG

70 80 90 100 110 120
ACGGCCTTCT GCAATTCCGA CCTCGTCATC AGGGCCAAGT TCGTGGGGAC ACCAGAAGTC

130 140 150 160 170 180
AACCAGACCA CCTTATACCA GCGTTATGAG ATCAAGATGA CCAAGATGTA TAAAGGGTTC

190 200 210 220 230 240
CAAGCCTTAG GGGATGCCGC TGACATCCGG TTCGTCTACA CCCCCGCCAT GGAGAGTGTC

250 260 270 280 290 300
TGCGGATACT TCCACAGGTC CCACAACCGC AGCGAGGAGT TTCTCATTGC TGGAAAACTG

310 320 330 340 350 360
CAGGATGGAC TCTTGCACAT CACTACCTGC AGTTTCGTGG CTCCCTGGAA CAGCCTGAGC

370 380 390 400 410 420
TTAGCTCAGC GCCGGGGCTT CACCAAGACC TACACTGTTG GCTGTGAGGA ATGCACAGTG

430 440 450 460 470 480
TTTCCCTGTT TATCCATCCC CTGCAAACTG CAGAGTGGCA CTCATTGCTT GTGGACGGAC
490 500 510 520 530 540
CAGCTCCTCC AAGGCTCTGA AAAGGGCTTC CAGTCCCGTC ACCTTGCCTG CCTGCCTCGG

550 560 570 580 590 600
GAGCCAGGGC TGTGCACCTG GCAGTCCCTG CGGTCCCAGA TAGCCTGAAT CCTGCCCGGA

610 620 630 640 650 660
GTGGAAGCTG AAGCCTGCAC AGTGTCCACC CTGTTCCCAC TCCCATCTTT CTTCCGGACA

670 680 690 700
ATGAAATAAA GAGTTACCAC CCAGCAAAAA AAAAAAGGAA TTC

3. The vector pUC9 - F5/237P10, deposited under ATCC 53003.

4. A recombinant-DNA method for bacterial production of a metalloproteinase inhibitor having a cysteine residue at the N-terminal comprising:
(a) preparation of a portable DNA sequence capable of directing a host bacterium to produce a protein sequence having potential for metalloproteinase inhibitor activity;
(b) cloning the portable DNA sequence into a vector capable of being transferred into and replicating in a host bacterium, such vector containing operational elements for the portable DNA sequence;
(c) transferring the vector containing the portable DNA sequence and operational elements into a host bacterium capable of expressing the metalloproteinase inhibitor protein;
(d) culturing the host bacterium under conditions appropriate for amplification of the vector and expression of the inhibitor; and
(e) in either order:
(i) harvesting the inhibitor; and
(ii) causing the inhibitor to assume an active, tertiary structure whereby it possesses metal - loproteinase inhibitor activity.

5. The method of Claim 4 wherein said metalloproteinase inhibitor is collagenase inhibitor.

6. The method of Claim 5 wherein said portable DNA sequence contains a portion of the following sequence:

10 20 30 40 50 60
GTTGTTGCTG TGGCTGATAG CCCCAGCAGG GCCTGCACCT GTGTCCCACC CCACCCACAG

70 80 90 100 110 120
ACGGCCTTCT GCAATTCCGA CCTCGTCATC AGGGCCAAGT TCGTGGGGAC ACCAGAAGTC

130 140 150 160 170 180
AACCAGACCA CCTTATACCA GCGTTATGAG ATCAAGATGA CCAAGATGTA TAAAGGGTTC

190 200 210 220 230 240
CAAGCCTTAG GGGATGCCGC TGACATCCGG TTCGTCTACA CCCCCGCCAT GGAGAGTGTC

250 260 270 280 290 300
TGCGGATACT TCCACAGGTC CCACAACCGC AGCGAGGAGT TTCTCATTGC TGGAAAACTG

310 320 330 340 350 360
CAGGATGGAC TCTTGCACAT CACTACCTGC AGTTTCGTGG CTCCCTGGAA CAGCCTGAGC

370 380 390 400 410 420
TTAGCTCAGC GCCGGGGCTT CACCAAGACC TACACTGTTG GCTGTGAGGA ATGCACAGTG

430 440 450 460 470 480
TTTCCCTGTT TATCCATCCC CTGCAAACTG CAGAGTGGCA CTCATTGCTT GTGGACGGAC

490 500 510 520 530 540
CAGCTCCTCC AAGGCTCTGA AAAGGGCTTC CAGTCCCGTC ACCTTGCCTG CCTGCCTCGG

550 560 570 580 590 600
GAGCCAGGGC TGTGCACCTG GCAGTCCCTG CGGTCCCAGA TAGCCTGAAT CCTGCCCGGA

610 620 630 640 650 660
GTGGAAGCTG AAGCCTGCAC AGTGTCCACC CTGTTCCCAC TCCCATCTTT CTTCCGGACA

670 680 690 700
ATGAAATAAA GAGTTACCAC CCAGCAAAAA AAAAAAGGAA TTC

7. The method of Claim 4 wherein the vector of Claim 4 is used.

8. The method of Claim 4 wherein said host bacterium is a member of the genus Bacillus.

9. The method of Claim 8 wherein said bacterium is Bacillus subtilis.

10. The method of Claim 4 wherein said bacterium is Escherichia coli.

11. The method of Claim 4 wherein said bacterium is a member of the genus Pseudomonas.

12. The method of Claim 11 wherein said bacterium is Pseudomonas aeruginosa.

13. The method of Claim 4 wherein said inhibitor is harvested prior to being caused to assume said active, tertiary structure.

14. The method of Claim 4 wherein said inhibitor is caused to assume said active, tertiary structure prior to being harvested.

15. A metalloproteinase inhibitor which is biologically equivalent to the collagenase inhibitor isolable from human skin fibroblasts produced by the method of Claim 4.

16. The microorganism C600/pUC9 - F5/237P1 0 having ATCC Accession No. 53003.

17. A portable DNA sequence capable of directing intracellular production of metalloproteinase inhibitors comprising the nucleotide sequence:

10 20 30 40 50 60
GGCCATCGCC GCAGATCCAG CGCCCAGAGA GACACCAGAG AACCCACCAT GGCCCCCTTT

70 80 90 100 110 120
GACCCCTGGC TTCTGCATCC TGTTGTTGCT GTGGCTGATA GCCCCAGCAG GGCCTGCACC

130 140 150 160 170 180
TGTGTCCCAC CCCACCCACA GACGGCCTTC TGCAATTCCG ACCTCGTCAT CAGGGCCAAG

190 200 210 220 230 240
TTCGTGGGGA CACCAGAAGT CAACCAGACC ACCTTATACC AGCGTTATGA GATCAAGATG

250 260 270 280 290 300
ACCAAGATGT ATAAAGGGTT CCAAGCCTTA GGGGATGCCG CTGACATCCG GTTCGTCTAC

310 320 330 340 350 360
ACCCCCGCCA TGGAGAGTGT CTGCGGATAC TTCCACAGGT CCCACAACCG CAGCGAGGAG

370 380 390 400 410 420
TTTCTCATTG CTGGAAAACT GCAGGATGGA CTCTTGCACA TCACTACCTG CAGTTTCGTG

430 440 450 460 470 480
GCTCCCTGGA ACAGCCTGAG CTTAGCTCAG CGCCGGGGCT TCACCAAGAC CTACACTGTT

490 500 510 520 530 540
GGCTGTGAGG AATGCACAGT GTTTCCCTGT TTATCCATCC CCTGCAAACT GCAGAGTGGC

550 560 570 580 590 600
ACTCATTGCT TGTGGACGGA CCAGCTCCTC CAAGGCTCTG AAAAGGGCTT CCAGTCCCGT

610 620 630 640 650 660
CACCTTGCCT GCCTGCCTCG GGAGCCAGGG CTGTGCACCT GGCAGTCCCT GCGGTCCCAG

670 680 690 700 710 720
ATAGCCTGAA TCCTGCCCGG AGTGGAAGCT GAAGCCTGCA CAGTGTCCAC CCTGTTCCCA

730 740 750 760 770 780
CTCCCATCTT TCTTCCGGAC AATGAAATAA AGAGTTACCA CCCAGCAAAA AAAAAAAGGA

18. A recombinant-DNA method for production of metalloproteinase inhibitors having a cysteine residue at the N-terminal from animal cells, comprising:
(a) Preparation of a portable DNA sequence capable of directing a host animal cell to produce a protein having metalloproteinase inhibitor activity;
(b) Cloning the portable DNA sequence into a vector capable of being transferred into and replicating in a host animal cell, said vector containing operational elements for the portable DNA sequence;
(c) Amplifying the vector containing the portable DNA sequence and operational elements in a bacterial host;
(d) Transferring the amplified recombinant vector into a host animal cell capable of expressing the metalloproteinase inhibitor protein;
(e) Culturing the host animal cells under conditions appropriate for expression of the inhibitor; and
(f) in either order:
(i) Harvesting the inhibitor; and
(ii) Causing the inhibitor to assume an active, tertiary structure whereby it possesses metal - loproteinase inhibitor activity.

19. The method of Claim 18 wherein said vector contains a portion of the following DNA sequence coding for an active metalloproteinase inhibitor:

10 20 30 40 50 60
GGCCATCGCC GCAGATCCAG CGCCCAGAGA GACACCAGAG AACCCACCAT GGCCCCCTTT
70 80 90 100 110 120
GAGCCCCTGG CTTCTGGCAT CCTGTTGTTG CTGTGGCTGA TAGCCCCCAG CAGGGCCTGC
130 140 150 160 170 180
ACCTGTGTCC CACCCCACCC ACAGACGGCC TTCTGCAATT CCGACCTCGT CATCAGGGCC

190 200 210 220 230 240
AAGTTCGTGG GGACACCAGA AGTCAACCAG ACCACCTTAT ACCAGCGTTA TGAGATCAAG

250 260 270 280 290 300
ATGACCAAGA TGTATAAAGG GTTCCAAGCC TTAGGGGATG CCGCTGACAT CCGGTTCGTC

310 320 330 340 350 360
TACACCCCCG CCATGGAGAG TGTCTGCGGA TACTTCCACA GGTCCCACAA CCGCAGCGAG

370 380 390 400 410 420
GAGTTTCTCA TTGCTGGAAA ACTGCAGGAT GGACTCTTGC ACATCACTAC CTGCAGTTTC

430 440 450 460 470 480
GTGGCTCCCT GGAACAGCCT GAGCTTAGCT CAGCGCCGGG GCTTCACCAA GACCTACACT

490 500 510 520 530 540
GTTGGCTGTG AGGAATGCAC AGTGTTTCCC TGTTTATCCA TCCCCTGCAA ACTGCAGAGT

550 560 570 580 590 600
GGCACTCATT GCTTGTGGAC GGACCAGCTC CTCCAAGGCT CTGAAAAGGG CTTCCAGTCC

610 620 630 640 650 660
CGTCACCTTG CCTGCCTGCC TCGGGAGCCA GGGCTGTGCA CCTGGCAGTC CCTGCGGTCC

670 680 690 700 710 720
CAGATAGCCT GAATCCTGCC CGGAGTGGAA GCTGAAGCCT GCACAGTGTC CACCCTGTTC

730 740 750 760 770 780
CCACTCCCAT CTTTCTTCCG GACAATGAAA TAAAGAGTTA CCACCCAGCA AAAAAAAAAA

GGAATTC

## Claims (Claims for the following Contracting State(s) : AT)

1. A portable DNA sequence capable of directing intracellular production of metalloproteinase inhibitors comprising the sequence:

10 20 30 40 50 60
GTTGTTGCTG TGGCTGATAG CCCCAGCAGG GCCTGCACCT GTGTCCCACC CCACCCACAG

70 80 90 100 110 120
ACGGCCTTCT GCAATTCCGA CCTCGTCATC AGGGCCAAGT TCGTGGGGAC ACCAGAAGTC

130 140 150 160 170 180
AACCAGACCA CCTTATACCA GCGTTATGAG ATCAAGATGA CCAAGATGTA TAAAGGGTTC

190 200 210 220 230 240
CAAGCCTTAG GGGATGCCGC TGACATCCGG TTCGTCTACA CCCCCGCCAT GGAGAGTGTC

250 260 270 280 290 300
TGCGGATACT TCCACAGGTC CCACAACCGC AGCGAGGAGT TTCTCATTGC TGGAAAACTG

310 320 330 340 350 360
CAGGATGGAC TCTTGCACAT CACTACCTGC AGTTTCGTGG CTCCCTGGAA CAGCCTGAGC

370 380 390 400 410 420
TTAGCTCAGC GCCGGGGCTT CACCAAGACC TACACTGTTG GCTGTGAGGA ATGCACAGTG

430 440 450 460 470 480
TTTCCCTGTT TATCCATCCC CTGCAAACTG CAGAGTGGCA CTCATTGCTT GTGGACGGAC

490 500 510 520 530 540
CAGCTCCTCC AAGGCTCTGA AAAGGGCTTC CAGTCCCGTC ACCTTGCCTG CCTGCCTCGG

550 560 570 580 590 600
GAGCCAGGGC TGTGCACCTG GCAGTCCCTG CGGTCCCAGA TAGCCTGAAT CCTGCCCGGA
610 620 630 640 650 660
GTGGAAGCTG AAGCCTGCAC AGTGTCCACC CTGTTCCCAC TCCCATCTTT CTTCCCGACA

670 680 690 700
ATGAAATAAA GAGTTACCAC CCAGCAAAAA AAAAAAGGAA TTC

2. A recombinant-DNA cloning vector capable of expression in a bacterial system comprising a nucleotide sequence capable of directing intracellular production of metalloproteinase inhibitors having a cysteine residue at the N-terminal, wherein said vector comprises a nucleotide sequence containing at least a portion of the following nucleotides coding for an active metalloproteinase inhibitor:

10 20 30 40 50 60
GTTGTTGCTG TGGCTGATAG CCCCAGCAGG GCCTGCACCT GTGTCCCACC CCACCCACAG

70 80 90 100 110 120
ACGGCCTTCT GCAATTCCGA CCTCGTCATC AGGGCCAAGT TCGTGGGGAC ACCAGAAGTC

130 140 150 160 170 180
AACCAGACCA CCTTATACCA GCGTTATGAG ATCAAGATGA CCAAGATGTA TAAAGGGTTC

190 200 210 220 230 240
CAAGCCTTAG GGGATGCCGC TGACATCCGG TTCGTCTACA CCCCCGCCAT GGAGAGTGTC

250 260 270 280 290 300
TGCGGATACT TCCACAGGTC CCACAACCGC AGCGAGGAGT TTCTCATTGC TGGAAAACTG

310 320 330 340 350 360
CAGGATGGAC TCTTGCACAT CACTACCTGC AGTTTCGTGG CTCCCTGGAA CAGCCTGAGC

370 380 390 400 410 420
TTAGCTCAGC GCCGGGGCTT CACCAAGACC TACACTGTTG GCTGTGAGGA ATGCACAGTG

430 440 450 460 470 480
TTTCCCTGTT TATCCATCCC CTGCAAACTG CAGAGTGGCA CTCATTGCTT GTGGACGGAC
490 500 510 520 530 540
CAGCTCCTCC AAGGCTCTGA AAAGGGCTTC CAGTCCCGTC ACCTTGCCTG CCTGCCTCGG

550 560 570 580 590 600
GAGCCAGGGC TGTGCACCTG GCAGTCCCTG CGGTCCCAGA TAGCCTGAAT CCTGCCCGGA

610 620 630 640 650 660
GTGGAAGCTG AAGCCTGCAC AGTGTCCACC CTGTTCCCAC TCCCATCTTT CTTCCGGACA

670 680 690 700
ATGAAATAAA GAGTTACCAC CCAGCAAAAA AAAAAAGGAA TTC

3. The vector pUC9 - F5/237P10, deposited under ATCC 53003.

4. A recombinant-DNA method for bacterial production of a metalloproteinase inhibitor having a cysteine residue at the N-terminal comprising:
(a) preparation of a portable DNA sequence capable of directing a host bacterium to produce a protein sequence having potential for metalloproteinase inhibitor activity;
(b) cloning the portable DNA sequence into a vector capable of being transferred into and replicating in a host bacterium, such vector containing operational elements for the portable DNA sequence;
(c) transferring the vector containing the portable DNA sequence and operational elements into a host bacterium capable of expressing the metalloproteinase inhibitor protein;
(d) culturing the host bacterium under conditions appropriate for amplification of the vector and expression of the inhibitor; and
(e) in either order:
(i) harvesting the inhibitor; and
(ii) causing the inhibitor to assume an active, tertiary structure whereby it possesses metal - loproteinase inhibitor activity.

5. The method of Claim 4 wherein said metalloproteinase inhibitor is collagenase inhibitor.

6. The method of Claim 5 wherein said portable DNA sequence contains a portion of the following sequence:

10 20 30 40 50 60
GTTGTTGCTG TGGCTGATAG CCCCAGCAGG GCCTGCACCT GTGTCCCACC CCACCCACAG

70 80 90 100 110 120
ACGGCCTTCT GCAATTCCGA CCTCGTCATC AGGGCCAAGT TCGTGGGGAC ACCAGAAGTC

130 140 150 160 170 180
AACCAGACCA CCTTATACCA GCGTTATGAG ATCAAGATGA CCAAGATGTA TAAAGGGTTC

190 200 210 220 230 240
CAAGCCTTAG GGGATGCCGC TGACATCCGG TTCGTCTACA CCCCCGCCAT GGAGAGTGTC

250 260 270 280 290 300
TGCGGATACT TCCACAGGTC CCACAACCGC AGCGAGGAGT TTCTCATTGC TGGAAAACTG

310 320 330 340 350 360
CAGGATGGAC TCTTGCACAT CACTACCTGC AGTTTCGTGG CTCCCTGGAA CAGCCTGAGC

370 380 390 400 410 420
TTAGCTCAGC GCCGGGGCTT CACCAAGACC TACACTGTTG GCTGTGAGGA ATGCACAGTG

430 440 450 460 470 480
TTTCCCTGTT TATCCATCCC CTGCAAACTG CAGAGTGGCA CTCATTGCTT GTGGACGGAC

490 500 510 520 530 540
CAGCTCCTCC AAGGCTCTGA AAAGGGCTTC CAGTCCCGTC ACCTTGCCTG CCTGCCTCGG

550 560 570 580 590 600
GAGCCAGGGC TGTGCACCTG GCAGTCCCTG CGGTCCCAGA TAGCCTGAAT CCTGCCCGGA

610 620 630 640 650 660
GTGGAAGCTG AAGCCTGCAC AGTGTCCACC CTGTTCCCAC TCCCATCTTT CTTCCGGACA

670 680 690 700
ATGAAATAAA GAGTTACCAC CCAGCAAAAA AAAAAAGGAA TTC

7. The method of Claim 4 wherein the vector of Claim 4 is used.

8. The method of Claim 4 wherein said host bacterium is a member of the genus Bacillus.

9. The method of Claim 8 wherein said bacterium is Bacillus subtilis.

10. The method of Claim 4 wherein said bacterium is Escherichia coli.

11. The method of Claim 4 wherein said bacterium is a member of the genus Pseudomonas.

12. The method of Claim 11 wherein said bacterium is Pseudomonas aeruginosa.

13. The method of Claim 4 wherein said inhibitor is harvested prior to being caused to assume said active, tertiary structure.

14. The method of Claim 4 wherein said inhibitor is caused to assume said active, tertiary structure prior to being harvested.

15. A metalloproteinase inhibitor which is biologically equivalent to the collagenase inhibitor isolable from human skin fibroblasts produced by the method of Claim 4.

16. The microorganism C600/pUC9 - F5/237P1 0 having ATCC Accession No. 53003.

17. A portable DNA sequence capable of directing intracellular production of metalloproteinase inhibitors comprising the nucleotide sequence:

10 20 30 40 50 60
GGCCATCGCC GCAGATCCAG CGCCCAGAGA GACACCAGAG AACCCACCAT GGCCCCCTTT

70 80 90 100 110 120
GACCCCTGGC TTCTGCATCC TGTTGTTGCT GTGGCTGATA GCCCCAGCAG GGCCTGCACC

130 140 150 160 170 180
TGTGTCCCAC CCCACCCACA GACGGCCTTC TGCAATTCCG ACCTCGTCAT CAGGGCCAAG

190 200 210 220 230 240
TTCGTGGGGA CACCAGAAGT CAACCAGACC ACCTTATACC AGCGTTATGA GATCAAGATG

250 260 270 280 290 300
ACCAAGATGT ATAAAGGGTT CCAAGCCTTA GGGGATGCCG CTGACATCCG GTTCGTCTAC

310 320 330 340 350 360
ACCCCCGCCA TGGAGAGTGT CTGCGGATAC TTCCACAGGT CCCACAACCG CAGCGAGGAG

370 380 390 400 410 420
TTTCTCATTG CTGGAAAACT GCAGGATGGA CTCTTGCACA TCACTACCTG CAGTTTCGTG

430 440 450 460 470 480
GCTCCCTGGA ACAGCCTGAG CTTAGCTCAG CGCCGGGGCT TCACCAAGAC CTACACTGTT

490 500 510 520 530 540
GGCTGTGAGG AATGCACAGT GTTTCCCTGT TTATCCATCC CCTGCAAACT GCAGAGTGGC

550 560 570 580 590 600
ACTCATTGCT TGTGGACGGA CCAGCTCCTC CAAGGCTCTG AAAAGGGCTT CCAGTCCCGT

610 620 630 640 650 660
CACCTTGCCT GCCTGCCTCG GGAGCCAGGG CTGTGCACCT GGCAGTCCCT GCGGTCCCAG

670 680 690 700 710 720
ATAGCCTGAA TCCTGCCCGG AGTGGAAGCT GAAGCCTGCA CAGTGTCCAC CCTGTTCCCA

730 740 750 760 770 780
CTCCCATCTT TCTTCCGGAC AATGAAATAA AGAGTTACCA CCCAGCAAAA AAAAAAAGGA

18. A recombinant-DNA method for production of metalloproteinase inhibitors having a cysteine residue at the N-terminal from animal cells, comprising:
(a) Preparation of a portable DNA sequence capable of directing a host animal cell to produce a protein having metalloproteinase inhibitor activity;
(b) Cloning the portable DNA sequence into a vector capable of being transferred into and replicating in a host animal cell, said vector containing operational elements for the portable DNA sequence;
(c) Amplifying the vector containing the portable DNA sequence and operational elements in a bacterial host;
(d) Transferring the amplified recombinant vector into a host animal cell capable of expressing the metalloproteinase inhibitor protein;
(e) Culturing the host animal cells under conditions appropriate for expression of the inhibitor; and
(f) in either order:
(i) Harvesting the inhibitor; and
(ii) Causing the inhibitor to assume an active, tertiary structure whereby it possesses metal - loproteinase inhibitor activity.

19. The method of Claim 18 wherein said vector contains a portion of the following DNA sequence coding for an active metalloproteinase inhibitor:

10          20          30          40          50          60
GGCCATCGCC GCAGATCCAG CGCCCAGAGA GACACCAGAG AACCCACCAT GGCCCCCTTT

70          80          90         100         110         120
GAGCCCCTGG CTTCTGGCAT CCTGTTGTTG CTGTGGCTGA TAGCCCCCAG CAGGGCCTGC

130         140         150         160         170         180
ACCTGTGTCC CACCCCACCC ACAGACGGCC TTCTGCAATT CCGACCTCGT CATCAGGGCC

190 200 210 220 230 240
AAGTTCGTGG GGACACCAGA AGTCAACCAG ACCACCTTAT ACCAGCGTTA TGAGATCAAG

250 260 270 280 290 300
ATGACCAAGA TGTATAAAGG GTTCCAAGCC TTAGGGGATG CCGCTGACAT CCGGTTCGTC

310 320 330 340 350 360
TACACCCCCG CCATGGAGAG TGTCTGCGGA TACTTCCACA GGTCCCACAA CCGCAGCGAG

370 380 390 400 410 420
GAGTTTCTCA TTGCTGGAAA ACTGCAGGAT GGACTCTTGC ACATCACTAC CTGCAGTTTC

430 440 450 460 470 480
GTGGCTCCCT GGAACAGCCT GAGCTTAGCT CAGCGCCGGG GCTTCACCAA GACCTACACT

490 500 510 520 530 540
GTTGGCTGTG AGGAATGCAC AGTGTTTCCC TGTTTATCCA TCCCCTGCAA ACTGCAGAGT

550 560 570 580 590 600
GGCACTCATT GCTTGTGGAC GGACCAGCTC CTCCAAGGCT CTGAAAAGGG CTTCCAGTCC

610 620 630 640 650 660
CGTCACCTTG CCTGCCTGCC TCGGGAGCCA GGGCTGTGCA CCTGGCAGTC CCTGCGGTCC

670 680 690 700 710 720
CAGATAGCCT GAATCCTGCC CGGAGTGGAA GCTGAAGCCT GCACAGTGTC CACCCTGTTC

730 740 750 760 770 780
CCACTCCCAT CTTTCTTCCG GACAATGAAA TAAAGAGTTA CCACCCAGCA AAAAAAAAAA

GGAATTC

20. A method for producing a portable DNA sequence capable of directing intracellular production of metalloproteinase inhibitors, characterized in that the following DNA sequence is provided:

10 20 30 40 50 60
GTTGTTGCTG TGGCTGATAG CCCCAGCAGG GCCTGCACCT GTGTCCCACC CCACCCACAG

70 80 90 100 110 120
ACGGCCTTCT GCAATTCCGA CCTCGTCATC AGGGCCAAGT TCGTGGGGAC ACCAGAAGTC

130 140 150 160 170 180
AACCAGACCA CCTTATACCA GCGTTATGAG ATCAAGATGA CCAAGATGTA TAAAGGGTTC

190 200 210 220 230 240
CAAGCCTTAG GGGATGCCGC TGACATCCGG TTCGTCTACA CCCCCGCCAT GGAGAGTGTC

250 260 270 280 290 300
TGCGGATACT TCCACAGGTC CCACAACCGC AGCGAGGAGT TTCTCATTGC TGGAAAACTG

310 320 330 340 350 360
CAGGATGGAC TCTTGCACAT CACTACCTGC AGTTTCGTGG CTCCCTGGAA CAGCCTGAGC

370 380 390 400 410 420
TTAGCTCAGC GCCGGGGCTT CACCAAGACC TACACTGTTG GCTGTGAGGA ATGCACAGTG

430 440 450 460 470 480
TTTCCCTGTT TATCCATCCC CTGCAAACTG CAGAGTGGCA CTCATTGCTT GT[?]GA[?][?]GAC

490 500 510 520 530 540
CAGCTCCTCC AAGGCTCTGA AAAGGGCTTC CAGTCCCGTC ACCTTGCCTG CCTGCCTCGG

550 560 570 580 590 600
GAGCCAGGGC TGTGCACCTG GCAGTCCCTG CGGTCCCAGA TAGCCTGAAT CCTGCCCGGA
610 620 630 640 650 [?][?][?]
GTGGAAGCTG AAGCCTGCAC AGTGTCCACC CTGTTCCCAC TCCCATCTTT CTTC[?][?]CA[?]A

670 680 690 700
ATGAAATAAA GAGTTACCAC CCAGCAAAAA AAAAAAGGAA TTC

21. A method for preparing a recombinant DNA cloning vector capable of expression in a bacterial system by introducing a nucleotide sequence capable of directing intracellular production of metalloproteinase inhibitors having a cysteine residue at the N-terminal into a cloning vector, wherein said nucleotide sequence comprises at least a portion of the following nucleotides coding for an active metal - loproteinase inhibitor:

10 20 30 40 50 60
GTTGTTGCTG TGGCTGATAG CCCCAGCAGG GCCTGCACCT GTGTCCCACC CCACCCACAG

70 80 90 100 110 120
ACGGCCTTCT GCAATTCCGA CCTCGTCATC AGGGCCAAGT TCGTGGGGAC ACCAGAAGTC

130 140 150 160 170 180
AACCAGACCA CCTTATACCA GCGTTATGAG ATCAAGATGA CCAAGATGTA TAAAGGGTTC

190 200 210 220 230 240
CAAGCCTTAG GGGATGCCGC TGACATCCGG TTCGTCTACA CCCCCGCCAT GGAGAGTGTC

250 260 270 280 290 300
TGCGGATACT TCCACAGGTC CCACAACCGC AGCGAGGAGT TTCTCATTGC TGGAAAACTG

310 320 330 340 350 360
CAGGATGGAC TCTTGCACAT CACTACCTGC AGTTTCGTGG CTCCCTGGAA CAGCCTGAGC

370 380 390 400 410 420
TTAGCTCAGC GCCGGGGCTT CACCAAGACC TACACTGTTG GCTGTGAGGA ATGCACAGTG

430 440 450 460 470 480
TTTCCCTGTT TATCCATCCC CTGCAAACTG CAGAGTGGCA CTCATTGCTT GTGGACGGAC
490 500 510 520 530 540
CAGCTCCTCC AAGGCTCTGA AAAGGGCTTC CAGTCCCGTC ACCTTGCCTG CCTGCCTCGG

550 560 570 580 590 600
GAGCCAGGGC TGTGCACCTG GCAGTCCCTG CGGTCCCAGA TAGCCTGAAT CCTGCCCGGA

610 620 630 640 650 660
GTGGAAGCTG AAGCCTGCAC AGTGTCCACC CTGTTCCCAC TCCCATCTTT CTTCCGGACA

670 680 690 700
ATGAAATAAA GAGTTACCAC CCAGCAAAAA AAAAAAGGAA TTC

22. A method for preparing vector pUC9-F5/237P10 by isolating the vector from the microorganism deposited under ATCC 53003.

23. A method for producing a microorganism containing pUC9-F5/237P10, ATCC 53003 by introducing said plasmid into E. coli C600.

24. A method for producing a portable DNA sequence capable of directing intracellular production of metalloproteinase inhibitors by providing the following nucleotide sequence:

10 20 30 40 50 60
GGCCATCGCC GCAGATCCAG CGCCCAGAGA GACACCAGAG AACCCACCAT GGCCCCCTTT

70 80 90 100 110 120
GACCCCTGGC TTCTGCATCC TGTTGTTGCT GTGGCTGATA GCCCCAGCAG GGCCTGCACC

130 140 150 160 170 180
TGTGTCCCAC CCCACCCACA GACGGCCTTC TGCAATTCCG ACCTCGTCAT CAGGGCCAAG

190 200 210 220 230 240
TTCGTGGGGA CACCAGAAGT CAACCAGACC ACCTTATACC AGCGTTATGA GATCAAGATG

250 260 270 280 290 300
ACCAAGATGT ATAAAGGGTT CCAAGCCTTA GGGGATGCCG CTGACATCCG GTTCGTCTAC

310 320 330 340 350 360
ACCCCCGCCA TGGAGAGTGT CTGCGGATAC TTCCACAGGT CCCACAACCG CAGCGAGGAG

370 380 390 400 410 420
TTTCTCATTG CTGGAAAACT GCAGGATGGA CTCTTGCACA TCACTACCTG CAGTTTCGTG

430 440 450 460 470 480
GCTCCCTGGA ACAGCCTGAG CTTAGCTCAG CGCCGGGGCT TCACCAAGAC CTACACTGTT

490 500 510 520 530 540
GGCTGTGAGG AATGCACAGT GTTTCCCTGT TTATCCATCC CCTGCAAACT GCAGAGTGGC

550 560 570 580 590 600
ACTCATTGCT TGTGGACGGA CCAGCTCCTC CAAGGCTCTG AAAAGGGCTT CCAGTCCCGT

610 620 630 640 650 660
CACCTTGCCT GCCTGCCTCG GGAGCCAGGG CTGTGCACCT GGCAGTCCCT GCGGTCCCAG

670 680 690 700 710 720
ATAGCCTGAA TCCTGCCCGG AGTGGAAGCT GAAGCCTGCA CAGTGTCCAC CCTGTTCCCA

730 740 750 760 770 780
CTCCCATCTT TCTTCCGGAC AATGAAATAA AGAGTTACCA CCCAGCAAAA AAAAAAAGGA

## Patentansprüche (Patentansprüche für folgende(n) Vertragsstaat(en) : BE, CH, DE, DK, ES, FR, GB, GR, IE, IT, LI, LU, MC, NL, PT, SE)

1. Tragbare DNA-Sequenz, die zur Steuerung der intrazellulären Erzeugung von Metalloproteinase-Inhibitoren, die die folgende Sequenz umfassen, fähig ist:

10 20 30 40 50 60
GTTGTTGCTG TGGCTGATAG CCCCAGCAGG GCCTGCACCT GTGTCCCACC CCACCCACAG

70 80 90 100 110 120
ACGGCCTTCT GCAATTCCGA CCTCGTCATC AGGGCCAAGT TCGTGGGGAC ACCAGAAGTC

130 140 150 160 170 180
AACCAGACCA CCTTATACCA GCGTTATGAG ATCAAGATGA CCAAGATGTA TAAAGGGTTC

190 200 210 220 230 240
CAAGCCTTAG GGGATGCCGC TGACATCCGG TTCGTCTACA CCCCCGCCAT GGAGAGTGTC

250 260 270 280 290 300
TGCGGATACT TCCACAGGTC CCACAACCGC AGCGAGGAGT TTCTCATTGC TGGAAAACTG

310 320 330 340 350 360
CAGGATGGAC TCTTGCACAT CACTACCTGC AGTTTCGTGG CTCCCTGGAA CAGCCTGAGC

370 380 390 400 410 420
TTAGCTCAGC GCCGGGGCTT CACCAAGACC TACACTGTTG GCTGTGAGGA ATGCACAGTG

430 440 450 460 470 480
TTTCCCTGTT TATCCATCCC CTGCAAACTG CAGAGTGGCA CTCATTGCTT GTGGACGGAC

490 500 510 520 530 540
CAGCTCCTCC AAGGCTCTGA AAAGGGCTTC CAGTCCCGTC ACCTTGCCTG CCTGCCTCGG

550 560 570 580 590 600
GAGCCAGGGC TGTGCACCTG GCAGTCCCTG CGGTCCCAGA TAGCCTGAAT CCTGCCCGGA

610 620 630 640 650 660
GTGGAAGCTG AAGCCTGCAC AGTGTCCACC CTGTTCCCAC TCCCATCTTT CTTCCGGACA

670 680 690 700
ATGAAATAAA GAGTTACCAC CCAGCAAAAA AAAAAAGGAA TTC

2. Rekombinanter DNA-Klonierungsvektor, der zur Expression in einem bakteriellen System fähig ist, umfassend eine Nukleotidsequenz, die zum Steuern der intrazellulären Erzeugung von Metalloproteinase - Inhibitoren mit einem Cysteinrest am N - Terminus fähig ist, wobei der Vektor eine Nukleotidsequenz umfaßt, die mindestens einen Teil der folgenden, für einen aktiven Metallproteinase-Inhibitor kodierenden Nukleotide enthält:

10 20 30 40 50 60
GTTGTTGCTG TGGCTGATAG CCCCAGCAGG GCCTGCACCT GTGTCCCACC CCACCCACAG

70 80 90 100 110 120
ACGGCCTTCT GCAATTCCGA CCTCGTCATC AGGGCCAAGT TCGTGGGGAC ACCAGAAGTC

130 140 150 160 170 180
AACCAGACCA CCTTATACCA GCGTTATGAG ATCAAGATGA CCAAGATGTA TAAAGGGTTC

190 200 210 220 230 240
CAAGCCTTAG GGGATGCCGC TGACATCCGG TTCGTCTACA CCCCCGCCAT GGAGAGTGTC

250 260 270 280 290 300
TGCGGATACT TCCACAGGTC CCACAACCGC AGCGAGGAGT TTCTCATTGC TGGAAAACTG

310 320 330 340 350 360
CAGGATGGAC TCTTGCACAT CACTACCTGC AGTTTCGTGG CTCCCTGGAA CAGCCTGAGC

370 380 390 400 410 420
TTAGCTCAGC GCCGGGGCTT CACCAAGACC TACACTGTTG GCTGTGAGGA ATGCACAGTG

430 440 450 460 470 480
TTTCCCTGTT TATCCATCCC CTGCAAACTG CAGAGTGGCA CTCATTGCTT GTGGACGGAC

490 500 510 520 530 540
CAGCTCCTCC AAGGCTCTGA AAAGGGCTTC CAGTCCCGTC ACCTTGCCTG CCTGCCTCGG

550 560 570 580 590 600
GAGCCAGGGC TGTGCACCTG GCAGTCCCTG CGGTCCCAGA TAGCCTGAAT CCTGCCCGGA

610 620 630 640 650 660
GTGGAAGCTG AAGCCTGCAC AGTGTCCACC CTGTTCCCAC TCCCATCTTT CTTCCGGACA

670 680 690 700
ATGAAATAAA GAGTTACCAC CCAGCAAAAA AAAAAAGGAA TTC

3. Vektor pUC9 - F5/237P10, hinterlegt unter ATCC 53003.

4. Verfahren zur bakteriellen Erzeugung eines Metalloproteinase - Inhibitors mit einem Cysteinrest am N - Terminus mittels rekombinanter DNA, umfassend:
(a) das Herstellen einer tragbaren DNA-Sequenz, die fähig ist, ein Wirtsbakterium zu steuern, so daß es eine Proteinsequenz mit dem Potential für eine Metalloproteinase - Inhibitoraktivität erzeugt;
(b) das Klonieren der tragbaren DNA-Sequenz in einem Vektor, der in ein Wirtsbakterium übertragen werden und darin replizieren kann, wobei der Vektor Steuerungselemente für die tragbare DNA-Sequenz enthält;
(c) das Übertragen des die tragbare DNA-Sequenz und die Steuerungselemente enthaltenden Vektors in ein Wirtsbakterium, das zum Exprimieren des Metalloproteinase-Inhibitorproteins fähig ist;
(d) das Kultivieren des Wirtsbakteriums unter Bedingungen, die für die Amplifikation des Vektors und die Expression des Inhibitors geeignet sind; und
(e) in beliebiger Reihenfolge:
(i) das Ernten des Inhibitors; und
(ii) das den Inhibitor Veranlassen, eine aktive Tertiärstruktur anzunehmen, wodurch er Metallproteinase - Inhibitoraktivität besitzt.

5. Verfahren nach Anspruch 4, wobei der Metalloproteinase - Inhibitor ein Collagenase - Inhibitor ist.

6. Verfahren nach Anspruch 5, wobei die tragbare DNA-Sequenz einen Teil der folgenden Sequenz enthält:

10         20         30         40         50         60
GTTGTTGCTG TGGCTGATAG CCCCAGCAGG GCCTGCACCT GTGTCCCACC CCACCCACAG

70         80         90         100        110        120
ACGGCCTTCT GCAATTCCGA CCTCGTCATC AGGGCCAAGT TCGTGGGGAC ACCAGAAGTC

130        140        150        160        170        180
AACCAGACCA CCTTATACCA GCGTTATGAG ATCAAGATGA CCAAGATGTA TAAAGGGTTC

190        200        210        220        230        240
CAAGCCTTAG GGGATGCCGC TGACATCCGG TTCGTCTACA CCCCCGCCAT GGAGAGTGTC

250        260        270        280        290        300
TGCGGATACT TCCACAGGTC CCACAACCGC AGCGAGGAGT TTCTCATTGC TGGAAAACTG

310        320        330        340        350        360
CAGGATGGAC TCTTGCACAT CACTACCTGC AGTTTCGTGG CTCCCTGGAA CAGCCTGAGC

370        380        390        400        410        420
TTAGCTCAGC GCCGGGGCTT CACCAAGACC TACACTGTTG GCTGTGAGGA ATGCACAGTG

430        440        450        460        470        480
TTTCCCTGTT TATCCATCCC CTGCAAACTG CAGAGTGGCA CTCATTGCTT GTGGACGGAC

490        500        510        520        530        540
CAGCTCCTCC AAGGCTCTGA AAAGGGCTTC CAGTCCCGTC ACCTTGCCTG CCTGCCTCGG

550        560        570        580        590        600
GAGCCAGGGC TGTGCACCTG GCAGTCCCTG CGGTCCCAGA TAGCCTGAAT CCTGCCCGGA

610        620        630        640        650        660
GTGGAAGCTG AAGCCTGCAC AGTGTCCACC CTGTTCCCAC TCCCATCTTT CTTCCGGACA

670        680        690        700
ATGAAATAAA GAGTTACCAC CCAGCAAAAA AAAAAAGGAA TTC

7. Verfahren nach Anspruch 4, wobei der Vektor gemäß Anspruch 4 verwendet wird.

8. Verfahren nach Anspruch 4, wobei das Wirtsbakterium ein Mitglied der Gattung Bacillus ist.

9. Verfahren nach Anspruch 8, wobei das Bakterium Bacillus subtilis ist.

10. Verfahren nach Anspruch 4, wobei das Bakterium Escherichia coli ist.

11. Verfahren nach Anspruch 4, wobei das Bakterium ein Mitglied der Gattung Pseudomonas ist.

12. Verfahren nach Anspruch 11, wobei das Bakterium ein Pseudomonas aeruginosa ist.

13. Verfahren nach Anspruch 4, wobei der Inhibitor geerntet wird, bevor er dazu veranlaßt wird, die aktive Tertiärstruktur anzunehmen.

14. Verfahren nach Anspruch 4, wobei der Inhibitor veranlaßt wird, die aktive, tertiäre Struktur anzunehmen, bevor er geerntet wird.

15. Metalloproteinase - Inhibitor, der biologisch dem gemäß dem Verfahren nach Anspruch 4 aus humanen Hautfibroblasten erzeugten Collagenase - Inhibitor äquivalent ist.

16. Mikroorganismus C600/pUC9-F5/237P10 mit der ATCC - Hinterlegungs Nr. 53003.

17. Tragbare DNA-Sequenz, die zur Steuerung der intrazellulären Produktion von Metalloproteinase-Inhibitoren fähig ist, umfassend die folgende Nukleotidsequenz:

10 20 30 40 50 60
GGCCATCGCC GCAGATCCAG CGCCCAGAGA GACACCAGAG AACCCACCAT GGCCCCCTTT

70 80 90 100 110 120
GACCCCTGGC TTCTGCATCC TGTTGTTGCT GTGGCTGATA GCCCCAGCAG GGCCTGCACC

130 140 150 160 170 180
TGTGTCCCAC CCCACCCACA GACGGCCTTC TGCAATTCCG ACCTCGTCAT CAGGGCCAAG

190 200 210 220 230 240
TTCGTGGGGA CACCAGAAGT CAACCAGACC ACCTTATACC AGCGTTATGA GATCAAGATG

250 260 270 280 290 300
ACCAAGATGT ATAAAGGGTT CCAAGCCTTA GGGGATGCCG CTGACATCCG GTTCGTCTAC

310 320 330 340 350 360
ACCCCCGCCA TGGAGAGTGT CTGCGGATAC TTCCACAGGT CCCACAACCG CAGCGAGGAG

370 380 390 400 410 420
TTTCTCATTG CTGGAAAACT GCAGGATGGA CTCTTGCACA TCACTACCTG CAGTTTCGTG

430 440 450 460 470 480
GCTCCCTGGA ACAGCCTGAG CTTAGCTCAG CGCCGGGGCT TCACCAAGAC CTACACTGTT

490 500 510 520 530 540
GGCTGTGAGG AATGCACAGT GTTTCCCTGT TTATCCATCC CCTGCAAACT GCAGAGTGGC

550 560 570 580 590 600
ACTCATTGCT TGTGGACGGA CCAGCTCCTC CAAGGCTCTG AAAAGGGCTT CCAGTCCCGT

610 620 630 640 650 660
CACCTTGCCT GCCTGCCTCG GGAGCCAGGG CTGTGCACCT GGCAGTCCCT GCGGTCCCAG

670 680 690 700 710 720
ATAGCCTGAA TCCTGCCCGG AGTGGAAGCT GAAGCCTGCA CAGTGTCCAC CCTGTTCCCA

730 740 750 760 770 780
CTCCCATCTT TCTTCCGGAC AATGAAATAA AGAGTTACCA CCCAGCAAAA AAAAAAAGGA

18. Verfahren zum Herstellen eines Metalloproteinase - Inhibitors aus Tierzellen mit einem Cysteinrest am N - Terminus mittels rekombinanter DNA, umfassend:
(a) das Herstellen einer tragbaren DNA-Sequenz, die fähig ist, eine Wirtstierzelle zu steuern, so daß sie ein Protein mit Metalloproteinase - Inhibitoraktivität erzeugt;
(b) das Klonieren der tragbaren DNA-Sequenz in einen Vektor, der in eine Wirtstierzelle übertragen werden und darin replizieren kann, wobei der Vektor Steuerungselemente für die tragbare DNA-Sequenz enthält;
(c) das Amplifizieren des die tragbare DNA-Sequenz und die Steuerungselemente enthaltenden Vektors in einem bakteriellen Wirt;
(d) das Übertragen des amplifizierten rekombinanten Vektors in eine Wirtstierzelle, die zur Expression des Metalloproteinase - Inhibitor - proteins fähig ist;
(e) das Kultivieren der Wirtstierzelle unter zur Expression des Inhibitors geeigneten Bedingungen; und
(f) in beliebiger Reihenfolge:
(i) das Ernten des Inhibitors; und
(ii) das den Inhibitor Veranlassen, eine aktive Tertiärstruktur anzunehmen, wodurch er Metalloproteinase - Inhibitor - aktivität besitzt.

19. Verfahren nach Anspruch 18, wobei der Vektor einen Teil der folgenden, für einen aktiven Metalloproteinase - Inhibitor kodierenden Sequenz enthält:

10 20 30 40 50 60
GGCCATCGCC GCAGATCCAG CGCCCAGAGA GACACCAGAG AACCCACCAT GGCCCCCTTT

70 80 90 100 110 120
GAGCCCCTGG CTTCTGGCAT CCTGTTGTTG CTGTGGCTGA TAGCCCCCAG CAGGGCCTGC

130 140 150 160 170 180
ACCTGTGTCC CACCCCACCC ACAGACGGCC TTCTGCAATT CCGACCTCGT CATCAGGGCC

190 200 210 220 230 240
AAGTTCGTGG GGACACCAGA AGTCAACCAG ACCACCTTAT ACCAGCGTTA TGAGATCAAG

250 260 270 280 290 300
ATGACCAAGA TGTATAAAGG GTTCCAAGCC TTAGGGGATG CCGCTGACAT CCGGTTCGTC

310 320 330 340 350 360
TACACCCCCG CCATGGAGAG TGTCTGCGGA TACTTCCACA GGTCCCACAA CCGCAGCGAG

370 380 390 400 410 420
GAGTTTCTCA TTGCTGGAAA ACTGCAGGAT GGACTCTTGC ACATCACTAC CTGCAGTTTC

430 440 450 460 470 480
GTGGCTCCCT GGAACAGCCT GAGCTTAGCT CAGCGCCGGG GCTTCACCAA GACCTACACT

490 500 510 520 530 540
GTTGGCTGTG AGGAATGCAC AGTGTTTCCC TGTTTATCCA TCCCCTGCAA ACTGCAGAGT

550 560 570 580 590 600
GGCACTCATT GCTTGTGGAC GGACCAGCTC CTCCAAGGCT CTGAAAAGGG CTTCCAGTCC

610 620 630 640 650 660
CGTCACCTTG CCTGCCTGCC TCGGGAGCCA GGGCTGTGCA CCTGGCAGTC CCTGCGGTCC

670 680 690 700 710 720
CAGATAGCCT GAATCCTGCC CGGAGTGGAA GCTGAAGCCT GCACAGTGTC CACCCTGTTC

730 740 750 760 770 780
CCACTCCCAT CTTTCTTCCG GACAATGAAA TAAAGAGTTA CCACCCAGCA AAAAAAAAAA

GGAATTC

## Patentansprüche (Patentansprüche für folgende(n) Vertragsstaat(en) : AT)

1. Tragbare DNA-Sequenz, die zur Steuerung der intrazellulären Erzeugung von Metalloproteinase-Inhibitoren, die die folgende Sequenz umfassen, fähig ist:

10 20 30 40 50 60
GTTGTTGCTG TGGCTGATAG CCCCAGCAGG GCCTGCACCT GTGTCCCACC CCACCCACAG

70 80 90 100 110 120
ACGGCCTTCT GCAATTCCGA CCTCGTCATC AGGGCCAAGT TCGTGGGGAC ACCAGAAGTC

130 140 150 160 170 180
AACCAGACCA CCTTATACCA GCGTTATGAG ATCAAGATGA CCAAGATGTA TAAAGGGTTC

190 200 210 220 230 240
CAAGCCTTAG GGGATGCCGC TGACATCCGG TTCGTCTACA CCCCCGCCAT GGAGAGTGTC

250 260 270 280 290 300
TGCGGATACT TCCACAGGTC CCACAACCGC AGCGAGGAGT TTCTCATTGC TGGAAAACTG

310 320 330 340 350 360
CAGGATGGAC TCTTGCACAT CACTACCTGC AGTTTCGTGG CTCCCTGGAA CAGCCTGAGC

370 380 390 400 410 420
TTAGCTCAGC GCCGGGGCTT CACCAAGACC TACACTGTTG GCTGTGAGGA ATGCACAGTG

430 440 450 460 470 480
TTTCCCTGTT TATCCATCCC CTGCAAACTG CAGAGTGGCA CTCATTGCTT GTGGACGGAC

490 500 510 520 530 540
CAGCTCCTCC AAGGCTCTGA AAAGGGCTTC CAGTCCCGTC ACCTTGCCTG CCTGCCTCGG

550 560 570 580 590 600
GAGCCAGGGC TGTGCACCTG GCAGTCCCTG CGGTCCCAGA TAGCCTGAAT CCTGCCCGGA

610 620 630 640 650 660
GTGGAAGCTG AAGCCTGCAC AGTGTCCACC CTGTTCCCAC TCCCATCTTT CTTCCGGACA

670 680 690 700
ATGAAATAAA GAGTTACCAC CCAGCAAAAA AAAAAAGGAA TTC

2. Rekombinanter DNA-Klonierungsvektor, der zur Expression in einem bakteriellen System fähig ist, umfassend eine Nukleotidsequenz, die zum Steuern der intrazellulären Erzeugung von Metalloproteinase - Inhibitoren mit einem Cysteinrest am N - Terminus fähig ist, wobei der Vektor eine Nukleotidsequenz umfaßt, die mindestens einen Teil der folgenden, für einen aktiven Metallproteinase-Inhibitor kodierenden Nukleotide enthält:

10 20 30 40 50 60
GTTGTTGCTG TGGCTGATAG CCCCAGCAGG GCCTGCACCT GTGTCCCACC CCACCCACAG

70 80 90 100 110 120
ACGGCCTTCT GCAATTCCGA CCTCGTCATC AGGGCCAAGT TCGTGGGGAC ACCAGAAGTC

130 140 150 160 170 180
AACCAGACCA CCTTATACCA GCGTTATGAG ATCAAGATGA CCAAGATGTA TAAAGGGTTC

190 200 210 220 230 240
CAAGCCTTAG GGGATGCCGC TGACATCCGG TTCGTCTACA CCCCCGCCAT GGAGAGTGTC

250 260 270 280 290 300
TGCGGATACT TCCACAGGTC CCACAACCGC AGCGAGGAGT TTCTCATTGC TGGAAAACTG

310 320 330 340 350 360
CAGGATGGAC TCTTGCACAT CACTACCTGC AGTTTCGTGG CTCCCTGGAA CAGCCTGAGC

370 380 390 400 410 420
TTAGCTCAGC GCCGGGGCTT CACCAAGACC TACACTGTTG GCTGTGAGGA ATGCACAGTG

430 440 450 460 470 480
TTTCCCTGTT TATCCATCCC CTGCAAACTG CAGAGTGGCA CTCATTGCTT GTGGACGGAC

490 500 510 520 530 540
CAGCTCCTCC AAGGCTCTGA AAAGGGCTTC CAGTCCCGTC ACCTTGCCTG CCTGCCTCGG

550 560 570 580 590 600
GAGCCAGGGC TGTGCACCTG GCAGTCCCTG CGGTCCCAGA TAGCCTGAAT CCTGCCCGGA

610 620 630 640 650 660
GTGGAAGCTG AAGCCTGCAC AGTGTCCACC CTGTTCCCAC TCCCATCTTT CTTCCGGACA

670 680 690 700
ATGAAATAAA GAGTTACCAC CCAGCAAAAA AAAAAAGGAA TTC

3. Vektor pUC9 - F5/237P10, hinterlegt unter ATCC 53003.

4. Verfahren zur bakteriellen Erzeugung eines Metalloproteinase - Inhibitors mit einem Cysteinrest am N - Terminus mittels rekombinanter DNA, umfassend:
(a) das Herstellen einer tragbaren DNA-Sequenz, die fähig ist, ein Wirtsbakterium zu steuern, so daß es eine Proteinsequenz mit dem Potential für eine Metalloproteinase - Inhibitoraktivität erzeugt;
(b) das Klonieren der tragbaren DNA-Sequenz in einem Vektor, der in ein Wirtsbakterium übertragen werden und darin replizieren kann, wobei der Vektor Steuerungselemente für die tragbare DNA-Sequenz enthält;
(c) das Übertragen des die tragbare DNA-Sequenz und die Steuerungselemente enthaltenden Vektors in ein Wirtsbakterium, das zum Exprimieren des Metalloproteinase-Inhibitorproteins fähig ist;
(d) das Kultivieren des Wirtsbakteriums unter Bedingungen, die für die Amplifikation des Vektors und die Expression des Inhibitors geeignet sind; und
(e) in beliebiger Reihenfolge:
(i) das Ernten des Inhibitors; und
(ii) das den Inhibitor Veranlassen, eine aktive Tertiärstruktur anzunehmen, wodurch er Metallproteinase - Inhibitoraktivität besitzt.

5. Verfahren nach Anspruch 4, wobei der Metalloproteinase - Inhibitor ein Collagenase - Inhibitor ist.

6. Verfahren nach Anspruch 5, wobei die tragbare DNA-Sequenz einen Teil der folgenden Sequenz enthält:

10 20 30 40 50 60
GTTGTTGCTG TGGCTGATAG CCCCAGCAGG GCCTGCACCT GTGTCCCACC CCACCCACAG

70 80 90 100 110 120
ACGGCCTTCT GCAATTCCGA CCTCGTCATC AGGGCCAAGT TCGTGGGGAC ACCAGAAGTC

130 140 150 160 170 180
AACCAGACCA CCTTATACCA GCGTTATGAG ATCAAGATGA CCAAGATGTA TAAAGGGTTC

190 200 210 220 230 240
CAAGCCTTAG GGGATGCCGC TGACATCCGG TTCGTCTACA CCCCCGCCAT GGAGAGTGTC

250 260 270 280 290 300
TGCGGATACT TCCACAGGTC CCACAACCGC AGCGAGGAGT TTCTCATTGC TGGAAAACTG

310 320 330 340 350 360
CAGGATGGAC TCTTGCACAT CACTACCTGC AGTTTCGTGG CTCCCTGGAA CAGCCTGAGC

370 380 390 400 410 420
TTAGCTCAGC GCCGGGGCTT CACCAAGACC TACACTGTTG GCTGTGAGGA ATGCACAGTG

430 440 450 460 470 480
TTTCCCTGTT TATCCATCCC CTGCAAACTG CAGAGTGGCA CTCATTGCTT GTGGACGGAC

490 500 510 520 530 540
CAGCTCCTCC AAGGCTCTGA AAAGGGCTTC CAGTCCCGTC ACCTTGCCTG CCTGCCTCGG

550 560 570 580 590 600
GAGCCAGGGC TGTGCACCTG GCAGTCCCTG CGGTCCCAGA TAGCCTGAAT CCTGCCCGGA

610 620 630 640 650 660
GTGGAAGCTG AAGCCTGCAC AGTGTCCACC CTGTTCCCAC TCCCATCTTT CTTCCGGACA

670 680 690 700
ATGAAATAAA GAGTTACCAC CCAGCAAAAA AAAAAAGGAA TTC

7. Verfahren nach Anspruch 4, wobei der Vektor gemäß Anspruch 4 verwendet wird.

8. Verfahren nach Anspruch 4, wobei das Wirtsbakterium ein Mitglied der Gattung Bacillus ist.

9. Verfahren nach Anspruch 8, wobei das Bakterium Bacillus subtilis ist.

10. Verfahren nach Anspruch 4, wobei das Bakterium Escherichia coli ist.

11. Verfahren nach Anspruch 4, wobei das Bakterium ein Mitglied der Gattung Pseudomonas ist.

12. Verfahren nach Anspruch 11, wobei das Bakterium ein Pseudomonas aeruginosa ist.

13. Verfahren nach Anspruch 4, wobei der Inhibitor geerntet wird, bevor er dazu veranlaßt wird, die aktive Tertiärstruktur anzunehmen.

14. Verfahren nach Anspruch 4, wobei der Inhibitor veranlaßt wird, die aktive, tertiäre Struktur anzunehmen, bevor er geerntet wird.

15. Metalloproteinase - Inhibitor, der biologisch dem gemäß dem Verfahren nach Anspruch 4 aus humanen Hautfibroblasten erzeugten Collagenase - Inhibitor äquivalent ist.

16. Mikroorganismus C600/pUC9-F5/237P10 mit der ATCC - Hinterlegungs Nr. 53003.

17. Tragbare DNA-Sequenz, die zur Steuerung der intrazellulären Produktion von Metalloproteinase-Inhibitoren fähig ist, umfassend die folgende Nukleotidsequenz:

10 20 30 40 50 60
GGCCATCGCC GCAGATCCAG CGCCCAGAGA GACACCAGAG AACCCACCAT GGCCCCCTTT

70 80 90 100 110 120
GACCCCTGGC TTCTGCATCC TGTTGTTGCT GTGGCTGATA GCCCCAGCAG GGCCTGCACC

130 140 150 160 170 180
TGTGTCCCAC CCCACCCACA GACGGCCTTC TGCAATTCCG ACCTCGTCAT CAGGGCCAAG

190 200 210 220 230 240
TTCGTGGGGA CACCAGAAGT CAACCAGACC ACCTTATACC AGCGTTATGA GATCAAGATG

250 260 270 280 290 300
ACCAAGATGT ATAAAGGGTT CCAAGCCTTA GGGGATGCCG CTGACATCCG GTTCGTCTAC

310 320 330 340 350 360
ACCCCCGCCA TGGAGAGTGT CTGCGGATAC TTCCACAGGT CCCACAACCG CAGCGAGGAG

370 380 390 400 410 420
TTTCTCATTG CTGGAAAACT GCAGGATGGA CTCTTGCACA TCACTACCTG CAGTTTCGTG

430 440 450 460 470 480
GCTCCCTGGA ACAGCCTGAG CTTAGCTCAG CGCCGGGGCT TCACCAAGAC CTACACTGTT

490 500 510 520 530 540
GGCTGTGAGG AATGCACAGT GTTTCCCTGT TTATCCATCC CCTGCAAACT GCAGAGTGGC

550 560 570 580 590 600
ACTCATTGCT TGTGGACGGA CCAGCTCCTC CAAGGCTCTG AAAAGGGCTT CCAGTCCCGT

610 620 630 640 650 660
CACCTTGCCT GCCTGCCTCG GGAGCCAGGG CTGTGCACCT GGCAGTCCCT GCGGTCCCAG

670 680 690 700 710 720
ATAGCCTGAA TCCTGCCCGG AGTGGAAGCT GAAGCCTGCA CAGTGTCCAC CCTGTTCCCA

730 740 750 760 770 780
CTCCCATCTT TCTTCCGGAC AATGAAATAA AGAGTTACCA CCCAGCAAAA AAAAAAAGGA

18. Verfahren zum Herstellen eines Metalloproteinase - Inhibitors aus Tierzellen mit einem Cysteinrest am N - Terminus mittels rekombinanter DNA, umfassend:
(a) das Herstellen einer tragbaren DNA-Sequenz, die fähig ist, eine Wirtstierzelle zu steuern, so daß sie ein Protein mit Metalloproteinase - Inhibitoraktivität erzeugt;
(b) das Klonieren der tragbaren DNA-Sequenz in einen Vektor, der in eine Wirtstierzelle übertragen werden und darin replizieren kann, wobei der Vektor Steuerungselemente für die tragbare DNA-Sequenz enthält;
(c) das Amplifizieren des die tragbare DNA-Sequenz und die Steuerungselemente enthaltenden Vektors in einem bakteriellen Wirt;
(d) das Übertragen des amplifizierten rekombinanten Vektors in eine Wirtstierzelle, die zur Expression des Metalloproteinase - Inhibitor - proteins fähig ist;
(e) das Kultivieren der Wirtstierzelle unter zur Expression des Inhibitors geeigneten Bedingungen; und
(f) in beliebiger Reihenfolge:
(i) das Ernten des Inhibitors; und
(ii) das den Inhibitor Veranlassen, eine aktive Tertiärstruktur anzunehmen, wodurch er Metalloproteinase - Inhibitoraktivität besitzt.

19. Verfahren nach Anspruch 18, wobei der Vektor einen Teil der folgenden, für einen aktiven Metalloproteinase - Inhibitor kodierenden Sequenz enthält:

10 20 30 40 50 60
GGCCATCGCC GCAGATCCAG CGCCCAGAGA GACACCAGAG AACCCACCAT GGCCCCCTTT

70 80 90 100 110 120
GAGCCCCTGG CTTCTGGCAT CCTGTTGTTG CTGTGGCTGA TAGCCCCCAG CAGGGCCTGC

130 140 150 160 170 180
ACCTGTGTCC CACCCCACCC ACAGACGGCC TTCTGCAATT CCGACCTCGT CATCAGGGCC

190 200 210 220 230 240
AAGTTCGTGG GGACACCAGA AGTCAACCAG ACCACCTTAT ACCAGCGTTA TGAGATCAAG

250 260 270 280 290 300
ATGACCAAGA TGTATAAAGG GTTCCAAGCC TTAGGGGATG CCGCTGACAT CCGGTTCGTC

310 320 330 340 350 360
TACACCCCCG CCATGGAGAG TGTCTGCGGA TACTTCCACA GGTCCCACAA CCGCAGCGAG

370 380 390 400 410 420
GAGTTTCTCA TTGCTGGAAA ACTGCAGGAT GGACTCTTGC ACATCACTAC CTGCAGTTTC

430 440 450 460 470 480
GTGGCTCCCT GGAACAGCCT GAGCTTAGCT CAGCGCCGGG GCTTCACCAA GACCTACACT

490 500 510 520 530 540
GTTGGCTGTG AGGAATGCAC AGTGTTTCCC TGTTTATCCA TCCCCTGCAA ACTGCAGAGT

550 560 570 580 590 600
GGCACTCATT GCTTGTGGAC GGACCAGCTC CTCCAAGGCT CTGAAAAGGG CTTCCAGTCC

610 620 630 640 650 660
CGTCACCTTG CCTGCCTGCC TCGGGAGCCA GGGCTGTGCA CCTGGCAGTC CCTGCGGTCC

670 680 690 700 710 720
CAGATAGCCT GAATCCTGCC CGGAGTGGAA GCTGAAGCCT GCACAGTGTC CACCCTGTTC

730 740 750 760 770 780
CCACTCCCAT CTTTCTTCCG GACAATGAAA TAAAGAGTTA CCACCCAGCA AAAAAAAAAA

GGAATTC

20. Verfahren zum Erzeugen einer tragbaren DNA- Sequenz, die zum Steuern der intrazellulären Erzeu - gung von Metalloproteinase-Inhibitor fähig ist, dadurch gekennzeichnet, daß die folgende DNA-Sequenz bereitgestellt wird:

10 20 30 40 50 60
GTTGTTGCTG TGGCTGATAG CCCCAGCAGG GCCTGCACCT GTGTCCCACC CCACCCACAG

70 80 90 100 110 120
ACGGCCTTCT GCAATTCCGA CCTCGTCATC AGGGCCAAGT TCGTGGGGAC ACCAGAAGTC

130 140 150 160 170 180
AACCAGACCA CCTTATACCA GCGTTATGAG ATCAAGATGA CCAAGATGTA TAAAGGGTTC

190 200 210 220 230 240
CAAGCCTTAG GGGATGCCGC TGACATCCGG TTCGTCTACA CCCCCGCCAT GGAGAGTGTC

250 260 270 280 290 300
TGCGGATACT TCCACAGGTC CCACAACCGC AGCGAGGAGT TTCTCATTGC TGGAAAACTG

310 320 330 340 350 360
CAGGATGGAC TCTTGCACAT CACTACCTGC AGTTTCGTGG CTCCCTGGAA CAGCCTGAGC

370 380 390 400 410 420
TTAGCTCAGC GCCGGGGCTT CACCAAGACC TACACTGTTG GCTGTGAGGA ATGCACAGTG

430 440 450 460 470 480
TTTCCCTGTT TATCCATCCC CTGCAAACTG CAGAGTGGCA CTCATTGCTT GTGGACGGAC

490 500 510 520 530 540
CAGCTCCTCC AAGGCTCTGA AAAGGGCTTC CAGTCCCGTC ACCTTGCCTG CCTGCCTCGG

550 560 570 580 590 600
GAGCCAGGGC TGTGCACCTG GCAGTCCCTG CGGTCCCAGA TAGCCTGAAT CCTGCCCGGA

610 620 630 640 650 660
GTGGAAGCTG AAGCCTGCAC AGTGTCCACC CTGTTCCCAC TCCCATCTTT CTTCCGGACA

670 680 690 700
ATGAAATAAA GAGTTACCAC CCAGCAAAAA AAAAAAGGAA TTC

21. Verfahren zum Herstellen eines rekombinanten DNA-Klonierungsvektors, der zur Expression in einem bakteriellen System fähig ist, durch Einführen einer Nukleotidsequenz, die zum Steuern der intrazellu - lären Erzeugung von Metalloproteinase - Inhibitoren mit einem Cysteinrest am N - Terminus fähig ist, in einen Klonierungsvektor, wobei die Nukleotidsequenz mindestens einen Teil der folgenden, für einen aktiven Metalloproteinase - Inhibitor kodierenden Nukleotide umfaßt:

10 20 30 40 50 60
GTTGTTGCTG TGGCTGATAG CCCCAGCAGG GCCTGCACCT GTGTCCCACC CCACCCACAG

70 80 90 100 110 120
ACGGCCTTCT GCAATTCCGA CCTCGTCATC AGGGCCAAGT TCGTGGGGAC ACCAGAAGTC

130 140 150 160 170 180
AACCAGACCA CCTTATACCA GCGTTATGAG ATCAAGATGA CCAAGATGTA TAAAGGGTTC

190 200 210 220 230 240
CAAGCCTTAG GGGATGCCGC TGACATCCGG TTCGTCTACA CCCCCGCCAT GGAGAGTGTC

250 260 270 280 290 300
TGCGGATACT TCCACAGGTC CCACAACCGC AGCGAGGAGT TTCTCATTGC TGGAAAACTG

310 320 330 340 350 360
CAGGATGGAC TCTTGCACAT CACTACCTGC AGTTTCGTGG CTCCCTGGAA CAGCCTGAGC

370 380 390 400 410 420
TTAGCTCAGC GCCGGGGCTT CACCAAGACC TACACTGTTG GCTGTGAGGA ATGCACAGTG

430 440 450 460 470 480
TTTCCCTGTT TATCCATCCC CTGCAAACTG CAGAGTGGCA CTCATTGCTT GTGGACGGAC

490 500 510 520 530 540
CAGCTCCTCC AAGGCTCTGA AAAGGGCTTC CAGTCCCGTC ACCTTGCCTG CCTGCCTCGG

550 560 570 580 590 600
GAGCCAGGGC TGTGCACCTG GCAGTCCCTG CGGTCCCAGA TAGCCTGAAT CCTGCCCGGA

610 620 630 640 650 660
GTGGAAGCTG AAGCCTGCAC AGTGTCCACC CTGTTCCCAC TCCCATCTTT CTTCCGGACA

670 680 690 700
ATGAAATAAA GAGTTACCAC CCAGCAAAAA AAAAAAGGAA TTC

22. Verfahren zum Erzeugen des Vektors pUC9-F5/237P10 durch Isolieren des Vektors aus dem mit der Hinterlegungs - Nr. ATCC 53003 hinterlegten Mikroorganismus.

23. Verfahren zum Erzeugen eines Mikroorganismus, der pUC9-F5/237P10, ATCC 53003, enthält, indem das Plasmid in E. coli C600 eingeführt wird.

24. Verfahren zum Erzeugen einer tragbaren DNA- Sequenz, die zum Steuern der intrazellulären Erzeu - gung von Metalloproteinase - Inhibitoren fähig ist, indem die folgende Nukleotidsequenz bereitgestellt wird:

10 20 30 40 50 60
GGCCATCGCC GCAGATCCAG CGCCCAGAGA GACACCAGAG AACCCACCAT GGCCCCCTTT

70 80 90 100 110 120
GACCCCTGGC TTCTGCATCC TGTTGTTGCT GTGGCTGATA GCCCCAGCAG GGCCTGCACC

130 140 150 160 170 180
TGTGTCCCAC CCCACCCACA GACGGCCTTC TGCAATTCCG ACCTCGTCAT CAGGGCCAAG

190 200 210 220 230 240
TTCGTGGGGA CACCAGAAGT CAACCAGACC ACCTTATACC AGCGTTATGA GATCAAGATG

250 260 270 280 290 300
ACCAAGATGT ATAAAGGGTT CCAAGCCTTA GGGGATGCCG CTGACATCCG GTTCGTCTAC

310 320 330 340 350 360
ACCCCCGCCA TGGAGAGTGT CTGCGGATAC TTCCACAGGT CCCACAACCG CAGCGAGGAG

370 380 390 400 410 420
TTTCTCATTG CTGGAAAACT GCAGGATGGA CTCTTGCACA TCACTACCTG CAGTTTCGTG

430 440 450 460 470 480
GCTCCCTGGA ACAGCCTGAG CTTAGCTCAG CGCCGGGGCT TCACCAAGAC CTACACTGTT

490 500 510 520 530 540
GGCTGTGAGG AATGCACAGT GTTTCCCTGT TTATCCATCC CCTGCAAACT GCAGAGTGGC

550 560 570 580 590 600
ACTCATTGCT TGTGGACGGA CCAGCTCCTC CAAGGCTCTG AAAAGGGCTT CCAGTCCCGT

610 620 630 640 650 660
CACCTTGCCT GCCTGCCTCG GGAGCCAGGG CTGTGCACCT GGCAGTCCCT GCGGTCCCAG

670 680 690 700 710 720
ATAGCCTGAA TCCTGCCCGG AGTGGAAGCT GAAGCCTGCA CAGTGTCCAC CCTGTTCCCA

730 740 750 760 770 780
CTCCCATCTT TCTTCCGGAC AATGAAATAA AGAGTTACCA CCCAGCAAAA AAAAAAAGGA

## Revendications (Revendications pour l'(les) Etat(s) contractant(s) suivant(s) : suivants : BE, CH, DE, DK, ES, FR, GB, GR, IE, IT, LI, LU, MC, NL, PT, SE)

1. Séquence d'ADN portable capable de contrôler la production intracellulaire d'inhibiteurs de métalloprotéinase, comprenant la séquence :

10 20 30 40 50 60
GTTGTTGCTG TGGCTGATAG CCCCAGCAGG GCCTGCACCT GTGTCCCACC CCACCCACAG

70 80 90 100 110 120
ACGGCCTTCT GCAATTCCGA CCTCGTCATC AGGGCCAAGT TCGTGGGGAC ACCAGAAGTC

130 140 150 160 170 180
AACCAGACCA CCTTATACCA GCGTTATGAG ATCAAGATGA CCAAGATGTA TAAAGGGTTC

190 200 210 220 230 240
CAAGCCTTAG GGGATGCCGC TGACATCCGG TTCGTCTACA CCCCCGCCAT GGAGAGTGTC

250 260 270 280 290 300
TGCGGATACT TCCACAGGTC CCACAACCGC AGCGAGGAGT TTCTCATTGC TGGAAAACTG

310 320 330 340 350 360
CAGGATGGAC TCTTGCACAT CACTACCTGC AGTTTCGTGG CTCCCTGGAA CAGCCTGAGC

370 380 390 400 410 420
TTAGCTCAGC GCCGGGGCTT CACCAAGACC TACACTGTTG GCTGTGAGGA ATGCACAGTG

430 440 450 460 470 480
TTTCCCTGTT TATCCATCCC CTGCAAACTG CAGAGTGGCA CTCATTGCTT GTGGACCCAC

490 500 510 520 530 540
CAGCTCCTCC AAGGCTCTGA AAAGGGCTTC CAGTCCCGTC ACCTTGCCTG CCTGCCTCGG

550 560 570 580 590 600
GAGCCAGGGC TGTGCACCTG GCAGTCCCTG CGGTCCCAGA TAGCCTGAAT CCTGCCCGGA
610 620 630 640 650 660
GTGGAAGCTG AAGCCTGCAC AGTGTCCACC CTGTTCCCAC TCCCATCTTT CTTCCCCACA

670 680 690 700
ATGAAATAAA GAGTTACCAC CCAGCAAAAA AAAAAAGGAA TTC

2. Vecteur de clonage d'ADN recombinant capable de s'exprimer dans un système bactérien, comprenant une séquence nucléotidique capable de contrôler la production intracellulaire d'inhibiteurs de métallo - protéinase comportant un résidu cystéine à l'extrémité N-terminale, ce vecteur comprenant une séquence nucléotidique contenant au moins une partie des nucléotides suivants codant pour un inhibiteur de métalloprotéinase actif :

10 20 30 40 50 60
GTTGTTGCTG TGGCTGATAG CCCCAGCAGG GCCTGCACCT GTGTCCCACC CCACCCACAG

70 80 90 100 110 120
ACGGCCTTCT GCAATTCCGA CCTCGTCATC AGGGCCAAGT TCGTGGGGAC ACCAGAAGTC

130 140 150 160 170 180
AACCAGACCA CCTTATACCA GCGTTATGAG ATCAAGATGA CCAAGATGTA TAAAGGGTTC

190 200 210 220 230 240
CAAGCCTTAG GGGATGCCGC TGACATCCGG TTCGTCTACA CCCCCGCCAT GGAGAGTGTC

250 260 270 280 290 300
TGCGGATACT TCCACAGGTC CCACAACCGC AGCGAGGAGT TTCTCATTGC TGGAAAACTG

310 320 330 340 350 360
CAGGATGGAC TCTTGCACAT CACTACCTGC AGTTTCGTGG CTCCCTGGAA CAGCCTGAGC

370 380 390 400 410 420
TTAGCTCAGC GCCGGGGCTT CACCAAGACC TACACTGTTG GCTGTGAGGA ATGCACAGTG

430 440 450 460 470 480
TTTCCCTGTT TATCCATCCC CTGCAAACTG CAGAGTGGCA CTCATTGCTT GTGGACGGAC
490 500 510 520 530 540
CAGCTCCTCC AAGGCTCTGA AAAGGGCTTC CAGTCCCGTC ACCTTGCCTG CCTGCCTCGG

550 560 570 580 590 600
GAGCCAGGGC TGTGCACCTG GCAGTCCCTG CGGTCCCAGA TAGCCTGAAT CCTGCCCGGA

610 620 630 640 650 660
GTGGAAGCTG AAGCCTGCAC AGTGTCCACC CTGTTCCCAC TCCCATCTTT CTTCCGGACA

670 680 690 700
ATGAAATAAA GAGTTACCAC CCAGCAAAAA AAAAAAGGAA TTC

3. Vecteur pUC9 - F5/237P10 déposé sous le numéro ATCC 53003.

4. Procédé à ADN recombinant pour la production bactérienne d'un inhibiteur de métalloprotéinase comportant un résidu cystéine à l'extrémité N-terminale, selon lequel :
(a) on prépare une séquence d'ADN portable capable de contrôler une bactérie-hôte pour produire une séquence protéique ayant une activité d'inhibition de métalloprotéinase potentielle ;
(b) on clone la séquence d'ADN portable dans un vecteur capable d'être transféré et de se répliquer dans une bactérie - hôte, ce vecteur contenant des éléments fonctionnels pour la séquence d'ADN portable ;
(c) on transfère le vecteur contenant la séquence d'ADN portable et des éléments fonctionnels dans une bactérie - hôte capable d'exprimer la protéine inhibitrice de métalloprotéinase ;
(d) on cultive la bactérie-hôte dans des conditions appropriées pour amplifier le vecteur et exprimer l'inhibiteur ; et
(e) on procède dans n'importe quel ordre :
(i) à la récupération de l'inhibiteur ; et
(ii) au traitement de l'inhibiteur pour lui conférer une structure tertiaire active de sorte qu'il possède une activité d'inhibition de métalloprotéinase.

5. Procédé selon la revendication 4, dans lequel l'inhibiteur de métalloprotéinase, est un inhibiteur de collagénase.

6. Procédé selon la revendication 5, dans lequel la séquence d'ADN portable contient une partie de la séquence suivante :

10 20 30 40 50 60
GTTGTTGCTG TGGCTGATAG CCCCAGCAGG GCCTGCACCT GTGTCCCACC CCACCCACAG

70 80 90 100 110 120
ACGGCCTTCT GCAATTCCGA CCTCGTCATC AGGGCCAAGT TCGTGGGGAC ACCAGAAGTC

130 140 150 160 170 180
AACCAGACCA CCTTATACCA GCGTTATGAG ATCAAGATGA CCAAGATGTA TAAAGGCTTC

190 200 210 220 230 240
CAAGCCTTAG GGGATGCCGC TGACATCCGG TTCGTCTACA CCCCCGCCAT GGAGAGTGTC

250 260 270 280 290 300
TGCGGATACT TCCACAGGTC CCACAACCGC AGCGAGGAGT TTCTCATTGC TGGAAAACTG

310 320 330 340 350 360
CAGGATGGAC TCTTGCACAT CACTACCTGC AGTTTCGTGG CTCCCTGGAA CAGCCTGAGC

370 380 390 400 410 420
TTAGCTCAGC GCCGGGGCTT CACCAAGACC TACACTGTTG GCTGTGAGGA ATGCACAGTG

430 440 450 460 470 480
TTTCCCTGTT TATCCATCCC CTGCAAACTG CAGAGTGGCA CTCATTGCTT GTGGACGGAC

490 500 510 520 530 540
CAGCTCCTCC AAGGCTCTGA AAAGGGCTTC CAGTCCCGTC ACCTTGCCTG CCTGCCTCGG

550 560 570 580 590 600
GAGCCAGGGC TGTGCACCTG GCAGTCCCTG CGGTCCCAGA TAGCCTGAAT CCTGCCCGGA

610 620 630 640 650 660
GTGGAAGCTG AAGCCTGCAC AGTGTCCACC CTGTTCCCAC TCCCATCTTT CTTCCGGACA

670 680 690 700
ATGAAATAAA GAGTTACCAC CCAGCAAAAA AAAAAAGGAA TTC

7. Procédé selon la revendication 4, dans lequel on emploie le vecteur de la revendication 4.

8. Procédé selon la revendication 4, dans lequel la bactérie - hôte est un membre du genre Bacillus.

9. Procédé selon la revendication 8, dans lequel la bactérie est Bacillus subtilis.

10. Procédé selon la revendication 4, dans lequel la bactérie est Escherichia coli.

11. Procédé selon la revendication 4, dans lequel la bactérie est un membre du genre Pseudomonas.

12. Procédé selon la revendication 11, dans lequel la bactérie est Pseudomonas aeruginosa.

13. Procédé selon la revendication 4, dans lequel l'inhibiteur est récupéré avant de le traiter pour lui conférer ladite structure tertiaire active.

14. Procédé selon la revendication 4, dans lequel l'inhibiteur est traité pour lui conférer ladite structure tertiaire active avant d'être récupéré.

15. Inhibiteur de métalloprotéinase qui est biologiquement équivalent à l'inhibiteur de collagénase isolable à partir de fibroblastes cutanés humains produits selon le procédé de la revendication 4.

16. Micro - organisme C600/pUC9 - F5/237P1 0 correspondant au numéro d'enregistrement ATCC 53003.

17. Séquence d'ADN portable capable de contrôler la production intracellulaire d'inhibiteurs de métallo - protéinase, comprenant la séquence nucléotidique :

10 20 30 40 50 60
GGCCATCGCC GCAGATCCAG CGCCCAGAGA GACACCAGAG AACCCACCAT GGCCCCCTTT

70 80 90 100 110 120
GACCCCTGGC TTCTGCATCC TGTTGTTGCT GTGGCTGATA GCCCCAGCAG GGCCTGCACC

130 140 150 160 170 180
TGTGTCCCAC CCCACCCACA GACGGCCTTC TGCAATTCCG ACCTCGTCAT CAGGGCCAAG

190 200 210 220 230 240
TTCGTGGGGA CACCAGAAGT CAACCAGACC ACCTTATACC AGCGTTATGA GATCAAGATG

250 260 270 280 290 300
ACCAAGATGT ATAAAGGGTT CCAAGCCTTA GGGGATGCCG CTGACATCCG GTTCGTCTAC

310 320 330 340 350 360
ACCCCCGCCA TGGAGAGTGT CTGCGGATAC TTCCACAGGT CCCACAACCG CAGCGAGGAG

370 380 390 400 410 420
TTTCTCATTG CTGGAAAACT GCAGGATGGA CTCTTGCACA TCACTACCTG CAGTTTCGTG

430 440 450 460 470 480
GCTCCCTGGA ACAGCCTGAG CTTAGCTCAG CGCCGGGGCT TCACCAAGAC CTACACTGTT

490 500 510 520 530 540
GGCTGTGAGG AATGCACAGT GTTTCCCTGT TTATCCATCC CCTGCAAACT GCAGAGTGGC

550 560 570 580 590 600
ACTCATTGCT TGTGGACGGA CCAGCTCCTC CAAGGCTCTG AAAAGGGCTT CCAGTCCCGT

610 620 630 640 650 660
CACCTTGCCT GCCTGCCTCG GGAGCCAGGG CTGTGCACCT GGCAGTCCCT GCGGTCCCAG

670 680 690 700 710 720
ATAGCCTGAA TCCTGCCCGG AGTGGAAGCT GAAGCCTGCA CAGTGTCCAC CCTGTTCCCA

730 740 750 760 770 780
CTCCCATCTT TCTTCCGGAC AATGAAATAA AGAGTTACCA CCCAGCAAAA AAAAAAAGGA

18. Procédé à ADN recombinant pour la production d'inhibiteurs de métalloprotéinase comprenant un résidu cystéine à l'extrémité N-terminale, à partir de cellules animales, selon lequel :
(a) on prépare une séquence d'ADN portable capable de contrôler une cellule animale - hôte pour produire une protéine ayant une activité inhibitrice de métalloprotéinase ;
(b) on clone la séquence d'ADN portable dans un vecteur capable d'être transféré et de se répliquer dans une cellule animale-hôte, ce vecteur contenant des éléments fonctionnels pour la séquence d'ADN portable ;
(c) on amplifie le vecteur contenant la séquence d'ADN portable et des éléments fonctionnels dans un hôte bactérien ;
(d) on transfère le vecteur recombinant amplifié dans une cellule animale - hôte capable d'exprimer la protéine inhibitrice de métalloprotéinase ;
(e) on cultive les cellules animales - hôtes dans des conditions appropriées pour exprimer l'inhibiteur ; et
(f) on procède dans n'importe quel ordre :
(i) à la récupération de l'inhibiteur ; et
(ii) au traitement de l'inhibiteur pour lui conférer une structure tertiaire active de sorte qu'il possède une activité d'inhibition de métalloprotéinase.

19. Procédé selon la revendication 18, dans lequel le vecteur contient une partie de la séquence d'ADN suivante, codant pour un inhibiteur de métalloprotéinase actif :

10 20 30 40 50 60
GGCCATCGCC GCAGATCCAG CGCCCAGAGA GACACCAGAG AACCCACCAT GGCCCCCTTT

70 80 90 100 110 120
GAGCCCCTGG CTTCTGGCAT CCTGTTGTTG CTGTGGCTGA TAGCCCCCAG CAGGGCCTGC

130 140 150 160 170 180
ACCTGTGTCC CACCCCACCC ACAGACGGCC TTCTGCAATT CCGACCTCGT CATCAGGGCC

190 200 210 220 230 240
AAGTTCGTGG GGACACCAGA AGTCAACCAG ACCACCTTAT ACCAGCGTTA TGAGATCAAG

250 260 270 280 290 300
ATGACCAAGA TGTATAAAGG GTTCCAAGCC TTAGGGGATG CCGCTGACAT CCGGTTCGTC

310 320 330 340 350 360
TACACCCCCG CCATGGAGAG TGTCTGCGGA TACTTCCACA GGTCCCACAA CCGCAGCGAG

370 380 390 400 410 420
GAGTTTCTCA TTGCTGGAAA ACTGCAGGAT GGACTCTTGC ACATCACTAC CTGCAGTTTC
430 440 450 460 470 480
GTGGCTCCCT GGAACAGCCT GAGCTTAGCT CAGCGCCGGG GCTTCACCAA GACCTACACT

490 500 510 520 530 540
GTTGGCTGTG AGGAATGCAC AGTGTTTCCC TGTTTATCCA TCCCCTGCAA ACTGCAGAGT

550 560 570 580 590 600
GGCACTCATT GCTTGTGGAC GGACCAGCTC CTCCAAGGCT CTGAAAAGGG CTTCCAGTCC

610 620 630 640 650 660
CGTCACCTTG CCTGCCTGCC TCGGGAGCCA GGGCTGTGCA CCTGGCAGTC CCTGCGGTCC

670 680 690 700 710 720
CAGATAGCCT GAATCCTGCC CGGAGTGGAA GCTGAAGCCT GCACAGTGTC CACCCTGTTC

730 740 750 760 770 780
CCACTCCCAT CTTTCTTCCG GACAATGAAA TAAAGAGTTA CCACCCAGCA AAAAAAAAAA

GGAATTC

## Revendications (Revendications pour l'(les) Etat(s) contractant(s) suivant(s) : suivant : AT)

1. Séquence d'ADN portable capable de contrôler la production intracellulaire d'inhibiteurs de métalloprotéinase, comprenant la séquence :

10         20         30         40         50         60
GTTGTTGCTG TGGCTGATAG CCCCAGCAGG GCCTGCACCT GTGTCCCACC CCACCCACAG

70         80         90         100        110        120
ACGGCCTTCT GCAATTCCGA CCTCGTCATC AGGGCCAAGT TCGTGGGGAC ACCAGAAGTC

130        140        150        160        170        180
AACCAGACCA CCTTATACCA GCGTTATGAG ATCAAGATGA CCAAGATGTA TAAAGGGTTC

190        200        210        220        230        240
CAAGCCTTAG GGGATGCCGC TGACATCCGG TTCGTCTACA CCCCCGCCAT GGAGAGTGTC

250        260        270        280        290        300
TGCGGATACT TCCACAGGTC CCACAACCGC AGCGAGGAGT TTCTCATTGC TGGAAAACTG

310        320        330        340        350        360
CAGGATGGAC TCTTGCACAT CACTACCTGC AGTTTCGTGG CTCCCTGGAA CAGCCTGAGC

370        380        390        400        410        420
TTAGCTCAGC GCCGGGGCTT CACCAAGACC TACACTGTTG GCTGTGAGGA ATGCACAGTG

430        440        450        460        470        480
TTTCCCTGTT TATCCATCCC CTGCAAACTG CAGAGTGGCA CTCATTGCTT GTGGACCCAC

490        500        510        520        530        540
CAGCTCCTCC AAGGCTCTGA AAAGGGCTTC CAGTCCCGTC ACCTTGCCTG CCTGCCTCGG

550        560        570        580        590        600
GAGCCAGGGC TGTGCACCTG GCAGTCCCTG CGGTCCCAGA TAGCCTGAAT CCTGCCCGGA
610        620        630        640        650        660
GTGGAAGCTG AAGCCTGCAC AGTGTCCACC CTGTTCCCAC TCCCATCTTT CTTCCCCACA

670        680        690        700
ATGAAATAAA GAGTTACCAC CCAGCAAAAA AAAAAAGGAA TTC

2. Vecteur de clonage d'ADN recombinant capable de s'exprimer dans un système bactérien, comprenant une séquence nucléotidique capable de contrôler la production intracellulaire d'inhibiteurs de métalloprotéinase comportant un résidu cystéine à l'extrémité N-terminale, ce vecteur comprenant une séquence nucléotidique contenant au moins une partie des nucléotides suivants codant pour un inhibiteur de métalloprotéinase actif :

10 20 30 40 50 60
GTTGTTGCTG TGGCTGATAG CCCCAGCAGG GCCTGCACCT GTGTCCCACC CCACCCACAG

70 80 90 100 110 120
ACGGCCTTCT GCAATTCCGA CCTCGTCATC AGGGCCAAGT TCGTGGGGAC ACCAGAAGTC

130 140 150 160 170 180
AACCAGACCA CCTTATACCA GCGTTATGAG ATCAAGATGA CCAAGATGTA TAAAGGGTTC

190 200 210 220 230 240
CAAGCCTTAG GGGATGCCGC TGACATCCGG TTCGTCTACA CCCCCGCCAT GGAGAGTGTC

250 260 270 280 290 300
TGCGGATACT TCCACAGGTC CCACAACCGC AGCGAGGAGT TTCTCATTGC TGGAAAACTG

310 320 330 340 350 360
CAGGATGGAC TCTTGCACAT CACTACCTGC AGTTTCGTGG CTCCCTGGAA CAGCCTGAGC

370 380 390 400 410 420
TTAGCTCAGC GCCGGGGCTT CACCAAGACC TACACTGTTG GCTGTGAGGA ATGCACAGTG

430 440 450 460 470 480
TTTCCCTGTT TATCCATCCC CTGCAAACTG CAGAGTGGCA CTCATTGCTT GTGGACGGAC
490 500 510 520 530 540
CAGCTCCTCC AAGGCTCTGA AAAGGGCTTC CAGTCCCGTC ACCTTGCCTG CCTGCCTCGG

550 560 570 580 590 600
GAGCCAGGGC TGTGCACCTG GCAGTCCCTG CGGTCCCAGA TAGCCTGAAT CCTGCCCGGA

610 620 630 640 650 660
GTGGAAGCTG AAGCCTGCAC AGTGTCCACC CTGTTCCCAC TCCCATCTTT CTTCCGGACA

670 680 690 700
ATGAAATAAA GAGTTACCAC CCAGCAAAAA AAAAAAGGAA TTC

3. Vecteur pUC9 - F5/237P10 déposé sous le numéro ATCC 53003.

4. Procédé à ADN recombinant pour la production bactérienne d'un inhibiteur de métalloprotéinase comportant un résidu cystéine à l'extrémité N-terminale, selon lequel :
(a) on prépare une séquence d'ADN portable capable de contrôler une bactérie-hôte pour produire une séquence protéique ayant une activité potentielle d'inhibition de métalloprotéinase ;
(b) on clone la séquence d'ADN portable dans un vecteur capable d'être transféré et de se répliquer dans une bactérie - hôte, ce vecteur contenant des éléments fonctionnels pour la séquence d'ADN portable ;
(c) on transfère le vecteur contenant la séquence d'ADN portable et des éléments fonctionnels, dans une bactérie - hôte capable d'exprimer la protéine inhibitrice de métalloprotéinase ;
(d) on cultive la bactérie-hôte dans des conditions appropriées pour amplifier le vecteur et exprimer l'inhibiteur ; et
(e) on procède dans n'importe quel ordre :
(i) à la récupération de l'inhibiteur ; et
(ii) au traitement de l'inhibiteur pour lui conférer une structure tertiaire active de sorte qu'il possède une activité d'inhibition de métalloprotéinase.

5. Procédé selon la revendication 4, dans lequel l'inhibiteur de métalloprotéinase, est un inhibiteur de collagénase.

6. Procédé selon la revendication 5, dans lequel la séquence d'ADN portable contient une partie de la séquence suivante :

10 20 30 40 50 60
GTTGTTGCTG TGGCTGATAG CCCCAGCAGG GCCTGCACCT GTGTCCCACC CCACCCACAG

70 80 90 100 110 120
ACGGCCTTCT GCAATTCCGA CCTCGTCATC AGGGCCAAGT TCGTGGGGAC ACCAGAAGTC

130 140 150 160 170 180
AACCAGACCA CCTTATACCA GCGTTATGAG ATCAAGATGA CCAAGATGTA TAAAGGGTTC

190 200 210 220 230 240
CAAGCCTTAG GGGATGCCGC TGACATCCGG TTCGTCTACA CCCCCGCCAT GGAGAGTGTC

250 260 270 280 290 300
TGCGGATACT TCCACAGGTC CCACAACCGC AGCGAGGAGT TTCTCATTGC TGGAAAACTG

310 320 330 340 350 360
CAGGATGGAC TCTTGCACAT CACTACCTGC AGTTTCGTGG CTCCCTGGAA CAGCCTGAGC

370 380 390 400 410 420
TTAGCTCAGC GCCGGGGCTT CACCAAGACC TACACTGTTG GCTGTGAGGA ATGCACAGTG

430 440 450 460 470 480
TTTCCCTGTT TATCCATCCC CTGCAAACTG CAGAGTGGCA CTCATTGCTT GTGGACGGAC

490 500 510 520 530 540
CAGCTCCTCC AAGGCTCTGA AAAGGGCTTC CAGTCCCGTC ACCTTGCCTG CCTGCCTCGC

550 560 570 580 590 600
GAGCCAGGGC TGTGCACCTG GCAGTCCCTG CGGTCCCAGA TAGCCTGAAT CCTGCCCGGA

610 620 630 640 650 660
GTGGAAGCTG AAGCCTGCAC AGTGTCCACC CTGTTCCCAC TCCCATCTTT CTTCCGGACA

670 680 690 700
ATGAAATAAA GAGTTACCAC CCAGCAAAAA AAAAAAGGAA TTC

7. Procédé selon la revendication 4, dans lequel on emploie le vecteur de la revendication 4.

8. Procédé selon la revendication 4, dans lequel la bactérie - hôte est un membre du genre Bacillus.

9. Procédé selon la revendication 8, dans lequel la bactérie est Bacillus subtilis.

10. Procédé selon la revendication 4, dans lequel la bactérie est Escherichia coli.

11. Procédé selon la revendication 4, dans lequel la bactérie est un membre du genre Pseudomonas.

12. Procédé selon la revendication 11, dans lequel la bactérie est Pseudomonas aeruginosa.

13. Procédé selon la revendication 4, dans lequel l'inhibiteur est récupéré avant de lui conférer ladite structure tertiaire active.

14. Procédé selon la revendication 4, dans lequel l'inhibiteur est traité pour lui conférer ladite structure tertiaire active avant qu'il ne soit récupéré.

15. Inhibiteur de métalloprotéinase qui est biologiquement équivalent à l'inhibiteur de collagénase isolable à partir de fibroblastes cutanés humains, produits selon le procédé de la revendication 4.

16. Micro - organisme C600/pUC9 - F5/237P1 0 correspondant au numéro d'enregistrement ATCC 53003.

17. Séquence d'ADN portable capable de contrôler la production intracellulaire d'inhibiteurs de métallo - protéinase, comprenant la séquence nucléotidique :

10 20 30 40 50 60
GGCCATCGCC GCAGATCCAG CGCCCAGAGA GACACCAGAG AACCCACCAT GGCCCCCTTT

70 80 90 100 110 120
GACCCCTGGC TTCTGCATCC TGTTGTTGCT GTGGCTGATA GCCCCAGCAG GGCCTGCACC

130 140 150 160 170 180
TGTGTCCCAC CCCACCCACA GACGGCCTTC TGCAATTCCG ACCTCGTCAT CAGGGCCAAG

190 200 210 220 230 240
TTCGTGGGGA CACCAGAAGT CAACCAGACC ACCTTATACC AGCGTTATGA GATCAAGATG

250 260 270 280 290 300
ACCAAGATGT ATAAAGGGTT CCAAGCCTTA GGGGATGCCG CTGACATCCG GTTCGTCTAC

310 320 330 340 350 360
ACCCCCGCCA TGGAGAGTGT CTGCGGATAC TTCCACAGGT CCCACAACCG CAGCGAGGAG

370 380 390 400 410 420
TTTCTCATTG CTGGAAAACT GCAGGATGGA CTCTTGCACA TCACTACCTG CAGTTTCGTG

430 440 450 460 470 480
GCTCCCTGGA ACAGCCTGAG CTTAGCTCAG CGCCGGGGCT TCACCAAGAC CTACACTGTT

490 500 510 520 530 540
GGCTGTGAGG AATGCACAGT GTTTCCCTGT TTATCCATCC CCTGCAAACT GCAGAGTGGC

550 560 570 580 590 600
ACTCATTGCT TGTGGACGGA CCAGCTCCTC CAAGGCTCTG AAAAGGGCTT CCAGTCCCGT

610 620 630 640 650 660
CACCTTGCCT GCCTGCCTCG GGAGCCAGGG CTGTGCACCT GGCAGTCCCT GCGGTCCCAG

670 680 690 700 710 720
ATAGCCTGAA TCCTGCCCGG AGTGGAAGCT GAAGCCTGCA CAGTGTCCAC CCTGTTCCCA

730 740 750 760 770 780
CTCCCATCTT TCTTCCGGAC AATGAAATAA AGAGTTACCA CCCAGCAAAA AAAAAAAGGA

18. Procédé à ADN recombinant pour la production d'inhibiteurs de métalloprotéinase comportant un résidu cystéine à l'extrémité N-terminale, à partir de cellules animales, selon lequel :
(a) on prépare une séquence d'ADN portable capable de contrôler une cellule animale - hôte pour produire une protéine ayant une activité d'inhibition de métalloprotéinase ;
(b) on clone la séquence d'ADN portable dans un vecteur capable d'être transféré et de se répliquer dans une cellule animale-hôte, ce vecteur contenant des éléments fonctionnels pour la séquence d'ADN portable ;
(c) on amplifie le vecteur contenant la séquence d'ADN portable et des éléments fonctionnels dans un hôte bactérien ;
(d) on transfère le vecteur recombinant amplifié dans une cellule animale - hôte capable d'exprimer la protéine d'inhibition de métalloprotéinase ;
(e) on cultive les cellules animales - hôtes dans des conditions appropriées pour exprimer l'inhibiteur ; et
(f) on procède dans n'importe quel ordre :
(i) à la récupération de l'inhibiteur ; et
(ii) au traitement de l'inhibiteur pour lui conférer une structure tertiaire active de sorte qu'il possède une activité d'inhibition de métalloprotéinase.

19. Procédé selon la revendication 18, dans lequel le vecteur contient une partie de la séquence d'ADN suivante, codant pour un inhibiteur de métalloprotéinase actif :

10 20 30 40 50 60
GGCCATCGCC GCAGATCCAG CGCCCAGAGA GACACCAGAG AACCCACCAT GGCCCCCTTT

70 80 90 100 110 120
GAGCCCCTGG CTTCTGGCAT CCTGTTGTTG CTGTGGCTGA TAGCCCCCAG CAGGGCCTGC

130 140 150 160 170 180
ACCTGTGTCC CACCCCACCC ACAGACGGCC TTCTGCAATT CCGACCTCGT CATCAGGGCC

190 200 210 220 230 240
AAGTTCGTGG GGACACCAGA AGTCAACCAG ACCACCTTAT ACCAGCGTTA TGAGATCAAG

250 260 270 280 290 300
ATGACCAAGA TGTATAAAGG GTTCCAAGCC TTAGGGGATG CCGCTGACAT CCGGTTCGTC

310 320 330 340 350 360
TACACCCCCG CCATGGAGAG TGTCTGCGGA TACTTCCACA GGTCCCACAA CCGCAGCGAG

370 380 390 400 410 420
GAGTTTCTCA TTGCTGGAAA ACTGCAGGAT GGACTCTTGC ACATCACTAC CTGCAGTTTC

430 440 450 460 470 480
GTGGCTCCCT GGAACAGCCT GAGCTTAGCT CAGCGCCGGG GCTTCACCAA GACCTACACT

490 500 510 520 530 540
GTTGGCTGTG AGGAATGCAC AGTGTTTCCC TGTTTATCCA TCCCCTGCAA ACTGCAGAGT

550 560 570 580 590 600
GGCACTCATT GCTTGTGGAC GGACCAGCTC CTCCAAGGCT CTGAAAAGGG CTTCCAGTCC

610 620 630 640 650 660
CGTCACCTTG CCTGCCTGCC TCGGGAGCCA GGGCTGTGCA CCTGGCAGTC CCTGCGGTCC

670 680 690 700 710 720
CAGATAGCCT GAATCCTGCC CGGAGTGGAA GCTGAAGCCT GCACAGTGTC CACCCTGTTC

730 740 750 760 770 780
CCACTCCCAT CTTTCTTCCG GACAATGAAA TAAAGAGTTA CCACCCAGCA AAAAAAAAAA

GGAATTC

20. Procédé de production d'une séquence d'ADN portable capable de contrôler la production intracellu - laire d'inhibiteurs de métalloprotéinase, caractérisé en ce que l'on prépare la séquence d'ADN suivante

10 20 30 40 50 60
GTTGTTGCTG TGGCTGATAG CCCCAGCAGG GCCTGCACCT GTGTCCCACC CCACCCACAG

70 80 90 100 110 120
ACGGCCTTCT GCAATTCCCA CCTCGTCATC AGGGCCAAGT TCGTGGGGAC ACCAGAAGTC

130 140 150 160 170 180
AACCAGACCA CCTTATACCA GCGTTATGAG ATCAAGATGA CCAAGATGTA TAAAGGGTTC

190 200 210 220 230 240
CAAGCCTTAG GGGATGCCGC TGACATCCGG TTCGTCTACA CCCCCGCCAT GGAGAGTGTC

250 260 270 280 290 300
TGCGGATACT TCCACAGGTC CCACAACCGC AGCGAGGAGT TTCTCATTGC TGGAAAACTG

310 320 330 340 350 360
CAGGATGGAC TCTTGCACAT CACTACCTGC AGTTTCGTGG CTCCCTGGAA CAGCCTGAGC

370 380 390 400 410 420
TTAGCTCAGC GCCGGGGCTT CACCAAGACC TACACTGTTG GCTGTGAGGA ATGCACAGTG

430 440 450 460 470 480
TTTCCCTGTT TATCCATCCC CTGCAAACTG CAGAGTGGCA CTCATTGCTT GTGGACGGAC

490 500 510 520 530 540
CAGCTCCTCC AAGGCTCTGA AAAGGGCTTC CAGTCCCGTC ACCTTGCCTG CCTGCCTCGG

550 560 570 580 590 600
GAGCCAGGGC TGTGCACCTG GCAGTCCCTG CGGTCCCAGA TAGCCTGAAT CCTGCCCGGA
610 620 630 640 650 660
GTGGAAGCTG AAGCCTGCAC AGTGTCCACC CTGTTCCCAC TCCCATCTTT CTTCCGGACA

670 680 690 700
ATGAAATAAA GAGTTACCAC CCAGCAAAAA AAAAAAGGAA TTC

21. Procédé de préparation d'un vecteur de clonage d'ADN recombinant, capable de s'exprimer dans un système bactérien, par introduction d'une séquence nucléotidique capable de contrôler la production intracellulaire d'inhibiteurs de métalloprotéinase comportant un résidu cystéine à l'extrémité N - terminale, dans un vecteur de clonage, selon lequel la séquence nucléotidique comprend au moins une partie des nucléotides suivants, codant pour un inhibiteur de métalloprotéinase actif :

10 20 30 40 50 60
GTTGTTGCTG TGGCTGATAG CCCCAGCAGG GCCTGCACCT GTGTCCCACC CCACC[?]ACAG

70 80 90 100 110 120
ACGGCCTTCT GCAATTCCGA CCTCGTCATC AGGGCCAAGT TCGTGGGGAC ACCAGAAGTC

130 140 150 160 170 180
AACCAGACCA CCTTATACCA GCGTTATGAG ATCAAGATGA CCAAGATGTA TAAAGGGTTC

190 200 210 220 230 240
CAAGCCTTAG GGGATGCCGC TGACATCCGG TTCGTCTACA CCCCCGCCAT GGAGAGTGTC

250 260 270 280 290 300
TGCGGATACT TCCACAGGTC CCACAACCGC AGCGAGGAGT TTCTCATTGC TGGAAAACTG

310 320 330 340 350 360
CAGGATGGAC TCTTGCACAT CACTACCTGC AGTTTCGTGG CTCCCTGGAA CAGCCTGAGC

370 380 390 400 410 420
TTAGCTCAGC GCCGGGGCTT CACCAAGACC TACACTGTTG GCTGTGAGGA ATGCACAGTG

430 440 450 460 470 480
TTTCCCTGTT TATCCATCCC CTGCAAACTG CAGAGTGGCA CTCATTGCTT GTGGACGGAC
490 500 510 520 530 540
CAGCTCCTCC AAGGCTCTGA AAAGGGCTTC CAGTCCCGTC ACCTTGCCTG CCTGCCT[?]GG

550 560 570 580 590 600
GAGCCAGGGC TGTGCACCTG GCAGTCCCTG CGGTCCCAGA TAGCCTGAAT CCTG[?][?][?][?]A
610 620 630 640 650 660
GTGGAAGCTG AAGCCTGCAC AGTGTCCACC CTGTTCCCAC TCCCATCTTT CTT[?][?][?]ACA

670 680 690 700
ATGAAATAAA GAGTTACCAC CCAGCAAAAA AAAAAAGGAA TTC

22. Procédé de préparation du vecteur pUC9-F5/237P10, en isolant le vecteur à partir du micro- organisme déposé sous le numéro ATCC 53003.

23. Procédé de production d'un micro - organisme contenant le plasmide pUC9 - F5/237P1 correspondant au numéro ATCC 53003, par introduction de ce plasmide dans E. coli C600.

24. Procédé de production d'une séquence d'ADN portable capable de contrôler la production intracellu - laire d'inhibiteurs de métalloprotéinase, en préparant la séquence nucléotidique suivante :
